(19) Europäisches Patentamt
European Patent Office
Office européen des brevets

(11) EP 3 816 283 A1

(12) EUROPEAN PATENT APPLICATION
published in accordance with Art. 153(4) EPC

(43) Date of publication:
**05.05.2021 Bulletin 2021/18**

(21) Application number: **19844916.7**

(22) Date of filing: **30.07.2019**

(51) Int Cl.:
***C12N 15/09*** (2006.01) ***C12M 1/00*** (2006.01)
***C12Q 1/6837*** (2018.01) ***G01N 33/53*** (2006.01)
***C12N 15/113*** (2010.01)

(86) International application number:
**PCT/JP2019/029761**

(87) International publication number:
**WO 2020/027098 (06.02.2020 Gazette 2020/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2018 JP 2018143668**

(71) Applicant: **Toray Industries, Inc.**
**Tokyo, 103-8666 (JP)**

(72) Inventors:
- **HOSHINO, Emi**
  **Kamakura-shi, Kanagawa 248-8555 (JP)**
- **SERIZAWA, Takashi**
  **Kamakura-shi, Kanagawa 248-8555 (JP)**
- **NATORI, Kazue**
  **Kamakura-shi, Kanagawa 248-8555 (JP)**

(74) Representative: **Prüfer & Partner mbB**
**Patentanwälte · Rechtsanwälte**
**Sohnckestraße 12**
**81479 München (DE)**

# (54) METHOD FOR EVALUATING QUALITY OF BODY FLUID SPECIMEN

(57) Reference miRNAs whose abundances are altered depending on quality change of a body fluid sample were identified to provide a method of evaluating the quality of a body fluid sample using as indices the abundances of the reference miRNAs in the sample.

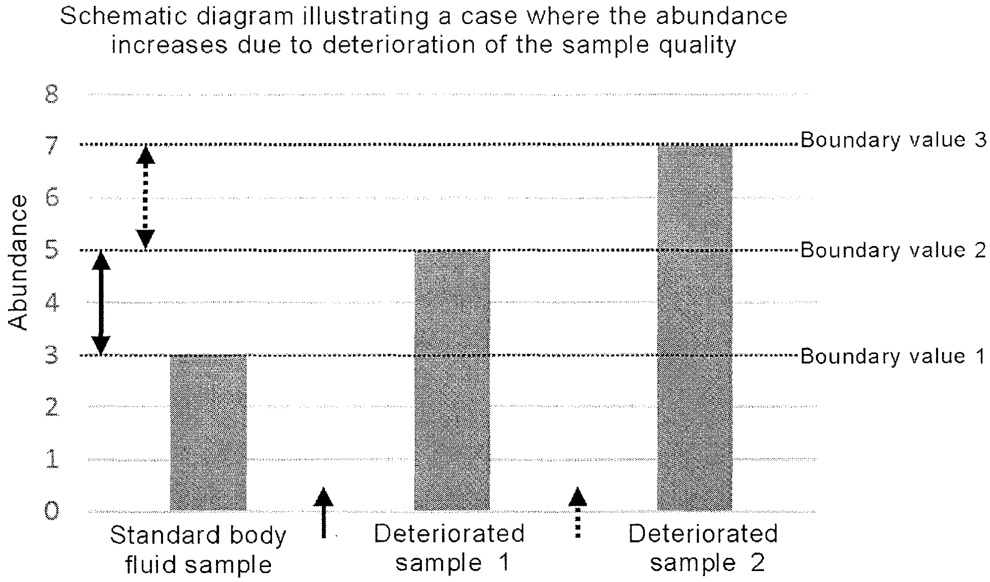


Fig.1

EP 3 816 283 A1

SEQUENCE LISTING

<110> Toray Industries, Inc.

<120> A method for quality evaluation of body fluid sample

<130> PF648-PCT

<160> 86

<170> PatentIn version 3.5

<210> 1
<211> 21
<212> RNA
<213> Homo Sapience

<400> 1
gcugggaagg caaagggacg u 21

<210> 2
<211> 23
<212> RNA
<213> Homo Sapience

<400> 2
cuggcggagc ccauuccaug cca 23

<210> 3
<211> 23
<212> RNA
<213> Homo Sapience

<400> 3
gggggccgau acacuguacg aga 23

<210> 4
<211> 24
<212> RNA
<213> Homo Sapience

<400> 4
ugggcgaggg gugggcucuc agag 24

<210> 5
<211> 21
<212> RNA
<213> Homo Sapience

<400> 5
caggcaggug uagggguggag c 21

<210> 6
<211> 22
<212> RNA
<213> Homo Sapience

<400> 6

ggcuacaaca caggacccgg gc 22

<210> 7
<211> 21
<212> RNA
<213> Homo Sapience

<400> 7
agcaugacag aggagaggug g 21

<210> 8
<211> 19
<212> RNA
<213> Homo Sapience

<400> 8
cauccguccg ucuguccac 19

<210> 9
<211> 22
<212> RNA
<213> Homo Sapience

<400> 9
ugcggggcua gggcuaacag ca 22

<210> 10
<211> 23
<212> RNA
<213> Homo Sapience

<400> 10
caggcagaag uggggcugac agg 23

<210> 11
<211> 22
<212> RNA
<213> Homo Sapience

<400> 11
uauagggauu ggagccgugg cg 22

<210> 12
<211> 21
<212> RNA
<213> Homo Sapience

<400> 12
aaggcagggc ccccgcuccc c 21

<210> 13
<211> 20
<212> RNA
<213> Homo Sapience

<400> 13

aaaagcuggg cugagaggcg 20

<210> 14
<211> 21
<212> RNA
<213> Homo Sapience

<400> 14
ccugcgaguc uccggcggug g 21

<210> 15
<211> 24
<212> RNA
<213> Homo Sapience

<400> 15
cugcaggcag aaguggggcu gaca 24

<210> 16
<211> 22
<212> RNA
<213> Homo Sapience

<400> 16
aggcagcggg guguagugga ua 22

<210> 17
<211> 110
<212> RNA
<213> Homo Sapience

<400> 17
ggcuacaguc uuucuucaug ugacucgugg acuucccuuu gucauccuau gccugagaau 60

auaugaagga ggcugggaag gcaaagggac guucaauugu caucacuggc 110

<210> 18
<211> 76
<212> RNA
<213> Homo Sapience

<400> 18
cgcaggccuc uggcggagcc cauuccaugc cagaugcuga gcgauggcug gugugugcug 60

cuccacaggc cuggug 76

<210> 19
<211> 84
<212> RNA
<213> Homo Sapience

<400> 19
ugugcagugg gaagggggc cgauacacug uacgagagug aguagcaggu cucacaguga 60

accggucucu uucccuacug uguc 84

<210> 20
<211> 64
<212> RNA
<213> Homo Sapience

<400> 20
ucugggcgag ggugggcuc ucagaggggc uggcaguacu gcucugaggc cugccucucc 60

ccag 64

<210> 21
<211> 69
<212> RNA
<213> Homo Sapience

<400> 21
ccgggcaggc agguguaggg uggagcccac uguggcuccu gacucagccc ugcugccuuc 60

accugccag 69

<210> 22
<211> 109
<212> RNA
<213> Homo Sapience

<400> 22
ggucgggcuc accaugacac agugugagac cucgggcuac aacacaggac ccgggcgcug 60

cucugacccc ucgugucuug uguugcagcc ggagggacgc agguccgca 109

<210> 23
<211> 113
<212> RNA
<213> Homo Sapience

<400> 23
agcaugacag aggagaggug gagguaggcg agaguaauau aauuucucca ggagaacauc 60

ugagagggga aguugcuuuc cugcccuggc ccuuucaccc uccugaguuu ggg 113

<210> 24
<211> 80
<212> RNA
<213> Homo Sapience

<400> 24
ggagaaagga guggaccgag gaaggaagga aggcaaggcu gucuguccau ccguccgucu 60

guccaccuac cugucagucc 80

<210> 25
<211> 98
<212> RNA
<213> Homo Sapience

<400> 25
uugggcaagg ugcggggcua gggcuaacag cagucuuacu gaagguuucc uggaaaccac 60

gcacaugcug uugccacuaa ccucaaccuu acucgguc 98

<210> 26
<211> 67
<212> RNA
<213> Homo Sapience

<400> 26
ccugcaggca gaaguggggc ugacagggca gaggguugcg cccccucacc aucccuucug 60

ccugcag 67

<210> 27
<211> 67
<212> RNA
<213> Homo Sapience

<400> 27
ccugcaggca gaaguggggc ugacagggca gaggguugcg cccccucacc aucccuucug 60

ccugcag 67

<210> 28
<211> 67
<212> RNA
<213> Homo Sapience

<400> 28
ccugcaggca gaaguggggc ugacagggca gaggguugcg cccccucacc aucccuucug 60

ccugcag 67

<210> 29
<211> 67
<212> RNA
<213> Homo Sapience

<400> 29
ccugcaggca gaaguggggc ugacagggca gaggguugcg cccccucacc aucccuucug 60

ccugcag 67

<210> 30
<211> 90
<212> RNA
<213> Homo Sapience

<400> 30
aggccucgcu guucucuaug gcuuuuuauu ccuaugugau ucuacugcuc acucauauag 60

ggauuggagc cguggcgcac ggcggggaca 90

<210> 31
<211> 94
<212> RNA
<213> Homo Sapience

<400> 31
gugaggugug ggcccggccc caggagcggg gccugggcag ccccgugugu ugaggaagga 60

aggcagggcc cccgcucccc gggccugacc ccac 94

<210> 32
<211> 73
<212> RNA
<213> Homo Sapience

<400> 32
agggagaaaa gcugggcuga gaggcgacug gugucuaauu uguuugucuc uccaacucag 60

acugccuggc cca 73

<210> 33
<211> 60
<212> RNA
<213> Homo Sapience

<400> 33
ccugcgaguc uccggcggug gcuuguggcu gagugucacg cugcuggcgc aggcucggcc 60

<210> 34
<211> 85
<212> RNA
<213> Homo Sapience

<400> 34
gggacggggc cugcaggcag aaguggggcu gacagggcag aggguugcgc cccucacca 60

ccccuucugc cugcagcggu gggcu 85

<210> 35
<211> 71
<212> RNA
<213> Homo Sapience

<400> 35
ggggccugca ggcagaagug gggcugacag ggcagagggu ugcgccccu caccaccccu 60

ucugccugca g 71

<210> 36
<211> 74
<212> RNA
<213> Homo Sapience

<400> 36
ccgcacucuc uccauuacac uacccugccu cuucuccaug agaggcagcg ggguguagug 60

gauagagcac gggu 74

<210> 37
<211> 22
<212> RNA
<213> Homo Sapience

<400> 37
gggaccaucc ugccugcugu gg 22

<210> 38
<211> 21
<212> RNA
<213> Homo Sapience

<400> 38
agccgcgggg aucgccgagg g 21

<210> 39
<211> 16
<212> RNA
<213> Homo Sapience

<400> 39
accgccugcc caguga 16

<210> 40
<211> 17
<212> RNA
<213> Homo Sapience

<400> 40
cuccgggacg gcugggc 17

<210> 41
<211> 23
<212> RNA
<213> Homo Sapience

<400> 41
ugaguggggc ucccgggacg gcg 23

<210> 42
<211> 22
<212> RNA
<213> Homo Sapience

<400> 42
gguggcccgg ccgugccuga gg 22

<210> 43
<211> 22
<212> RNA
<213> Homo Sapience

<400> 43
gggugggau uuguugcauu ac 22

<210> 44
<211> 19
<212> RNA
<213> Homo Sapience

<400> 44
aucccaccac ugccaccau 19

<210> 45
<211> 23
<212> RNA
<213> Homo Sapience

<400> 45
ggaggcgcag gcucggaaag gcg 23

<210> 46
<211> 23
<212> RNA
<213> Homo Sapience

<400> 46
ugagcaccac acaggccggg cgc 23

<210> 47
<211> 18
<212> RNA
<213> Homo Sapience

<400> 47
ccagaggugg ggacugag 18

<210> 48
<211> 19
<212> RNA
<213> Homo Sapience

<400> 48
ggcuugcaug ggggacugg 19

<210> 49
<211> 22
<212> RNA
<213> Homo Sapience

<400> 49
caggaaggau uuagggacag gc 22

<210> 50
<211> 18
<212> RNA
<213> Homo Sapience

<400> 50
aggcugggcu gggacgga 18

<210> 51
<211> 17
<212> RNA
<213> Homo Sapience

<400> 51
ccccggggag cccggcg 17

<210> 52
<211> 21
<212> RNA
<213> Homo Sapience

<400> 52
cugggcucgg gacgcgcggc u 21

<210> 53
<211> 22
<212> RNA
<213> Homo Sapience

<400> 53
gagcaggcga ggcugggcug aa 22

<210> 54
<211> 18
<212> RNA
<213> Homo Sapience

<400> 54
cggugagcgc ucgcuggc 18

<210> 55
<211> 24
<212> RNA
<213> Homo Sapience

<400> 55
agggcuggac ucagcggcgg agcu 24

<210> 56
<211> 21
<212> RNA
<213> Homo Sapience

<400> 56
acggcccagg cggcauuggu g 21

<210> 57
<211> 19
<212> RNA
<213> Homo Sapience

<400> 57
agcagggcug gggauugca 19

<210> 58
<211> 25
<212> RNA
<213> Homo Sapience

<400> 58
agggggcac ugcgcaagca aagcc 25

<210> 59
<211> 23
<212> RNA
<213> Homo Sapience

<400> 59
uggggaaggc uuggcaggga aga 23

<210> 60
<211> 24
<212> RNA
<213> Homo Sapience

<400> 60
ugggggagcc augagauaag agca 24

<210> 61
<211> 22
<212> RNA
<213> Homo Sapience

<400> 61
uucagauccc agcggugccu cu 22

<210> 62
<211> 83
<212> RNA
<213> Homo Sapience

<400> 62
acggcaucuu ugcacucagc aggcaggcug gugcagcccg uggugggga ccauccugcc 60

ugcuguggg uaaggacggc ugu 83

<210> 63
<211> 180
<212> RNA
<213> Homo Sapience

<400> 63
cgcgacugcg gcggcggugg ugggggagc cgcggggauc gccgagggcc ggucggccgc 60

cccgggugcc gcgcggugcc gccggcggcg gugaggcccc gcgcguguguu cccggcugcg 120

gucggccgcg cucgaggggu ccccguggcg uccccuuccc cgccggccgc cuuucucgcg 180

<210> 64
<211> 57
<212> RNA
<213> Homo Sapience

<400> 64
agaggcaccg ccugcccagu gacaugcguu uaacggccgc gguacccuaa cugugca 57

<210> 65
<211> 89
<212> RNA
<213> Homo Sapience

<400> 65
accuccggga cggcugggcg ccggcggccg ggagauccgc gcuuccugaa ucccggccgg 60

cccgcccggc gcccguccgc ccgcggguc 89

<210> 66
<211> 62
<212> RNA
<213> Homo Sapience

<400> 66
gugagugggg cucccgggac ggcgcccgcc cuggcccugg cccggcgacg ucucacgguc 60

cc 62

<210> 67
<211> 115
<212> RNA
<213> Homo Sapience

<400> 67
ggugccgagg gccguccggc auccuaggcg ggucgcugcg guaccucccu ccugucugug 60

gcggugggau cccguggccg uguuuuccug guggcccggc cgugccugag guuuc 115

<210> 68
<211> 75
<212> RNA
<213> Homo Sapience

<400> 68
ucaucccugg guggggauuu guugcauuac uuguguucua uauaaaguau ugcacuuguc 60

ccggccugug gaaga 75

<210> 69
<211> 89
<212> RNA
<213> Homo Sapience

<400> 69
ucuccguuua ucccaccacu gccaccauua uugcuacugu ucagcaggug cugcuggugg 60

ugauggugau agucuggugg gggcggugg 89

<210> 70
<211> 73
<212> RNA
<213> Homo Sapience

<400> 70

ggcgagggga ggcgcaggcu cggaaaggcg cgcgaggcuc caggcuccuu cccgauccac 60

cgcucuccuc gcu 73

<210> 71
<211> 97
<212> RNA
<213> Homo Sapience

<400> 71
cccgggaccu ugguccaggc gcuggucugc guggugcucg gguggauaag ucugaucuga 60

gcaccacaca ggccgggcgc cgggaccaag ggggcuc 97

<210> 72
<211> 86
<212> RNA
<213> Homo Sapience

<400> 72
ggcuuagaaa cagucccuag guaggauuug gggaggagcu aagaagcccc uacagggccc 60

agaggugggg acugagccuu aguugg 86

<210> 73
<211> 85
<212> RNA
<213> Homo Sapience

<400> 73
ggccugggua ggcuugcaug ggggacuggg aagagaccau gaacagguua guccagggag 60

uucucaucaa gccuuuacuc aguag 85

<210> 74
<211> 70
<212> RNA
<213> Homo Sapience

<400> 74
aaaagccugu cccuaagucc cucccagccu uccagaguug gugccaggaa ggauuuaggg 60

acaggcuuug 70

<210> 75
<211> 73
<212> RNA
<213> Homo Sapience

<400> 75
ggaggcuggg cugggacgga cacccggccu ccacuuucug uggcagguac cuccuccaug 60

ucggcccgcc uug 73

<210> 76
<211> 51
<212> RNA

<213> Homo Sapience

<400> 76
acagaccccg gggagcccgg cggugaagcu ccugguaucc ugggugucug a 51

<210> 77
<211> 87
<212> RNA
<213> Homo Sapience

<400> 77
cccaggcgcc cgcucccgac ccacgccgcg ccgccggguc ccuccucccc ggagaggcug 60

ggcucgggac gcgcggcuca gcucggg 87

<210> 78
<211> 60
<212> RNA
<213> Homo Sapience

<400> 78
gagcaggcga ggcugggcug aacccguggg ugaggagugc agcccagcug aggccucugc 60

<210> 79
<211> 74
<212> RNA
<213> Homo Sapience

<400> 79
gcagcccggu gagcgcucgc uggccuggca gugcgucgga agaacagggc gggugggcc 60

gcgcacaucu cugc 74

<210> 80
<211> 100
<212> RNA
<213> Homo Sapience

<400> 80
agcucagggc ggcugcgcag agggcuggac ucagcggcgg agcuggcugc uggccucagu 60

ucugccucug uccagguccu ugugacccgc ccgcucuccu 100

<210> 81
<211> 95
<212> RNA
<213> Homo Sapience

<400> 81
gacaccacau gcuccuccag gccugccugc ccuccagguc auguuccagu gucccacaga 60

ugcagcacca cggcccaggc ggcauuggug ucacc 95

<210> 82
<211> 109
<212> RNA
<213> Homo Sapience

```
<400>  82
ugcuauuguc uuacugcuac agcagggcug gggauugcag uauccgcugu ugcugcugcu      60

cccaguccug ccccugcugc uaccuagucc agccucaccg caucccaga                 109


<210>  83
<211>  66
<212>  RNA
<213>  Homo Sapience

<400>  83
ccuggagggg ggcacugcgc aagcaaagcc agggacccug agaggcuuug cuuccugcuc      60

cccuag                                                                  66


<210>  84
<211>  79
<212>  RNA
<213>  Homo Sapience

<400>  84
cagccugggg aaggcuuggc agggaagaca caugagcagu gccuccacuu cacgccucuc      60

ccuugucucc uuucccuag                                                    79


<210>  85
<211>  81
<212>  RNA
<213>  Homo Sapience

<400>  85
aguuggugggg ggagccauga gauaagagca ccuccuagag aauguugaac uaaaggugcc     60

cucucuggcu ccucccccaaa g                                                81


<210>  86
<211>  119
<212>  RNA
<213>  Homo Sapience

<400>  86
ccaugaggag cuggcagugg gauggccugg ggguaggagc guggcuucug gagcuagacc      60

acaugguuc agaucccagc ggugccucua acuggccaca ggaccuuggg cagucagcu       119
```

**Description**

TECHNICAL FIELD

The present invention relates to a method of evaluating the quality of a body fluid sample based on the abundance(s) of a particular miRNA(s) contained in the body fluid sample.

BACKGROUND ART

A miRNA (microRNA) is transcribed from genomic DNA as an RNA (precursor) having a hairpin-like structure. This precursor is cleaved by a particular enzyme, dsRNA cleavage enzyme (Drosha, Dicer), having RNase III cleavage activity, and converted into a double-stranded form and then into single strands. It is thought that the antisense strand, which is one of the double-strands, is incorporated into a protein complex called RISC, to be involved in translational suppression of mRNA. Thus, miRNA takes various forms in various stages after its transcription. Therefore, when a miRNA is to be detected, various forms including the hairpin structure, double-stranded structure, and single-stranded structure need to be taken into account. A miRNA consists of an RNA of 15 to 25 bases, and the presence of miRNAs has been confirmed in various organisms.

In recent years, it has been suggested that a large amount of miRNAs are present not only in cells, but also in body fluids such as serum, plasma, urine, and spinal fluid, which are samples containing no cells, and that the abundances of those miRNAs may become biomarkers for various diseases including cancers. As of June 2018, there are not less than 2600 kinds of miRNAs in human, and, when a highly sensitive assay system such as a DNA microarray is used, expression of more than 1000 kinds of miRNAs among them can be detected simultaneously in serum or plasma. Thus, studies are being carried out to find biomarkers in body fluids such as serum/plasma, urine, and spinal fluid using the DNA microarray method, and development of biomarker tests that enable early detection of diseases is expected.

On the other hand, RNA is a substance whose degradation easily occurs due to various physical and chemical factors such as heat, degradative enzymes, and freeze-thawing. In gene expression analysis using a DNA microarray, degradation of RNA is known to affect measurement of the abundance. In a test by measurement of the abundance of miRNA contained in a body fluid as a disease biomarker, if the test/diagnosis is carried out based on an inaccurate measured value of the abundance, the patient may miss the chance of an appropriate treatment, or may be forced to bear an unnecessary economical or physical burden due to application of wrong medical care. Thus, for accurate measurement of the abundance, it is very important to carry out the test using a sample in which the target miRNA to be tested is not degraded.

Conventionally, electrophoresis has been commonly used as a method for measuring the degree of degradation of RNA. For example, the measurement can be carried out based on the band intensity ratio (28S/18S) between a band derived from 28S ribosome RNA and a band derived from 18S ribosome RNA. As another method, Patent Document 1 proposes a method in which the degree of RNA degradation is quantitatively evaluated based on the lengths of RNA segments, which method utilizes the property of long-chain RNA that degradation of nucleotides causes shortening of the segment lengths.

However, RNA in a short-chain fraction is often used for measurement of the abundance of a miRNA, and the fraction does not contain long-chain RNA in such cases. Therefore, conventional methods such as those described above cannot be effective methods for measuring the degree of degradation of RNA. Although the degree of degradation of RNA used can also be measured based on correlation coefficients among the total genes obtained from the result of gene expression analysis, this method requires data on the total genes and thus it takes a lot of time and labor. In view of this, a method focusing on degraded fragments derived from long-chain RNA, wherein the degree of degradation of miRNA in a short-chain fraction is evaluated using as an index degraded fragments contained in the short-chain fraction, has been developed (Patent Document 2). Patent Document 3 discloses a method in which the degree of degradation of miRNA contained in a body fluid sample is measured to evaluate the quality.

PRIOR ART DOCUMENTS

PATENT DOCUMENTS

Patent Document 1: JP 2015-519045 A
Patent Document 2: JP 2008-35779 A
Patent Document 3: WO 2017/146033

## SUMMARY OF THE INVENTION

### PROBLEMS TO BE SOLVED BY THE INVENTION

As described above, for accurate measurement of the abundance of a target RNA, it is important to evaluate the sample quality by measuring the degree of degradation of RNA in the sample. However, the methods of Patent Document 1 and Patent Document 2 are methods utilizing ribosomal RNA or long-chain RNA. Ribosomal RNA and long-chain RNA are RNAs present in nuclei and cytoplasm, and they are hardly present in body fluid samples such as serum, plasma, urine, and spinal fluid. Thus, by these methods, accurate measurement of the degree of degradation of miRNA contained in a body fluid sample has been impossible, so that evaluation of the quality has been impossible.

On the other hand, Patent Document 3 discloses a plurality of miRNAs whose abundances change in accordance with degradation of miRNA contained in body fluid. More specifically, miRNAs whose degradation occurs when they are left to stand at 4°C from 0 hour to 2 weeks in the serum state were selected. However, based on comparison with their abundances at 0 hour, they hardly show changes in the abundance after 6 hour-standing, and show changes of only about 10% in the abundance even after 24 hour-standing. When whether deterioration of the sample quality has occurred by leaving a sample to stand at 4°C for 24 hours wants to be judged, detection of the small difference, as small as 10%, in the abundance may be impossible due to variation in the assay system. Therefore, the judgment may be difficult by the method described in Patent Document 3. When gene expression analysis is carried out using a DNA microarray, and deterioration of the sample quality during a period of as short as several hours to about one day after collection of the sample has been found to affect measurement and diagnosis, a sensitive index or method is required for detecting the deterioration of the sample quality during the short period in order to judge whether the measurement is possible or not.

An object of the present invention is to discover a method of measuring the degree of degradation of miRNA contained in a body fluid sample, to evaluate the quality, in particular, a method which enables sensitive detection of deterioration of the body fluid sample quality which occurs during a period of as short as several hours to about one day after collection of the body fluid sample.

### MEANS FOR SOLVING THE PROBLEMS

In order to solve the above-described problem, the present inventors discovered that the quality of a body fluid sample can be evaluated by measuring, as a reference(s), the abundance(s) of a miRNA(s) (hereinafter referred to as "reference miRNA(s)") whose abundance(s) change(s) depending on deterioration of the body fluid sample that has occurred in several hours to about one day after collection of the body fluid sample, thereby completing the present invention. More specifically, the present invention is a method of evaluating the quality of a body fluid sample by using one or more of the miRNAs shown in SEQ ID NOs:1 to 16 and 37 to 61 as a reference miRNA(s), wherein the abundance(s) of the reference miRNA(s) contained in the body fluid sample is/are compared with an arbitrarily predetermined threshold(s) to evaluate the quality of the body fluid sample. The present invention includes the following modes.

(1) A method of evaluating the quality of a body fluid sample, the method comprising:

a measuring step of measuring the abundance(s) of one or more reference miRNAs selected from miRNAs consisting of the base sequences shown in SEQ ID NOs: 1 to 16 and 37 to 61 in the body fluid sample; and

a judging step of judging the quality of the body fluid sample by comparing the abundance(s) of the one or more reference miRNAs obtained in the measuring step, or by comparing an index value(s) calculated from the abundances of the plurality of reference miRNAs, with an arbitrarily predetermined threshold(s).

(2) The method according to (1), wherein the index value is a difference or ratio between the abundances of two arbitrarily selected reference miRNAs.

(3) The method according to (1) or (2),
wherein:

each of the miRNAs consisting of the base sequences shown in SEQ ID NOs: 1, 5, and 7 is a miRNA which indicates poor quality of the body fluid sample in a case where the abundance in the body fluid sample is higher than a first threshold or lower than a second threshold;

each of the miRNAs consisting of the base sequences shown in SEQ ID NOs: 2, 3, 4, 6, 11, 37 to 43, 45, 46, 49, 51, 52, 54, and 58 is a miRNA which indicates poor quality of the body fluid sample in a case where the abundance in the body fluid sample is higher than a threshold; and

each of the miRNAs consisting of the base sequences shown in SEQ ID NOs: 8, 9, 10, 12 to 16, 44, 47, 48, 50, 53, 55 to 57, and 59 to 61 is a miRNA which indicates poor quality of the body fluid sample in a case where the abundance in the body fluid sample is lower than a threshold.

(4) The method according to any one of (1) to (3), wherein the measuring step is a step of carrying out hybridization by bringing a probe(s) for capturing one or more reference miRNAs selected from miRNAs consisting of the base sequences shown in SEQ ID NOs:1 to 16 and 37 to 61, the probe(s) being immobilized on a support, into contact with a nucleic acid sample which is derived from the body fluid sample and labeled with a labeling substance, to measure the abundance(s) of the one or more reference miRNAs in the body fluid sample.
(5) The method according to any one of (1) to (4), further comprising a correction step of correcting the measured value(s) of the abundance(s) of the one or more reference miRNAs obtained in the measuring step, wherein the judging step is carried out using the corrected value(s) of the abundance(s).
(6) The method according to any one of (1) to (5), wherein the measuring step comprises measuring the abundance(s) of a target miRNA(s) in the body fluid sample at the same time as the measurement of the abundance(s) of the one or more reference miRNAs in the body fluid sample.
(7) The method according to (6), wherein the measuring step is a step of carrying out hybridization by bringing a probe(s) for capturing a target miRNA(s) and a probe(s) for capturing one or more reference miRNAs selected from miRNAs consisting of the base sequences shown in SEQ ID NOs:1 to 16 and 37 to 61, the probes being immobilized on a support, into contact with a nucleic acid sample which is derived from the body fluid sample and labeled with a labeling substance, to measure the abundance of each of the target miRNA(s) and the one or more reference miRNAs in the body fluid sample.
(8) The method according to (6) or (7), further comprising a correction step of correcting the measured value(s) of the abundance(s) of the target miRNA(s) and the measured value(s) of the abundance(s) of the one or more reference miRNAs in the body fluid sample, obtained in the measuring step.
(9) The method according to any one of (1) to (8), wherein the body fluid sample is whole blood, serum, or plasma.
(10) A program(s) for evaluating the quality of a body fluid sample, said program(s) causing one or more computers to execute:

a measured value-obtaining step of obtaining a measured value(s) of the abundance(s) of one or more reference miRNAs selected from miRNAs consisting of the base sequences shown in SEQ ID NOs:1 to 16 and 37 to 61 in the body fluid sample, the measured value(s) being a value(s) measured using an RNA sample prepared from the body fluid sample; and
a judging step of judging the quality of the body fluid sample by comparing the abundance(s) of the one or more reference miRNAs, or by comparing an index value(s) calculated from the abundances of the plurality of reference miRNAs, with an arbitrarily predetermined threshold(s).

(11)A computer-readable recording medium in which the program(s) according to (10) is recorded.
(12) A chip for miRNA quality evaluation, comprising a support on which a probe(s) for capturing one or more reference miRNAs selected from miRNAs consisting of the base sequences shown in SEQ ID NOs:1 to 16 and 37 to 61 is/are immobilized.

EFFECT OF THE INVENTION

The present invention enables highly-accurate and simple evaluation of the degree of deterioration of the quality of a body fluid sample, in particular, evaluation of whether or not deterioration of the sample quality (mainly miRNA degradation) occurred in a period of as short as several hours to about one day after collection of the body fluid sample, which has been difficult for the conventional methods. Further, since the present invention enables highly-accurate and simple evaluation of whether or not a body fluid sample has a quality suitable for, for example, gene expression analysis using miRNA, a more accurate test result can be obtained in a test for a disease using as an index the abundance(s) of a biomarker(s) in the body fluid sample.

BRIEF DESCRIPTION OF THE DRAWINGS

Fig. 1 is a schematic diagram related to setting of thresholds.
Fig. 2 is a schematic diagram illustrating cases where a threshold is set taking measurement variation, variation among samples, and the like into account.
Fig. 3 shows alteration in the abundance of hsa-miR-204-3p detected by a DNA microarray in Example 1 when different coagulation temperatures (7 conditions in total) were applied to samples in the whole-blood state.

Fig. 4 shows alteration in the abundance ofhsa-miR-4730 detected by a DNA microarray in Example 1 when different coagulation times (4 conditions in total) were applied at room temperature to samples in the whole-blood state.

Fig. 5 shows alteration in the abundances of hsa-miR-204-3p and hsa-miR-4730 detected by a DNA microarray in Example 2 when different coagulation temperatures (2 conditions in total) were applied to samples in the whole-blood state.

Fig. 6 shows alteration in the difference between the abundances of hsa-miR-204-3p and hsa-miR-4730 detected by a DNA microarray in Example 2 when different coagulation temperatures (2 conditions in total) were applied to samples in the whole-blood state.

Fig. 7 shows alteration in the abundance of hsa-miR-4800-3p detected by a DNA microarray in Example 3 when different standing times and standing temperatures (8 conditions in total) were applied to samples in the serum state.

Fig. 8 shows alteration in the abundance of hsa-miR-135a-3p detected by a DNA microarray in Example 3 when different standing times (6 conditions in total) were applied at room temperature to samples in the serum state.

Fig. 9 shows alteration in the abundances of hsa-miR-204-3p and hsa-miR-4800-3p detected by a DNA microarray in Example 4 when different standing times (2 conditions in total) were applied to samples in the serum state.

Fig. 10 shows alteration in the difference between the abundances of hsa-miR-204-3p and hsa-miR-4800-3p detected by a DNA microarray in Example 4 when different standing times (2 conditions in total) were applied to samples in the serum state.

Fig. 11 shows alteration in the abundance of hsa-miR-3648 detected by a DNA microarray in Example 5 when different coagulation temperatures and coagulation times (7 conditions in total) were applied to samples in the whole-blood state.

Fig. 12 shows alteration in the abundance of hsa-miR-4632-5p detected by a DNA microarray in Example 5 when different coagulation temperatures and coagulation times (7 conditions in total) were applied to samples in the whole-blood state.

Fig. 13 shows alteration in the abundances of hsa-miR-3648 and hsa-miR-6780b-5p detected by a DNA microarray in Example 6 when different coagulation times (2 conditions in total) were applied to samples in the whole-blood state.

Fig. 14 shows alteration in the difference between the abundances of hsa-miR-3648 and hsa-miR-6780b-5p detected by a DNA microarray in Example 6 when different coagulation times (2 conditions in total) were applied to samples in the whole-blood state.

Fig. 15 shows alteration in the abundance of hsa-miR-4497 detected by a DNA microarray in Example 7 when different standing times and standing temperatures (8 conditions in total) were applied to samples in the serum state.

Fig. 16 shows alteration in the abundance of hsa-miR-744-5p detected by a DNA microarray in Example 7 when different standing times and standing temperatures (8 conditions in total) were applied to samples in the serum state.

Fig. 17 shows alteration in the abundances of hsa-miR-4497 and hsa-miR-744-5p detected by a DNA microarray in Example 8 when different standing times (2 conditions in total) were applied to samples in the serum state.

Fig. 18 shows alteration in the difference between the abundances of hsa-miR-4497 and hsa-miR-744-5p detected by a DNA microarray in Example 8 when different standing times (2 conditions in total) were applied to samples in the serum state.

Fig. 19 shows alteration in the abundance of hsa-miR-204-3p detected by quantitative RT-PCR in Example 9 when different standing times (2 conditions in total) were applied to samples in the serum state.

## MODE FOR CARRYING OUT THE INVENTION

The present invention is a method of evaluating the quality of a body fluid sample, the method comprising:

a measuring step of measuring a reference miRNA(s) contained in the body fluid sample, wherein one or more miRNAs selected from miRNAs consisting of the base sequences shown in SEQ ID NOs: 1 to 16 and 37 to 61 were used as a reference miRNA(s); and

a judging step of judging the quality of the body fluid sample by comparing the abundance(s) of the one or more reference miRNAs obtained in the measuring step, or by comparing an index value(s) calculated from the abundances of the plurality of reference miRNAs, with an arbitrarily predetermined threshold(s).

The method of the present invention can be used for preliminarily evaluating the quality of miRNA contained in a body fluid sample for gene expression analysis, for example, for analysis using an array chip such as a microarray or for analysis by the polymerase chain reaction (PCR) method or the sequencing method, to thereby judge whether the analysis can be appropriately carried out. Examples of the gene expression analysis include: a process in which miRNA in a body fluid is labeled, and a support on which a probe(s) for capturing one or more target miRNAs and a probe(s) for capturing the reference miRNA(s) are immobilized is used to measure the abundance of each miRNA; a process in which probes for amplifying one or more target miRNAs and probes for amplifying a reference miRNA(s) are used to carry out amplification reaction, to measure the abundance(s) of the target miRNA(s); and further, a process in which results from the above-described processes are utilized to carry out an analysis or a test of gene expression, for example, a test by measurement of gene expression in a clinical sample for understanding pathological conditions.

"miRNA" is a non-coding RNA (ncRNA), which means a short-chain RNA produced in a living body whose chain length is about 15 to 25 bases, and is thought to have a function to regulate expression of mRNA. A miRNA is transcribed as an RNA (precursor) having a hairpin-like structure from genomic DNA. This precursor is cleaved by a particular enzyme, dsRNA cleavage enzyme (Drosha, Dicer), having RNase III cleavage activity, and converted into a double-stranded form and then into single strands. It is thought that the antisense strand, which is one of the double-strands, is incorporated into a protein complex called RISC and that the RISC is involved in suppression of translation of mRNA. Thus, miRNA takes various forms in the various stages after its transcription. Therefore, usually, when a miRNA is targeted (to be detected), its various forms including the hairpin structure, double-stranded structure, and single-stranded structure need to be taken into account. The presence of miRNAs has been confirmed in various organisms.

The body fluid samples to which the present invention is applicable are body fluid samples separated from living bodies, and examples of the body fluid samples include, but are not limited to, body fluids such as blood (whole blood, serum, and plasma), urine, spinal fluid, saliva, swab, and various tissue fluids. The type of the living body from which the body fluid sample is derived is not limited, and includes various organism species. It is typically a mammal, especially human.

A body fluid sample contains various biomolecules. Examples of the biomolecules include proteins; peptides; nucleic acids such as DNA and RNA; and metabolites. These biomolecules are suitable as biomarkers for various diseases.

Deterioration of the quality of a body fluid sample means that the abundances of the biomolecules change from those at the time point when the sample was collected, and mainly means that degradation of RNA including miRNA proceeds. Possible causes thereof include temperature and heat; external forces on the body fluids, such as vibration and ultrasonic waves; and direct or indirect physical forces such as electric fields and magnetic fields; but the cause of quality deterioration is not limited thereto.

In the present invention, RNA may be extracted from these samples, and the extracted RNA may be used for measuring the abundances of miRNAs. For the extraction of RNA, a known method (for example, a method by Favaloro et al. (Favaloro et al., Methods Enzymol. 65: 718 (1980))) or a commercially available kit for RNA extraction (for example, miRNeasy, manufactured by QIAGEN; or "3D-Gene" RNA extraction reagent from liquid sample, manufactured by Toray Industries, Inc.) may be applied.

<Measuring Step>

In the present invention, the abundance(s) of one or more reference miRNAs selected from miRNAs consisting of the base sequences shown in SEQ ID NOs:1 to 16 and 37 to 61, contained in a body fluid sample is/are measured. Concurrently with the measurement of the abundance(s) of the reference miRNA(s) contained in the body fluid sample, measurement of the abundance(s) of a target miRNA(s) may be carried out. The target miRNA is defined as the miRNA to be measured for each purpose, among the miRNAs contained in the body fluid sample.

The miRNAs consisting of the base sequences shown in SEQ ID NOs:1 to 16 and 37 to 61, which may be used as reference miRNAs in the present invention, are miRNAs which were discovered by the present inventors as miRNAs whose abundances are altered depending on the change in the quality of a body fluid sample. A change in (or deterioration of) the quality of a body fluid sample causes a change in the abundance of RNA of each gene contained in the sample. In such a situation, a correlation between RNA in a body fluid sample intentionally deteriorated by warming or the like (deteriorated body fluid sample) and RNA in a completely fresh body fluid sample free from deterioration (standard body fluid sample) is lowered in all genes detected in gene expression analysis. The degree of deterioration of the quality of the deteriorated body fluid sample can be evaluated, for example, using twice the standard deviation (2SD) of the abundance ratio (FCi) of each miRNA that can be calculated according to the following Equations 1 and 2. In the present invention, the 2SD value is referred to as the overall change index value. An overall change index value of not less than 1.5 indicates that the degree of change in the abundance of each miRNA measured in the deteriorated body fluid sample is large, and hence that the degree of deterioration of the quality of the deteriorated body fluid sample is large. The reference miRNAs used in the present invention are miRNAs whose abundances are altered in correlation with such an overall change of RNA.

$$FC_i = miRNA_{i_control} - miRNA_{i_sample} \quad \text{(Equation 1)}$$

$$2SD = 2^{2\times\sqrt{\frac{1}{n-1}\sum_{i=1}^{n}(FC_i - F\ average)^2}} \quad \text{(Equation 2)}$$

Here, in Equation 1 and Equation 2,
$miRNA_{i_control}$ is the abundance of the *i*th miRNA in the standard body fluid sample, expressed as a base-2 logarithm;
$miRNA_{i_sample}$ is the abundance of the *i*th miRNA in the deteriorated body fluid sample, expressed as a base-2 logarithm; and
$FC_{average}$ is the average of the abundance ratios ($miRNA_{i_control}$ - $miRNA_{i_sample}$) of the *n* miRNAs.

In cases where serum (blood) is used as the body fluid sample, miRNA whose abundance is altered depending on the storage time and/or storage temperature during storage of the sample in the whole blood state after blood collection or in the serum state may be selected as a reference miRNA used in the present invention. miRNAs whose abundances are altered depending on the storage time in the whole-blood state may be selected by, for example, storing a sample, in the state of whole-blood immediately after blood collection, under a certain temperature condition (for example, at room temperature (22°C to 24°C)), separating sera at 0 hour, 3 hours, 6 hours, and 9 hours after the start of the storage, measuring the abundances of miRNAs in the sera, and then comparing the degree of change in the abundance of each miRNA. In cases where a blood sample is stored as whole blood for a longer period in an actual test of clinical samples or the like, the storage time may be extended to, for example, 12 hours or 24 hours so as to cover the storage period, and the abundance of each miRNA may be measured and compared. In such a manner, the abundances of each miRNA obtained from the sera which have undergone the different storage times in the whole-blood state may be compared among the different storage conditions, to select miRNAs showing a difference. In general, in an assay using a DNA microarray, a 2-fold change in the abundance is thought to be a sufficient difference. Therefore, miRNAs showing a 2-fold or greater difference among the different storage conditions are preferably selected. miRNAs whose abundances are altered depending on the storage time of serum may be selected by, for example, preparing a serum sample after blood collection; storing the serum sample in a refrigerator (for example, at 4°C); measuring the abundances of miRNAs in the serum at 0 hour, 6 hours, 12 hours, and 24 hours after the start of the storage, and then comparing the degree of change in the abundance of each miRNA. Similarly, miRNAs whose abundances are altered depending on the storage temperature during storage in the whole-blood state or the serum state may also be selected by storing a sample in the state of whole-blood immediately after blood collection or in the serum state under temperature conditions according to requirement for a certain period, measuring the abundances of miRNAs in each sample, and then comparing the degree of change in the abundance of each miRNA.

In the measuring step in the present invention, the abundance(s) of one or more reference miRNAs selected from miRNAs consisting of the base sequences shown in SEQ ID NOs:1 to 16 and 37 to 61, contained in a body fluid sample is/are measured.

Probes for capturing reference miRNAs and target miRNAs are hereinafter collectively referred to as "capture probes" or, simply, "probes".

Measurement of the abundance of a miRNA may be carried out by, for example, a hybridization assay using an array chip such as a microarray in which a probe that specifically binds to the subject miRNA is immobilized on a support. In the present invention, an array chip comprising a support on which a "reference miRNA capture probe(s)" for capturing one or more reference miRNAs is/are immobilized may be used. An array chip comprising a support on which a "target miRNA capture probe(s)" for capturing a target miRNA(s) is/are further immobilized may also be used.

The "capture probe" or the "probe for capturing" means a substance capable of directly or indirectly, preferably directly, and selectively binding to the miRNA to be captured. Representative examples of such a probe include nucleic acids, proteins, saccharides, and other antigenic compounds. In the present invention, nucleic acid probes may be preferably used. Examples of the nucleic acids that may be used include not only DNA and RNA, but also nucleic acid derivatives such as PNA (peptide nucleic acid) and LNA (Locked Nucleic Acid). The term "derivatives" means, when used for nucleic acids, chemically modified derivatives, such as labeled derivatives prepared using a fluorophore or the like, and derivatives comprising a modified nucleotide (a nucleotide containing halogen, or containing a group such as alkyl including methyl; alkoxy including methoxy; thio; or carboxymethyl; a nucleotide that has undergone, for example, reconstruction of the base, saturation of the double bonds, deamination, and/or substitution of an oxygen molecule(s) into a sulfur molecule(s); and/or the like).

From the viewpoint of securing stability and specificity in the hybridization, the chain length of the nucleic acid probe is preferably not less than the length of the miRNA to be detected. Usually, when the chain length is about 17 to 25 bases, the probe can sufficiently exert the selective binding capacity to the subject miRNA. Such an oligonucleic acid probe having a short chain length can be easily prepared by a well-known chemical synthesis method or the like.

The stringency in the hybridization is known to be a function of the temperature, the salt concentration, the chain length of the probe, the GC content of the nucleotide sequence of the probe, and the concentration of the chaotropic agent in the hybridization buffer. As stringent conditions, those described in Sambrook, J. et al. (1998) Molecular Cloning:

A Laboratory Manual (2nd ed.), Cold Spring Harbor Laboratory Press, New York, and the like may be employed. A stringent temperature condition is not less than about 30°C. Examples of other conditions include the hybridization time, the concentration of the washing agent (for example, SDS), and the presence or absence of carrier DNA. By combining these conditions, various stringencies can be set. Those skilled in the art can appropriately determine conditions for obtaining the function of the capture probe provided for detection of a desired sample RNA.

The nucleic acid probe is the complementary strand of the miRNA to be captured. It is, however, evident to those skilled in the art that cross-hybridization may cause binding of the probe to sequences other than the sequence to be captured. Thus, in the present invention, the abundances of miRNAs are measured using the complementary strands of the reference miRNAs represented by SEQ ID NOs:1 to 16 and 37 to 61 as probes, and changes in the abundances of the miRNAs due to deterioration may include changes in the abundances of cross-hybridizing RNAs other than the reference miRNAs.

When deterioration of a sample proceeds to cause degradation of RNA in the sample, degradation of miRNAs also proceeds. In some cases, molecules that cross-hybridize with reference miRNA capture probes may increase in the sample as the degradation proceeds. In addition, in cases where a blood sample is left to stand in the whole-blood state, miRNAs are secreted with time from blood cells, which may lead to increases in the reference miRNAs themselves and/or miRNAs that cross-hybridize with the reference miRNA capture probes in the sample (Koberle V. et al., (2016) Translational Res.169:40-46). Thus, a "change in the abundance of a miRNA due to deterioration" detected with a capture probe includes not only a decrease, but also an increase in the abundance of the miRNA.

Sequence information of miRNA can be obtained from databases such as GenBank (http://www.ncbi.nlm.nih.gov/genbank/), or the website of miRBase (http://www.mirbase.org/). The reference miRNA capture probe(s) and the target miRNA capture probe(s) can be designed based on sequence information available from these sites.

The number of the miRNA capture probe(s) immobilized on the support is not limited. For example, the abundance(s) of the miRNA(s) may be measured using a support comprising miRNA capture probes immobilized thereon, with which all known miRNAs whose sequences have been identified are comprehensively covered. Or, a support comprising a desired number of miRNA capture probes immobilized thereon, depending on the purpose of the test or the like, may be used.

The support on which the capture probes are to be aligned and immobilized may be the same as a support used in a known microarray or macroarray. Examples of the support include slide glasses, membranes, and beads. The support described in JP 4244788 B, which has a plurality of protruded portions on its surface, may also be used. Examples of the material of the support include, but are not limited to, inorganic materials such as glass, ceramic, and silicon; and polymers such as polyethylene terephthalate, cellulose acetate, polycarbonate, polystyrene, polymethyl methacrylate, and silicone rubber.

Examples of known methods for immobilizing capture probes on a support include methods in which oligo-DNAs are synthesized on the surface of the support, and methods in which oligo-DNAs preliminarily synthesized are added dropwise to the surface of the support and then immobilized thereon.

Examples of the former methods include the method of Ronald et al. (US 5705610 B), the method of Michel et al. (US 6142266 B), and the method of Francesco et al. (US 7037659 B). Since these methods use an organic solvent for DNA synthesis reaction, the material of the support is preferably resistant to organic solvents. In the method of Francesco et al., the DNA synthesis is controlled by irradiation with light from the back side of the support, and therefore the material of the support is preferably a light-transmitting material.

Examples of the latter methods include the method of Hirota et al. (JP 3922454 B) and methods using a spotter. Examples of the spotting method include the pin method, which is based on mechanical contact of a pin tip with a solid phase; the ink jet method, which utilizes the principle of ink jet printers; and the capillary method, which uses a capillary. If necessary, after the spotting treatment, posttreatment such as cross-linking by UV irradiation and/or surface blocking is carried out. For allowing immobilization of the oligo-DNAs through covalent bonds on the surface of the surface-treated support, functional groups such as amino groups and/or SH groups are introduced to the termini of the oligo-DNAs. The surface modification of the support is usually carried out by treatment with a silane coupling agent having an amino group and/or the like.

The hybridization with the miRNA capture probes immobilized on the support is carried out by preparing, from RNA extracted from the sample, a nucleic acid sample (nucleic acid sample derived from the sample) labeled with a labeling substance, and bringing the labeled nucleic acid sample into contact with the probes. Examples of the "nucleic acid sample derived from the sample" include not only RNA extracted from the sample, but also cDNA prepared by reverse transcription reaction from the RNA, and cRNA. The labeled nucleic acid sample derived from the sample may be a sample prepared by directly or indirectly labeling the sample RNA with a labeling substance, or a sample prepared by directly or indirectly labeling cDNA or cRNA prepared from the sample RNA, with a labeling substance.

Examples of the method for binding the labeling substance to the nucleic acid sample derived from the sample include methods in which the labeling substance is bound to the 3'-end of the nucleic acid sample, methods in which the labeling substance is bound to the 5'-end of the nucleic acid sample, and methods in which a nucleotide to which the labeling substance is bound is incorporated into the nucleic acid. In the methods in which the labeling substance is bound to the 3'-end and the methods in which the labeling substance is bound to the 5'-end, enzymatic reaction may be used. In the enzymatic reaction, T4 RNA Ligase, Terminal Deoxytidyl Transferase, Poly A polymerase, or the like may be used. All these labeling methods may be carried out by reference to the methods described in "Shao-Yao Ying (ed.), miRNA Experimental Protocols, Yodosha Co., Ltd. (2008)". Various kits for directly or indirectly binding a labeling substance to an RNA terminus are commercially available. Examples of kits for directly or indirectly binding a labeling substance to the 3'-end include "3D-Gene" miRNA labeling kit (Toray Industries, Inc.), miRCURY miRNA HyPower labeling kit (Exiqon), NCode miRNA Labeling system (Life Technologies), and FlashTag Biotin RNA Labeling Kit (Genisphere).

In addition, in the same manner as a conventional method, cDNA or cRNA may be synthesized from sample RNA in the presence of labeled deoxyribonucleotides or labeled ribonucleotides to prepare cDNA or cRNA in which a labeled substance is incorporated, and the resulting cDNA or cRNA may be hybridized with the probes on the array.

Examples of labeling substances that may be used in the present invention include various labeling substances that are also used in known microarray analyses. Specific examples of the labeling substances include, but are not limited to, fluorescent dyes, phosphorescent dyes, enzymes, and radioisotopes. Fluorescent dyes are preferred since they can be simply measured and easily detectable. Specific examples of the fluorescent dyes include, but are not limited to, known fluorescent dyes such as Cyanine (Cyanine 2), aminomethylcoumarin, fluorescein, indocarbocyanine (Cyanine 3), Cyanine 3.5, tetramethylrhodamine, rhodamine red, Texas red, indocarbocyanine (Cyanine 5), Cyanine 5.5, Cyanine 7, and Oyster.

As the labeling substance, luminescent semiconductor particles may also be used. Examples of such semiconductor particles include cadmium selenium (CdSe), cadmium tellurium (CdTe), indium gallium phosphide (InGaP), and silver indium zinc sulfide (AgInZnS).

The thus labeled nucleic acid sample derived from the sample is brought into contact with the miRNA capture probes on the support, to allow hybridization of the nucleic acid sample with the probes. This hybridization step may be carried out in completely the same manner as the conventional hybridization step. The reaction temperature and the reaction time are appropriately selected depending on the chain length of the nucleic acid to be subjected to the hybridization. In cases of nucleic acid hybridization, the hybridization is usually carried out at about 30°C to 70°C for 1 minute to ten and several hours. After the hybridization and the washing, the signal intensity from the labeling substance in each probe-immobilized area on the support is detected. The detection of the signal intensity is carried out using an appropriate signal reader depending on the type of the labeling substance. When a fluorescent dye is used as the labeling substance, a fluorescence microscope, a fluorescence scanner, or the like may be used.

The measured value of the detected fluorescence intensity is compared with the surrounding noise. More specifically, the measured value obtained from the probe-immobilized area is compared with a measured value obtained from another position, and, in cases where the former value is higher, the signal intensity is regarded as being detected (effectively judged positive).

In cases where a background noise is included in the detected measured value, the background noise may be subtracted therefrom. The surrounding noise may be regarded as the background noise, and may be subtracted from the detected measured value. The method described in "Wataru Fujibuchi and Katsuhisa Horimoto (eds.), Microarray data statistical analysis protocols, Yodosha Co., Ltd. (2008)" may also be used.

<Correction Step>

In the present invention, the measured value of the abundance of each reference miRNA obtained in the measuring step may be used as it is in the judging step described later. However, for example, in cases where gene expression analysis of a target miRNA(s) contained in a body fluid sample is carried out, the measured value may be corrected by the methods exemplified below to obtain a corrected value of the abundance, and the corrected value may be used in the judging step.

The correction method may be a conventional method. Examples of the method include the global normalization method and the quantile normalization method, wherein the correction is carried out using the measured values of all miRNAs detected. The correction may also be carried out using a housekeeping RNA such as U1 snoRNA, U2 snoRNA, U3 snoRNA, U4 snoRNA, U5 snoRNA, U6 snoRNA, 5S rRNA, or 5.8S rRNA, or a particular endogenous miRNA for correction; or using an external standard nucleic acid added upon the RNA extraction or the labeling. The term "endogenous" means that the substance is not a substance artificially added to the sample, but a substance naturally present in the sample. For example, "endogenous miRNA" means a miRNA which is naturally present in the sample and derived from the organism from which the sample was provided. In cases where the method of the present invention is applied to gene expression analysis of a target miRNA contained in a body fluid sample, it is preferred to use a correction method utilizing an external standard nucleic acid such as a spike control which does not depend on the sample.

<Judging Step>

The judging step in the present invention is a step in which the abundance(s) of one or more reference miRNAs in a body fluid sample obtained in the measuring step, or an index value(s) calculated from corrected abundances of a plurality of reference miRNAs, is/are compared with an arbitrarily predetermined threshold(s), to judge the quality of the body fluid sample based on which value(s) is/are larger than the other(s). The reference miRNAs consisting of the base sequences shown in SEQ ID NOs:1 to 16 and 37 to 61 include both miRNAs which exhibit increased abundances (for example, hsa-miR-4730 consisting of the base sequence shown in SEQ ID NO:2) and miRNAs which exhibit decreased abundances (for example, hsa-miR-4800-3p consisting of the base sequence shown in SEQ ID NO:8) when the quality of the body fluid sample is poor. Thus, there are both cases where the quality can be judged to be poor when the abundance of the reference miRNA is higher than the arbitrarily predetermined threshold, and cases where the quality can be judged to be poor when the abundance is lower than the threshold. Therefore, the judgment criterion needs to be selected in accordance with the reference miRNA used in the judgment. Which type each of the 41 kinds of reference miRNAs consisting of the base sequences shown in SEQ ID NOs:1 to 16 and 37 to 61 belongs to when a blood sample is used as the body fluid sample is shown in Table 3, Table 5, Table 7, and Table 9 described later. The miRNAs consisting of the base sequences shown in SEQ ID NOs:2, 3, 4, 6, 11, 37 to 43, 45, 46, 49, 51, 52, 54, and 58 are miRNAs that exhibit increased abundances in a deteriorated body fluid sample, and the miRNAs consisting of the base sequences shown in SEQ ID NOs:8, 9, 10, 12 to 16, 44, 47, 48, 50, 53, 55 to 57, and 59 to 61 are miRNAs that exhibit decreased abundances in a deteriorated body fluid sample. The miRNAs consisting of the base sequences shown in SEQ ID NOs: 1, 5, and 7 are miRNAs which exhibit either decreased abundances or increased abundances depending on in which step of the sample treatment the deterioration has occurred.

In the judging step, the abundance(s) of one or more reference miRNAs obtained in the measuring step may be log-transformed, and the resulting logarithmic value(s) may be used to carry out the judgment. In cases where the log transformation is carried out, the conversion is generally conversion to a base-2 logarithm.

Regarding the threshold to be used as the judgment criterion, a standard body fluid sample and a deteriorated body fluid sample may be prepared, and the abundances of each reference miRNA contained in these body fluid samples may be measured. Based on the result, the threshold may be arbitrarily set depending on, for example, the purpose of the evaluation and the accuracy demanded.

The setting of the threshold is described below based on the schematic diagrams shown in Fig. 1 and Fig. 2. Fig. 1 and Fig. 2 are schematic diagrams showing measured abundances of a reference miRNA contained in a standard body fluid sample and two deteriorated body fluid samples (deteriorated samples 1 and 2), which diagrams illustrate the case where the abundance of the reference miRNA increases due to deterioration of the sample quality. The deteriorated sample 2 is a sample whose degree of deterioration is higher than that of the deteriorated sample 1.

In Fig. 1, the boundary values 1 to 3 are the abundances of the reference miRNA in the samples. In cases where the sample quality is to be judged between the standard body fluid sample and the deteriorated sample 1, the threshold may be set between the boundary values 1 and 2. If the quality deterioration is to be judged more severely, the threshold may be set to the boundary value 1, and if the quality deterioration is to be judged more mildly, the threshold may be set to the boundary value 2. In cases where the sample quality is to be judged between the deteriorated sample 1 and the deteriorated sample 2, the threshold may be set between the boundary values 2 and 3. If the quality deterioration is to be judged more severely, the threshold may be set to the boundary value 2, and if the quality deterioration is to be judged more mildly, the threshold may be set to the boundary value 3.

When there is some sort of variation such as variation among repeated measurements or variation among samples, the threshold may be set taking such variation into account. Fig. 2 is a bar chart showing the average abundance of a reference miRNA in each sample, wherein each error bar schematically shows the standard deviation (SD), and wherein the boundary values 4 to 9 are values each corresponding to the top or bottom of the error bar for each condition. In cases where the sample quality is to be judged between the standard body fluid sample and the deteriorated sample 1, the threshold may be set between the boundary values 5 and 6. If the quality deterioration is to be judged more severely, the threshold may be set to the boundary value 5, and if the quality deterioration is to be judged more mildly, the threshold may be set to the boundary value 6. In cases where the sample quality is to be judged between the deteriorated sample 1 and the deteriorated sample 2, the threshold may be set between the boundary values 7 and 8. If the quality deterioration is to be judged more severely, the threshold may be set to the boundary value 7, and if the quality deterioration is to be judged more mildly, the threshold may be set to the boundary value 8. If the quality is to be judged most severely, the threshold may be set to the boundary value 4, and if the quality deterioration is to be judged most mildly, the threshold may be set to the boundary value 9. The threshold may be set using 1SD, 2SD, or a range wider than these, and may be selected depending on the purpose. Figs. 1 and 2 show examples of the method of setting the threshold using the standard deviation, and the threshold may also be set using a method commonly used for evaluating variation in statistics, such as the standard error, confidence interval, or prediction interval.

As shown in Table 3, Table 5, Table 7, and Table 9 described later, each of the miRNAs consisting of the base sequences shown in SEQ ID NOs:2, 3, 4, 6, 11, 37 to 43, 45, 46, 49, 51, 52, 54, and 58 is a miRNA that exhibits an increased abundance in a deteriorated body fluid sample irrespective of in which step of the sample treatment the deterioration has occurred. The quality of the body fluid sample can be judged to be poor when its abundance in the body fluid sample is higher than the threshold. In cases where these miRNAs are used as reference miRNAs, the judgment of the quality is possible by setting one threshold for each miRNA.

Each of the miRNAs consisting of the base sequences shown in SEQ ID NOs:8, 9, 10, 12 to 16, 44, 47, 48, 50, 53, 55 to 57, and 59 to 61 is a miRNA that exhibits a decreased abundance in a deteriorated body fluid sample irrespective of in which step of the sample treatment the deterioration has occurred. The quality of the body fluid sample can be judged to be poor when its abundance in the body fluid sample is lower than the threshold. In cases where these miRNAs are used as reference miRNAs, the judgment of the quality is possible by setting one threshold for each miRNA.

Each of the miRNAs consisting of the base sequences shown in SEQ ID NOs: 1, 5, and 7 is a miRNA that exhibits either a decreased abundance or increased abundance depending on in which step of the sample treatment the deterioration has occurred. More specifically, these miRNAs exhibit decreased abundances in cases where the serum sample has undergone deterioration by being left to stand under conditions where the temperature is higher than room temperature (for example, at 28°C or higher) for several hours (for example, 6 hours or longer) in the whole-blood state before the serum separation, while the miRNAs exhibit increased abundances in cases where the sample has undergone deterioration in the serum state after the serum separation. Thus, in cases where these miRNAs are used as reference miRNAs to evaluate the quality of an arbitrary clinical body fluid sample, it is preferred to set the following two thresholds for each reference miRNA: a "first threshold", with which the quality of the sample is judged to be poor when the value is higher than this threshold; and a "second threshold", with which the quality of the sample is judged to be poor when the value is lower than this threshold. In cases where the abundance of each of these reference miRNAs in the serum sample is higher than the first threshold or lower than the second threshold, the quality of the sample can be judged to be poor. In cases where the abundance of the reference miRNA in the serum sample is higher than the first threshold, deterioration can be assumed to have occurred in the serum state, while in cases where the abundance is lower than the second threshold, deterioration can be assumed to have occurred in the whole-blood state. In cases where the abundance of the reference miRNA in the serum sample is between the first threshold and the second threshold, the quality of the sample can be judged to be good.

In cases where a plurality of reference miRNAs selected from the miRNAs consisting of the base sequences shown in SEQ ID NOs:1 to 16 and 37 to 61 are used, the abundance of each individual reference miRNA in the body fluid sample and a threshold predetermined for the individual miRNA may be compared to determine which is larger than the other, and judgment may be carried out for each individual miRNA based on a judgment criterion. The results may then be evaluated as a whole to judge the quality of the body fluid sample. In such cases, it is preferred to employ an additional judgement criterion by, for example, assigning the order of priority or weight to the individual judgments that are made based on the plurality of reference miRNAs.

More specifically, for example, if the number of reference miRNAs bringing the result that the quality is good exceeds the number of reference miRNAs bringing the result that the quality is poor, or exceeds an arbitrary predetermined number in the judgment by each individual reference miRNA, the overall quality of miRNA contained in the body fluid sample can be judged to be good. Conversely, if the number of reference miRNAs bringing the result that the quality is poor exceeds the number of reference miRNAs bringing the result that the quality is good, or exceeds a predetermined number, the overall quality of miRNA contained in the body fluid sample may be judged to be poor. In cases where severer or more accurate evaluation is to be carried out, if one particular reference miRNA brings the result that the quality is poor, the quality of miRNA contained in the body fluid sample may be judged to be poor.

In cases where a plurality of reference miRNAs selected from the miRNAs consisting of the base sequences shown in SEQ ID NOs:1 to 16 and 37 to 61 are used, an index value(s) may be calculated from the abundances of the plurality of reference miRNAs in the body fluid sample, and the quality of the body fluid sample may be judged based on whether the index value(s) is/are higher or lower than a predetermined threshold(s). As an index value, a difference or a ratio between two reference miRNAs can be used.

In cases of a combination in which the abundances come close to each other due to deterioration (for example, the combination of hsa-miR-204-3p and hsa-miR-4730, which is shown in Fig. 5), the index value (difference) becomes smaller as deterioration of the body fluid sample proceeds. Thus, when such a combination is used, the quality can be judged to be poor if the index value (difference) is lower than a predetermined threshold. When a ratio is employed as the index value in use of such a combination, the judgment may be carried out as follows. When the index value employed is A/B, wherein A represents the abundance, in the body fluid sample, of a reference miRNA that is more abundant in a non-deteriorated standard body fluid sample (hsa-miR-204-3p in the example shown in Fig. 5), and wherein B represents the abundance, in the body fluid sample, of a reference miRNA that is less abundant in a non-deteriorated standard body fluid sample (hsa-miR-4730 in the example shown in Fig. 5), the value A/B decreases as the deterioration proceeds. The body fluid sample can therefore be judged to have poor quality when the value is lower than a predetermined threshold. When B/A is used as the index value, the value B/A increases as the deterioration proceeds. The body fluid sample can therefore be judged to have poor quality when the value is higher than a predetermined threshold.

In cases of a combination in which the abundances get away from each other due to deterioration (for example, the combination of hsa-miR-204-3p and hsa-miR-4800-3p, which is shown in Fig. 9), the index value (difference) becomes larger as deterioration of the body fluid sample proceeds. Thus, when such a combination is used, the quality can be judged to be poor if the index value (difference) is higher than a predetermined threshold. When a ratio is employed as the index value in use of such a combination, the judgment may be carried out as follows. When the index value employed is A/B, wherein A represents the abundance, in the body fluid sample, of a reference miRNA that is more abundant in a non-deteriorated standard body fluid sample (hsa-miR-204-3p in the example shown in Fig. 9), and wherein B represents the abundance, in the body fluid sample, of a reference miRNA that is less abundant in a non-deteriorated standard body fluid sample (hsa-miR-4800-3p in the example shown in Fig. 9), the value A/B increases as the deterioration proceeds. The body fluid sample can therefore be judged to have poor quality when the value is higher than a predetermined threshold. When B/A is used as the index value, the value B/A decreases as the deterioration proceeds. The body fluid sample can therefore be judged to have poor quality when the value is lower than a predetermined threshold.

In cases where judgment based on an index value(s) is carried out using three or more reference miRNAs, combinations of two reference miRNAs may be selected such that each combination is a preferred combination in which the abundances come close to each other due to deterioration, or a preferred combination in which the abundances get away from each other due to deterioration. The index value(s) may be calculated using all of the three or more reference miRNAs, or the index value(s) may be calculated using only part of the three or more reference miRNAs. For example, when four reference miRNAs A, B, C, and D are used, one possible method is as follows. A difference or a ratio between A and B may be calculated to obtain Index Value 1, and a difference or a ratio between A and C may be calculated to obtain Index Value 2. Each index value may be compared with a threshold for each index value to determine whether it is higher or lower than the threshold. D may be compared with a threshold for D to determine whether it is higher or lower than the threshold (and further, A, B, and C may also be compared individually with their thresholds, respectively, to determine whether they are higher or lower than the thresholds). The results may then be judged as a whole. Another possible method is as follows. A difference or a ratio between A and B may be calculated to obtain Index Value 1, and a difference or a ratio between C and D may be calculated to obtain Index Value 2. Each index value may be compared with a threshold for each index value to determine whether it is higher or lower than the threshold, and the results may then be judged as a whole.

In cases where one reference miRNA is used, the one miRNA may be arbitrarily selected from the miRNAs shown in SEQ ID NOs:1 to 16 and 37 to 61. It is preferred to select a miRNA whose abundance is remarkably altered depending on the storage time. Among the miRNAs shown in the later-described Table 2, Table 4, Table 6, and Table 8, the following 12 miRNAs exhibit 3-fold or greater changes in the abundance, that is, changes in the $\log_2$ value by not less than 1.6, relative to those under the reference condition: hsa-miR-204-3p (SEQ ID NO:1), hsa-miR-4730 (SEQ ID NO:2), hsa-miR-4800-3p (SEQ ID NO:8), hsa-miR-744-5p (SEQ ID NO:9), hsa-miR-6511a-5p (SEQ ID NO:10), hsa-miR-135a-3p (SEQ ID NO:11), hsa-miR-940 (SEQ ID NO:12), hsa-miR-3648 (SEQ ID NO:38), hsa-miR-4497 (SEQ ID NO:40), hsa-miR-4745-5p (SEQ ID NO:41), hsa-miR-92a-2-5p (SEQ ID NO:43), and hsa-miR-6132 (SEQ ID NO:57). Any of these miRNAs may be preferably selected. Further, among these, if miRNAs whose abundances are largely altered depending on the storage time are defined as miRNAs that exhibit 3.6-fold or greater changes in the abundance, that is, changes in the $\log_2$ value by not less than 1.85, relative to those under reference conditions, then the following seven miRNAs correspond to such miRNAs: hsa-miR-204-3p, hsa-miR-4730, hsa-miR-4800-3p, hsa-miR-744-5p, hsa-miR-135a-3p, hsa-miR-940, hsa-miR-4497. Any of these miRNAs may be especially preferably selected.

Also in cases where a plurality of reference miRNAs are used, the reference miRNAs are preferably selected from the 12 miRNAs described above. By using a plurality of reference miRNAs, severer or more highly accurate evaluation can be carried out. It is also preferred to carry out the judgment using a difference or a ratio between two reference miRNAs. In such a case, one miRNA selected from the group consisting of hsa-miR-204-3p, hsa-miR-4730, hsa-miR-135a-3p, hsa-miR-3648, hsa-miR-4497, hsa-miR-4745-5p, and hsa-miR-92a-2-5p, whose abundances increase with deterioration, and one miRNA selected from the group consisting of hsa-miR-204-3p, hsa-miR-4800-3p, hsa-miR-744-5p, hsa-miR-6511a-5p, hsa-miR-940, and hsa-miR-6132, whose abundances decrease with deterioration, are preferably used in combination.

It is more preferred to select a plurality of miRNAs from the above-described seven reference miRNAs whose abundances are especially largely altered with deterioration. In cases where a difference or a ratio between two reference miRNAs is used for the judgment, it is preferred, as described above, to use a combination of one miRNA selected from the group consisting of hsa-miR-204-3p, hsa-miR-4730, hsa-miR-135a-3p, and hsa-miR-4497, whose abundances increase with deterioration, and one miRNA selected from the group consisting of hsa-miR-204-3p, hsa-miR-4800-3p, hsa-miR-744-5p, and hsa-miR-940, whose abundances decrease with deterioration. For example, the combination of hsa-miR-204-3p and hsa-miR-4730, the combination of hsa-miR-204-3p and hsa-miR-4800-3p, or the combination of hsa-miR-744-5p and hsa-miR-4497 may be preferably used. As described above, hsa-miR-204-3p is a miRNA that exhibits either a decreased abundance or an increased abundance depending on in which step of the sample treatment the deterioration has occurred. Thus, in cases where deterioration of a serum sample that has occurred in the whole blood state due to leaving the whole blood to stand under conditions where the temperature is higher than room temperature (for example, at 28°C or higher) for several hours (for example, 6 hours or longer) is to be evaluated by using hsa-miR-204-3p, this miRNA needs to be selected as a miRNA whose abundance decreases with deterioration, while in cases where deterioration that has occurred in the serum state after the serum separation is to be evaluated, this miRNA needs to be selected as a miRNA whose abundance increases with deterioration.

Some reference miRNAs exhibit changes in the abundance even when deterioration of a body fluid sample is mild, and some other reference miRNAs begin to exhibit changes in the abundance when deterioration of a body fluid sample largely proceeds. Thus, it is preferred to select a reference miRNA(s) in accordance with the purpose.

In cases of evaluation of deterioration that has occurred during a standing time of as short as several hours (such as 6 hours) or less in the sample preparation, two miRNAs selected from hsa-miR-204-3p, hsa-miR-4730, hsa-miR-4800-3p, hsa-miR-744-5p, hsa-miR-940, and hsa-miR-4497 are preferably used in combination.

In cases of evaluation of deterioration that has occurred during a standing time of several hours (such as 6 hours) to 1 day in the sample preparation, two miRNAs selected from hsa-miR-204-3p, hsa-miR-4730, hsa-miR-4800-3p, hsa-miR-744-5p, hsa-miR-135a-3p, and hsa-miR-940 are preferably used in combination.

When, for example, gene expression analysis is to be carried out, and a target miRNA in the analysis corresponds to one of the miRNAs of SEQ ID NOs:1 to 16 and 37 to 61, a reference miRNA(s) may be selected from the miRNAs excluding the target miRNA.

The present invention also provides a program(s) for evaluating the quality of a body fluid sample, in accordance with the method of evaluating the quality of a body fluid sample of the present invention, the program(s) causing one or more computers to execute:

a measured value-obtaining step of obtaining a measured value(s) of the abundance(s) of one or more reference miRNAs selected from miRNAs consisting of the base sequences shown in SEQ ID NOs:1 to 16 and 37 to 61 in the body fluid sample, the measured value(s) being a value(s) measured using an RNA sample prepared from the body fluid sample; and

a judging step of judging the quality of the body fluid sample by comparing the abundance(s) of the one or more reference miRNAs, or by comparing an index value(s) calculated from the abundances of the plurality of reference miRNAs, with an arbitrarily predetermined threshold(s)

(that is, a program(s) comprising instructions which cause one or more computers to execute each step described above), and also provides a computer-readable recording medium in which the program is recorded.

For example, the program(s) may be installed in a device for analysis of the expression levels of miRNAs, and a measured value(s) of the abundance(s) of a reference miRNA(s) in a body fluid sample measured by an expression measurement section contained in the device or by an expression measurement device separate from the device may be obtained in the measured value-obtaining step. Each step may then be carried out using the measured value(s). Each measured value obtained may be a corrected measured value. The program(s) may include instructions which cause a computer(s) to execute a process of correcting the measured value obtained. Details of each step are as described above in relation to the method of evaluating the quality of a body fluid sample of the present invention.

The "program" is a data processing method written in an arbitrary language or written by an arbitrary description method, and may be in any format, for example, may be a source code or binary code. The "program" is not limited to a single configuration, and includes a program having a distributed configuration as a plurality of modules and/or libraries, and a program which implements its function in cooperation with a separate program(s) represented by an OS (Operating System). Well-known configurations and procedures may be used as a specific configuration for reading the recording medium, a reading procedure, an installation procedure after the reading, and the like.

The "recording medium" may be an arbitrary "portable physical medium" (non-transient recording medium) such as a flexible disk, magnetic optical disk, ROM, EPROM, EEPROM, CD-ROM, MO, or DVD. Or, the "recording medium" may be a "communication medium" which retains the program(s) for a short period, such as a communication line or a carrier wave used in transmitting the program(s) via a network represented by LAN, WAN, or internet.

The present invention also provides a chip for miRNA quality evaluation, comprising a support on which a probe(s) for capturing one or more reference miRNAs selected from miRNAs consisting of the base sequences shown in SEQ ID NOs:1 to 16 and 37 to 61 is/are immobilized. The present invention also provides a chip for miRNA expression analysis, comprising a support on which a probe(s) for capturing a target miRNA(s) and a probe(s) for capturing one or more reference miRNAs selected from miRNAs consisting of the base sequences shown in SEQ ID NOs:1 to 16 and 37 to 61 are immobilized. The target miRNA(s), the one or more reference miRNAs selected from miRNAs consisting of the base sequences shown in SEQ ID NOs:1 to 16 and 37 to 61, the probes for capturing these, and the support on which these capture probes are immobilized are as described above.

In the chip for miRNA expression analysis of the present invention, a probe(s) for capturing a correcting nucleic acid(s) to be used in the correction step, such as a housekeeping RNA(s), particular correcting endogenous miRNA(s), external standard nucleic acid(s) added, etc., especially a probe(s) for capturing a correcting endogenous miRNA(s), may be further immobilized on the support.

The following are known information and the like on miRNAs consisting of the base sequences shown in SEQ ID NOs:1 to 16 and 37 to 61, which may be used as reference miRNAs in the present invention.

The term "miR-204-3p gene" or "miR-204-3p" used in the present description includes the hsa-miR-204-3p gene described in SEQ ID NO:1, which is a human gene (miRBase Accession No. MIMAT0022693), and its homologues, orthologues and the like in other organism species. The hsa-miR-204-3p gene can be obtained by the method described in Lim LP et al. (2003), Science, vol. 299, p. 1540. As a precursor of "hsa-miR-204-3p", "hsa-mir-204" (miRBase Accession No. MI0000284, SEQ ID NO:17), which has a hairpin-like structure, is known.

The term "miR-4730 gene" or "miR-4730" used in the present description includes the hsa-miR-4730 gene described in SEQ ID NO:2, which is a human gene (miRBase Accession No. MIMAT0019852), and its homologues, orthologues and the like in other organism species. The hsa-miR-4730 gene can be obtained by the method described in Persson H et al. (2011), Cancer Res, vol. 71, pp. 78-86. As a precursor of "hsa-miR-4730", "hsa-mir-4730" (miRBase Accession No. MI0017367, SEQ ID NO:18), which has a hairpin-like structure, is known.

The term "miR-128-2-5p gene" or "miR-128-2-5p" used in the present description includes the hsa-miR-128-2-5p gene described in SEQ ID NO:3, which is a human gene (miRBase Accession No. MIMAT0031095), and its homologues, orthologues and the like in other organism species. The hsa-miR-128-2-5p gene can be obtained by the method described in Lagos-Quintana M et al. (2002), Curr Biol, vol. 12, pp. 735-739. As a precursor of "hsa-miR-128-2-5p", "hsa-mir-128-2" (miRBase Accession No. MI0000727, SEQ ID NO:19), which has a hairpin-like structure, is known.

The term "miR-4649-5p gene" or "miR-4649-5p" used in the present description includes the hsa-miR-4649-5p gene described in SEQ ID NO:4, which is a human gene (miRBase Accession No. MIMAT0019711), and its homologues, orthologues and the like in other organism species. The hsa-miR-4649-5p gene can be obtained by the method described in Persson H et al. (2011), Cancer Res, vol. 71, pp. 78-86. As a precursor of "hsa-miR-4649-5p", "hsa-mir-4649" (miRBase Accession No. MI0017276, SEQ ID NO:20), which has a hairpin-like structure, is known.

The term "miR-6893-5p gene" or "miR-6893-5p" used in the present description includes the hsa-miR-6893-5p gene described in SEQ ID NO:5, which is a human gene (miRBase Accession No. MIMAT0027686), and its homologues, orthologues and the like in other organism species. The hsa-miR-6893-5p gene can be obtained by the method described in Ladewig E et al. (2012), Genome Research, vol. 22, pp.1634-1645. As a precursor of "hsa-miR-6893-5p", "hsa-mir-6893" (miRBase Accession No. MI0022740, SEQ ID NO:21), which has a hairpin-like structure, is known.

The term "miR-187-5p gene" or "miR-187-5p" used in the present description includes the hsa-miR-187-5p gene described in SEQ ID NO:6, which is a human gene (miRBase Accession No. MIMAT0004561), and its homologues, orthologues and the like in other organism species. The hsa-miR-187-5p gene can be obtained by the method described in Lim LP et al. (2003), Science, vol. 299, p. 1540. As a precursor of "hsa-miR-187-5p", "hsa-mir-187" (miRBase Accession No. MI0000274, SEQ ID NO:22), which has a hairpin-like structure, is known.

The term "miR-6076 gene" or "miR-6076" used in the present description includes the hsa-miR-6076 gene described in SEQ ID NO:7, which is a human gene (miRBase Accession No. MIMAT0023701), and its homologues, orthologues and the like in other organism species. The hsa-miR-6076 gene can be obtained by the method described in Voellenkle C et al. (2012), RNA, vol. 18, pp. 472-484. As a precursor of "hsa-miR-6076", "hsa-mir-6076" (miRBase Accession No. MI0020353, SEQ ID NO:23), which has a hairpin-like structure, is known.

The term "miR-4800-3p gene" or "miR-4800-3p" used in the present description includes the hsa-miR-4800-3p gene described in SEQ ID NO:8, which is a human gene (miRBase Accession No. MIMAT0019979), and its homologues, orthologues and the like in other organism species. The hsa-miR-4800-3p gene can be obtained by the method described in Persson H et al. (2011), Cancer Res, vol. 71, pp. 78-86. As a precursor of "hsa-miR-4800-3p", "hsa-mir-4800" (miRBase Accession No. MI0017448, SEQ ID NO:24), which has a hairpin-like structure, is known.

The term "miR-744-5p gene" or "miR-744-5p" used in the present description includes the hsa-miR-744-5p gene described in SEQ ID NO:9, which is a human gene (miRBase Accession No. MIMAT0004945), and its homologues, orthologues and the like in other organism species. The hsa-miR-744-5p gene can be obtained by the method described in Berezikov E et al. (2006), Genome Res, vol. 16, pp. 1289-1298. As a precursor of "hsa-miR-744-5p", "hsa-mir-744" (miRBase Accession No. MI0005559, SEQ ID NO:25), which has a hairpin-like structure, is known.

The term "miR-6511a-5p gene" or "miR-6511a-5p" used in the present description includes the hsa-miR-6511a-5p gene described in SEQ ID NO:10, which is a human gene (miRBase Accession No. MIMAT0025478), and its homologues, orthologues and the like in other organism species. The hsa-miR-6511a-5p gene can be obtained by the method described in Joyce CE et al. (2011), Hum Mol Genet, vol. 20, pp. 4025-4040. As precursors of"hsa-miR-6511a-5p", "hsa-mir-6511a-1, hsa-mir-6511a-2, hsa-mir-6511a-3, and hsa-mir-6511a-4" (miRBase Accession Nos. MI0022223, MI0023564, MI0023565, and MI0023566; SEQ ID NOs:26 to 29), which have hairpin-like structures, are known.

The term "miR-135a-3p gene" or "miR-135a-3p" used in the present description includes the hsa-miR-135a-3p gene described in SEQ ID NO:11, which is a human gene (miRBase Accession No. MIMAT0004595), and its homologues, orthologues and the like in other organism species. The hsa-miR-135a-3p gene can be obtained by the method described in Lagos-Quintana M et al. (2002), Curr Biol, vol. 12, pp. 735-739. As a precursor of "hsa-miR-135a-3p", "hsa-mir-135a" (miRBase Accession No. MI0000452, SEQ ID NO:30), which has a hairpin-like structure, is known.

The term "miR-940 gene" or "miR-940" used in the present description includes the hsa-miR-940 gene described in SEQ ID NO:12, which is a human gene (miRBase Accession No. MIMAT0004983), and its homologues, orthologues and the like in other organism species. The hsa-miR-940 gene can be obtained by the method described in Lui WO et al. (2007), Cancer Res., vol. 67, pp. 6031-6043. As a precursor of "hsa-miR-940", "hsa-mir-940" (miRBase Accession No. MI0005762, SEQ ID NO:31), which has a hairpin-like structure, is known.

The term "miR-4429 gene" or "miR-4429" used in the present description includes the hsa-miR-4429 gene described in SEQ ID NO:13, which is a human gene (miRBase Accession No. MIMAT0018944), and its homologues, orthologues and the like in other organism species. The hsa-miR-4429 gene can be obtained by the method described in Jima DD et al. (2010), Blood, vol. 116, e118-e127. As a precursor of "hsa-miR-4429", "hsa-mir-4429" (miRBase Accession No. MI0016768, SEQ ID NO:32), which has a hairpin-like structure, is known.

The term "miR-6068 gene" or "miR-6068" used in the present description includes the hsa-miR-6068 gene described in SEQ ID NO:14, which is a human gene (miRBase Accession No. MIMAT0023693), and its homologues, orthologues and the like in other organism species. The hsa-miR-6068 gene can be obtained by the method described in Voellenkle C et al. (2012), RNA, vol. 18, pp. 472-484. As a precursor of "hsa-miR-6068", "hsa-mir-6068" (miRBase Accession No. MI0020345, SEQ ID NO:33), which has a hairpin-like structure, is known.

The term "miR-6511b-5p gene" or "miR-6511b-5p" used in the present description includes the hsa-miR-6511b-5p gene described in SEQ ID NO:15, which is a human gene (miRBase Accession No. MIMAT0025847), and its homologues, orthologues and the like in other organism species. The hsa-miR-6511b-5p gene can be obtained by the method described in Li Y et al. (2012), Gene, vol. 497, pp. 330-335. As precursors of "hsa-miR-6511b-5p", "hsa-mir-6511b-1 and hsa-mir-6511b-2" (miRBase Accession Nos. MI0022552 and MI0023431; SEQ ID NOs:34 and 35), which have hairpin-like structures, are known.

The term "miR-885-3p gene" or "miR-885-3p" used in the present description includes the hsa-miR-885-3p gene described in SEQ ID NO:16, which is a human gene (miRBase Accession No. MIMAT0004948), and its homologues, orthologues and the like in other organism species. The hsa-miR-885-3p gene can be obtained by the method described in Berezikov E et al. (2006), Genome Res, vol. 16, pp. 1289-1298. As a precursor of "hsa-miR-885-3p", "hsa-mir-885" (miRBase Accession No. MI0005560, SEQ ID NO:36), which has a hairpin-like structure, is known.

The term "miR-3619-3p gene" or "miR-3619-3p" used in the present description includes the hsa-miR-3619-3p gene described in SEQ ID NO:37 (miRBase Accession No. MIMAT0019219), and its homologues, orthologues and the like in other organism species. The hsa-miR-3619-3p gene can be obtained by the method described in Witten D et al. (2010), BMC Biol, vol. 8, p. 58. As a precursor of "hsa-miR-3619-3p", "hsa-mir-3619" (miRBase Accession No. MI0016009, SEQ ID NO:62), which has a hairpin-like structure, is known.

The term "miR-3648 gene" or "miR-3648" used in the present description includes the hsa-miR-3648 gene described in SEQ ID NO:38 (miRBase Accession No. MIMAT0018068), and its homologues, orthologues and the like in other organism species. The hsa-miR-3648 gene can be obtained by the method described in Meiri E et al. (2010), Nucleic Acids Res, vol. 38, pp. 6234-6246. As a precursor of "hsa-miR-3648", "hsa-mir-3648-1" (miRBase Accession No. MI0016048, SEQ ID NO:63), which has a hairpin-like structure, is known.

The term "miR-4485-5p gene" or "miR-4485-5p" used in the present description includes the hsa-miR-4485-5p gene described in SEQ ID NO:39 (miRBase Accession No. MIMAT0032116), and its homologues, orthologues and the like in other organism species. The hsa-miR-5p gene can be obtained by the method described in Jima DD et al. (2010), Blood, vol. 116, e118-e127. As a precursor of "hsa-miR-4485-5p", "hsa-mir-4485" (miRBase Accession No. MI0016846, SEQ ID NO:64), which has a hairpin-like structure, is known.

The term "miR-4497 gene" or "miR-4497" used in the present description includes the hsa-miR-4497 gene described in SEQ ID NO:40 (miRBase Accession No. MIMAT0019032), and its homologues, orthologues and the like in other organism species. The hsa-miR-4497 gene can be obtained by the method described in Jima DD et al. (2010), Blood, vol. 116, e118-e127. As a precursor of "hsa-miR-4497", "hsa-mir-4497" (miRBase Accession No. MI0016859, SEQ ID NO:65), which has a hairpin-like structure, is known.

The term "miR-4745-5p gene" or "miR-4745-5p" used in the present description includes the hsa-miR-4745-5p gene described in SEQ ID NO:41 (miRBase Accession No. MIMAT0019878), and its homologues, orthologues and the like in other organism species. The hsa-miR-4745-5p gene can be obtained by the method described in Persson H et al. (2011), Cancer Res, vol. 71, pp. 78-86. As a precursor of "hsa-miR-4745-5p", "hsa-mir-4745" (miRBase Accession No. MI0017384, SEQ ID NO:66), which has a hairpin-like structure, is known.

The term "miR-663b gene" or "miR-663b" used in the present description includes the hsa-miR-663b gene described in SEQ ID NO:42 (miRBase Accession No. MIMAT0005867), and its homologues, orthologues and the like in other organism species. The hsa-miR-663b gene can be obtained by the method described in Takada S et al. (2008), Leukemia, vol. 22, pp. 1274-1278. As a precursor of "hsa-miR-663b", "hsa-mir-663b" (miRBase Accession No.

MI0006336, SEQ ID NO:67), which has a hairpin-like structure, is known.

The term "miR-92a-2-5p" or "miR-92a-2-5p" used in the present description includes the hsa-miR-92a-2-5p gene described in SEQ ID NO:43 (miRBase Accession No. MIMAT0004508), and its homologues, orthologues and the like in other organism species. The hsa-miR-92a-2-5p gene can be obtained by the method described in Mourelatos Z et al. (2002), Genes Dev, vol. 16, pp. 720-728. As a precursor of "hsa-miR-92a-2-5p", "hsa-miR-92a-2" (miRBase Accession No. MI0000094, SEQ ID NO:68), which has a hairpin-like structure, is known.

The term "miR-1260b gene" or "miR-1260b" used in the present description includes the hsamiR-1260b gene described in SEQ ID NO:44 (miRBase Accession No. MIMAT0015041), and its homologues, orthologues and the like in other organism species. The hsa-miR-1260b gene can be obtained by the method described in Stark MS et al. (2010), PLoS One, vol. 5, e9685. As a precursor of "hsa-miR-1260b", "hsa-mir-1260b" (miRBase Accession No. MI0014197, SEQ ID NO:69), which has a hairpin-like structure, is known.

The term "miR-3197 gene" or "miR-3197" used in the present description includes the hsa-miR-3197 gene described in SEQ ID NO:45 (miRBase Accession No. MIMAT0015082), and its homologues, orthologues and the like in other organism species. The hsa-miR-3197 gene can be obtained by the method described in Stark MS et al. (2010), PLoS One, vol. 5, e9685. As a precursor of "hsa-miR-3197", "hsa-mir-3197" (miRBase Accession No. MI0014245, SEQ ID NO:70), which has a hairpin-like structure, is known.

The term "miR-3663-3p gene" or "miR-3663-3p" used in the present description includes the hsa-miR-3663-3p gene described in SEQ ID NO:46 (miRBase Accession No. MIMAT0018085), and its homologues, orthologues and the like in other organism species. The hsa-miR-3663-3p gene can be obtained by the method described in Liao JY et al. (2010), PLoS One, vol. 5, e10563. As a precursor of "hsa-miR-3663-3p", "hsa-mir-3663" (miRBase Accession No. MI0016064, SEQ ID NO:71), which has a hairpin-like structure, is known.

The term "miR-4257 gene" or "miR-4257" used in the present description includes the hsa-miR-4257 gene described in SEQ ID NO:47 (miRBase Accession No. MIMAT0016878), and its homologues, orthologues and the like in other organism species. The hsa-miR-4257 gene can be obtained by the method described in Goff LA et al. (2009), PLoS One, vol. 4, e7192. As a precursor of "hsa-miR-4257", "hsa-mir-4257" (miRBase Accession No. MI0015856, SEQ ID NO:72), which has a hairpin-like structure, is known.

The term "miR-4327 gene" or "miR-4327" used in the present description includes the hsa-miR-4327 gene described in SEQ ID NO:48 (miRBase Accession No. MIMAT0016889), and its homologues, orthologues and the like in other organism species. The hsa-miR-4327 gene can be obtained by the method described in Goff LA et al. (2009), PLoS One, vol. 4, e7192. As a precursor of "hsa-miR-4327", "hsa-mir-4327" (miRBase Accession No. M10015867, SEQ ID NO:73), which has a hairpin-like structure, is known.

The term "miR-4476 gene" or "miR-4476" used in the present description includes the hsa-miR-4476 gene described in SEQ ID NO:49 (miRBase Accession No. MIMAT0019003), and its homologues, orthologues and the like in other organism species. The hsa-miR-4476 gene can be obtained by the method described in Jima DD et al. (2010), Blood, vol. 116, e118-e127. As a precursor of "hsa-miR-4476", "hsa-mir-4476" (miRBase Accession No. MI0016828, SEQ ID NO:74), which has a hairpin-like structure, is known.

The term "miR-4505 gene" or "miR-4505" used in the present description includes the hsa-miR-4505 gene described in SEQ ID NO:50 (miRBase Accession No. MIMAT0019041), and its homologues, orthologues and the like in other organism species. The hsa-miR-4505 gene can be obtained by the method described in Jima DD et al. (2010), Blood, vol. 116, e118-e127. As a precursor of "hsa-miR-4505", "hsa-mir-4505" (miRBase Accession No. MI0016868, SEQ ID NO:75), which has a hairpin-like structure, is known.

The term "miR-4532 gene" or "miR-4532" used in the present description includes the hsa-miR-4532 gene described in SEQ ID NO:51 (miRBase Accession No. MIMAT0019071), and its homologues, orthologues and the like in other organism species. The hsa-miR-4532 gene can be obtained by the method described in Jima DD et al. (2010), Blood, vol. 116, e118-e127. As a precursor of "hsa-miR-4532", "hsa-mir-4532" (miRBase Accession No. MI0016899, SEQ ID NO:76), which has a hairpin-like structure, is known.

The term "miR-4674 gene" or "miR-4674" used in the present description includes the hsa-miR-4674 gene described in SEQ ID NO:52 (miRBase Accession No. MIMAT0019756), and its homologues, orthologues and the like in other organism species. The hsa-miR-4674 gene can be obtained by the method described in Persson H et al. (2011), Cancer Res, vol. 71, pp. 78-86. As a precursor of "hsa-miR-4674", "hsa-mir-4674" (miRBase Accession No. MI0017305, SEQ ID NO:77), which has a hairpin-like structure, is known.

The term "miR-4690-5p gene" or "miR-4690-5p" used in the present description includes the hsa-miR-4690-5p gene described in SEQ ID NO:53 (miRBase Accession No. MIMAT0019779), and its homologues, orthologues and the like in other organism species. The hsa-miR-4690-5p gene can be obtained by the method described in Persson H et al. (2011), Cancer Res, vol. 71, pp. 78-86. As a precursor of "hsa-miR-4690-5p", "hsa-mir-4690" (miRBase Accession No. MI0017323, SEQ ID NO:78), which has a hairpin-like structure, is known.

The term "miR-4792 gene" or "miR-4792" used in the present description includes the hsa-miR-4792 gene described in SEQ ID NO:54 (miRBase Accession No. MIMAT0019964), and its homologues, orthologues and the like in other organism species. The hsa-miR-4792 gene can be obtained by the method described in Persson H et al. (2011), Cancer Res, vol. 71, pp. 78-86. As a precursor of "hsa-miR-4792", "hsa-mir-4792" (miRBase Accession No. MI0017439, SEQ ID NO:79), which has a hairpin-like structure, is known.

The term "miR-5001-5p gene" or "miR-5001-5p" used in the present description includes the hsa-miR-5001-5p gene described in SEQ ID NO:55 (miRBase Accession No. MIMAT0021021), and its homologues, orthologues and the like in other organism species. The hsa-miR-5001-5p gene can be obtained by the method described in Hansen TB et al. (2011), RNA Biol, vol. 8, pp. 378-383. As a precursor of "hsa-miR-5001-5p", "hsa-mir-5001" (miRBase Accession No. MI0017867, SEQ ID NO:80), which has a hairpin-like structure, is known.

The term "miR-6075 gene" or "miR-6075" used in the present description includes the hsa-miR-6075 gene described in SEQ ID NO:56 (miRBase Accession No. MIMAT0023700), and its homologues, orthologues and the like in other organism species. The hsa-miR-6075 gene can be obtained by the method described in Voellenkle C et al. (2012), RNA, vol. 18, pp. 472-484. As a precursor of "hsa-miR-6075", "hsa-mir-6075" (miRBase Accession No. MI0020352, SEQ ID NO:81), which has a hairpin-like structure, is known.

The term "miR-6132 gene" or "miR-6132" used in the present description includes the hsa-miR-6132 gene described in SEQ ID NO:57 (miRBase Accession No. MIMAT0024616), and its homologues, orthologues and the like in other organism species. The hsa-miR-6132 gene can be obtained by the method described in Dannemann M et al. (2012), Genome Biol Evol, vol. 4, pp. 552-564. As a precursor of "hsa-miR-6132", "hsa-mir-6132" (miRBase Accession No. MI0021277, SEQ ID NO:82), which has a hairpin-like structure, is known.

The term "miR-6885-5p gene" or "miR-6885-5p" used in the present description includes the hsa-miR-6885-5p gene described in SEQ ID NO:58 (miRBase Accession No. MIMAT0027670), and its homologues, orthologues and the like in other organism species. The hsa-miR-6885-5p gene can be obtained by the method described in Ladewig E et al. (2012), Genome Research, vol. 22, pp.1634-1645. As a precursor of "hsa-miR-6885-5p", "hsa-mir-6885" (miRBase Accession No. MI0022732, SEQ ID NO:83), which has a hairpin-like structure, is known.

The term "miR-6780b-5p gene" or "miR-6780b-5p" used in the present description includes the hsa-miR-6780b-5p gene described in SEQ ID NO:59 (miRBase Accession No. MIMAT0027572), and its homologues, orthologues and the like in other organism species. The hsa-miR-6780b-5p gene can be obtained by the method described in Ladewig E et al. (2012), Genome Research, vol. 22, pp.1634-1645. As a precursor of "hsa-miR-6780b-5p", "hsa-mir-6780b" (miRBase Accession No. MI0022681, SEQ ID NO:84), which has a hairpin-like structure, is known.

The term "miR-4723-5p gene" or "miR-4723-5p" used in the present description includes the hsa-miR-4723-5p gene described in SEQ ID NO:60 (miRBase Accession No. MIMAT0019838), and its homologues, orthologues and the like in other organism species. The hsa-miR-4723-5p gene can be obtained by the method described in Persson H et al. (2011), Cancer Res, vol. 71, pp. 78-86. As a precursor of "hsa-miR-4723-5p", "hsa-mir-4723" (miRBase Accession No. MI0017359, SEQ ID NO:85), which has a hairpin-like structure, is known.

The term "miR-5100 gene" or "miR-5100" used in the present description includes the hsa-miR-5100 gene described in SEQ ID NO:61 (miRBase Accession No. MIMAT0022259), and its homologues, orthologues and the like in other organism species. The hsa-miR-5100 gene can be obtained by the method described in Tandon M et al. (2012), Oral Dis, vol. 18, pp. 127-131. As a precursor of "hsa-miR-5100", "hsa-mir-5100" (miRBase Accession No. MI0019116, SEQ ID NO:86), which has a hairpin-like structure, is known.

## EXAMPLES

The process of selecting the reference miRNAs that exhibit changes depending on the quality of RNA in the present invention is described below more concretely. However, the present invention is not limited to the following Examples.

(Collection of Serum Samples)

In the Examples, serum is selected as an example of the body fluid sample, and the Examples include descriptions related to evaluation of the quality of the body fluid sample. The process of obtaining the serum consists of the following three steps: (1) collection of blood from a subject, (2) coagulation of the blood in the whole-blood state, and (3) separation of serum by centrifugation. Among these, for the (2) leaving of the sample to stand during the coagulation, and for the (3) leaving of the sample to stand during the period between the separation of the serum and cryopreservation, a plurality of conditions were set in terms of the standing time and the temperature, and the following experiments were carried out using serum samples prepared in accordance therewith.

Among Examples 1 to 8, Examples 1, 2, 5, and 6 are related to the (2) leaving of the sample to stand during the coagulation, and Examples 3, 4, 7, and 8 are related to the (3) leaving of the sample to stand during the period between the separation of the serum and cryopreservation. The experiments in Example 5 to 8 employed shorter standing times than in Examples 1 to 4 during the sample preparation. Examples 5 and 6 correspond to Examples 1 and 2, and Examples 7 and 8 correspond to Examples 3 and 4. Table 1 shows sample preparation conditions for Examples 1 to 8.

[Table 1]

| Evaluation item | Example number | Test conditions | |
|---|---|---|---|
| | | Coagulation condition | Condition used from serum separation to cryopreservation |
| Quality change during coagulation | 1, 2 | • Left to stand at 4, 18, 20, 23, 28, or 30°C for 6 hours<br>• Left to stand at 23°C for 0.5, 3, 6, or 9 hours | Stored at -80°C immediately after serum separation |
| | 5,6 | • Left to stand at 24°C for 0.5, 1, or 3 hours<br>• Left to stand at 20, 22, 26, or 28°C for 1 hour | Stored at -80°C immediately after serum separation |
| Quality change after serum separation | 3,4 | Left to stand at room temperature for 0.5 hour | • Left to stand at 4°C for 0, 12, 21, or 24 hours<br>• Left to stand at 23°C for 0.5, 1, 2, 3, or 6 hours<br>• Left to stand at 4, 10, or 14°C for 21 hours |
| | 7,8 | Left to stand at room temperature for 0.5 hour | • Left to stand at 24°C for 0, 1, or 2 hours<br>• Left to stand at 20, 22, 26, or 28°C for 1 hour |

(DNA Microarray)

Using a "3D-Gene" human miRNA oligo chip (which is in accordance with miRBase release 21), manufactured by Toray Industries, Inc., the following experiments of Examples 1 to 8 were carried out.

<Example 1> Selection of Reference miRNAs Capable of Detecting Deterioration That Has Occurred during Whole-Blood Coagulation

(Preparation of Samples for Detecting Deterioration Due to Influence of Temperature)

From each of three healthy individuals, blood was collected into seven blood collection tubes. In the whole-blood state, one out of the seven tubes was left to stand at room temperature (23°C) for 0.5 hour (which condition is referred to as a reference condition), and the remaining six tubes were left to stand at a temperature of 4°C, 18°C, 20°C, room temperature (23°C), 28°C, or 30°C, respectively, for 6 hours. After a lapse of each standing time, centrifugation was performed to obtain serum, and the serum obtained was aliquoted in 300-μL volumes within 10 minutes after the centrifugation, followed by storing the aliquots in a freezer at -80°C.

(Preparation of Samples for Detecting Deterioration Due to Long Standing Time at Room Temperature)

From each of three healthy individuals, blood was collected into four blood collection tubes. In the whole-blood state, one out of the four tubes was left to stand at room temperature (23°C) for 0.5 hour (which condition is referred to as a reference condition), and the remaining three tubes were left to stand similarly at room temperature (23°C), for 3 hours, 6 hours, or 9 hours, respectively. After a lapse of each standing time, centrifugation was performed to obtain serum, and the serum obtained was aliquoted in 300-μL volumes within 10 minutes after the centrifugation, followed by storing the aliquots in a freezer at -80°C.

(Preparation of Sample RNAs and Measurement of miRNA Abundances)

The sera prepared and stored in the freezer as described above were thawed at the same time, and RNA contained in each serum sample (hereinafter referred to as sample RNA) was extracted. For the extraction, a "3D-Gene" RNA extraction reagent from liquid sample kit (manufactured by Toray Industries, Inc.) was used. For purification, an RNeasy 96 QIAcube HT kit (QIAGEN) was used.

Each sample RNA obtained was labeled using a "3D-Gene" miRNA labeling kit (manufactured by Toray Industries, Inc.). In the labeling, an external standard nucleic acid was added for correcting the measured value of miRNA. The labeled sample RNA was subjected to hybridization using a "3D-Gene" miRNA chip (manufactured by Toray Industries, Inc.) according to the manufacturer's standard protocol. The DNA microarray after the hybridization was subjected to a microarray scanner (manufactured by Toray Industries, Inc.) to measure the fluorescence intensity. The following settings for the scanner were used: laser output, 100%; photomultiplier voltage, AUTO.

Each miRNA contained in the sample RNA prepared under each condition was measured with the DNA microarray. The measured value of each miRNA detected was converted to a base-2 logarithm, and an appropriate correction was carried out for standardization of data among the samples, to determine the miRNA abundance in each serum sample.

(Selection of Reference miRNAs)

The miRNA abundances in the serum samples obtained as described above were compared, and miRNAs showing high degrees of changes in the abundance depending on the standing time and/or standing temperature were extracted to select reference miRNAs.

Table 2 shows eight (SEQ ID NOs:1 to 8) reference miRNAs; their average changes, among the individuals, of the abundance under each condition from the abundance under the reference condition; and the overall change index value of miRNA in each sample calculated according to the above-described Equation 1 and Equation 2. These miRNAs exhibited 2-fold or greater changes in the abundance (the difference between the base-2 logarithmic values of the abundances was ≥1) under conditions where samples were left to stand for a long time at room temperature, or left to stand at a temperature of 28°C or higher, that is, conditions where samples were stored in a state where miRNAs in the sera were relatively unstable. In general, in an assay using a DNA microarray, a 2-fold change in the abundance is thought to be a sufficient difference. Further, as the standing temperature (coagulation temperature) of the whole blood increased, or as the standing time at room temperature increased, the overall change index value increased to exhibit a value of as high as 1.5 or more, indicating that the degree of deterioration of the sample quality was high. Thus, it was confirmed that these miRNAs can be used as miRNA indices whose abundances are altered depending on the quality of a body fluid sample. It was thus found that the quality of a body fluid sample can be known by measuring the abundances of the reference miRNAs shown in Table 2.

[Table 2]

Average changes, among individuals, of the expression levels of eight reference miRNAs capable of detecting deterioration that has occurred in the whole-blood state

| SEQ ID NO | Reference miRNA | Reference Condition | Whole blood 6 hours (4°C) | Whole blood 6 hours (18°C) | Whole blood 6 hours (20°C) | Whole blood 6 hours (room temp.) | Whole blood 6 hours (28°C) | Whole blood 6 hours (30°C) | Whole blood 3 hours (room temp.) | Whole blood 6 hours (room temp.) | Whole blood 9 hours (room temp.) |
|---|---|---|---|---|---|---|---|---|---|---|---|
| 1 | hsa-miR-204-3p | 0 | 0.2 | 0.7 | 0.6 | 0.6 | **-1.1** | **-1.4** | 0.3 | 0.1 | -0.4 |
| 2 | hsa-miR-4730 | 0 | -0.1 | 0.6 | 0.7 | 0.7 | **1.0** | **1.3** | 0.7 | **1.1** | **1.4** |
| 3 | hsa-miR-128-2-5p | 0 | -0.1 | 0.5 | 0.6 | 0.6 | 0.6 | **1.0** | 0.5 | 0.6 | 0.8 |
| 4 | hsa-miR-4649-5p | 0 | 0.0 | 0.5 | 0.5 | 0.6 | 0.7 | **1.2** | 0.4 | 0.5 | 0.8 |
| 5 | hsa-miR-6893-5p | 0 | 0.3 | 0.4 | 0.2 | 0.1 | -0.9 | **-1.0** | 0.0 | -0.4 | -0.6 |
| 6 | hsa-miR-187-5p | 0 | -0.2 | 0.4 | 0.6 | 0.6 | 0.9 | **1.0** | 0.4 | 0.7 | **1.0** |
| 7 | hsa-miR-6076 | 0 | -0.1 | 0.2 | -0.1 | -0.1 | **-1.0** | **-1.2** | 0.1 | -0.2 | -0.8 |
| 8 | hsa-miR-4800-3p | 0 | -0.2 | -0.3 | -0.2 | -0.1 | -0.4 | -0.2 | -0.5 | -0.7 | **-1.1** |
| Overall change index value | | - | 1.2 | 1.4 | 1.4 | 1.4 | **1.5** | **1.5** | 1.3 | 1.4 | **1.6** |

Fig. 3 shows the abundances of hsa-miR-204-3p (SEQ ID NO:1) under the reference condition, and under the conditions where different coagulation temperatures were applied to samples in the whole-blood state (seven conditions in total). The abundance of hsa-miR-204-3p sharply decreased at the coagulation temperature of 28°C or higher. For example, in cases where deterioration of the quality of a body fluid sample caused by leaving the sample to stand in the whole-blood state at 28°C or higher is to be judged, the threshold of the abundance of hsa-miR-204-3p may be set to 12, and, when the abundance of hsa-miR-204-3p in a body fluid sample is lower than this value, the sample may be judged to be deteriorated, that is, to have poor quality.

Fig. 4 shows the abundances of hsa-miR-4730 (SEQ ID NO:2) under the reference condition, and under the conditions where different standing times were applied to samples in the whole-blood state at room temperature (four conditions in total). The abundance of hsa-miR-4730 increased as the standing time at room temperature in the whole-blood state increased. For example, in cases where deterioration of the quality of a body fluid sample caused by leaving the sample to stand in the whole-blood state for 6 hours or longer is to be judged, the threshold of the abundance of hsa-miR-4730 may be set to 11, and, when the abundance of hsa-miR-4730 in a body fluid sample is higher than this value, the sample may be judged to be deteriorated, that is, to have poor quality.

Specific examples of the thresholds of the eight reference miRNAs shown in Table 2, which can be set based on the results of the present Example 1, are shown in Table 3 below together with the average abundances under the reference condition. These thresholds can be used as thresholds for detection of deterioration that has occurred in the whole-blood state, for example, during storage as a whole blood. For example, these thresholds may be preferably used in cases where a long time was required before separation of serum from a clinical blood sample. After measuring a reference miRNA(s) in each body fluid sample whose quality is to be evaluated, each measured value may be converted to a base-2 logarithm, and an appropriate correction may be carried out for standardization of data among samples, followed by comparing the resulting value with its threshold. Depending on how severely the judgement is carried out, the thresholds shown in Table 3 $\pm \alpha$ (wherein $\alpha$ is an arbitrary value which may be, for example, about 0.5 to 3) may be set as thresholds.

[Table 3]

| Examples of the thresholds of eight reference miRNAs capable of detecting deterioration that has occurred in the whole-blood state | | | | | |
|---|---|---|---|---|---|
| SEQ ID NO | Reference miRNA | Abundance under reference condition (average) | Threshold | Change upon deterioration | Judgment criterion |
| 1 | hsa-miR-204-3p | 12.7 | 12.0 | Decrease | Lower abundance indicates poor quality |
| 2 | hsa-miR-4730 | 10.0 | 11.0 | Increase | Higher abundance indicates poor quality |
| 3 | hsa-miR-128-2-5p | 9.3 | 10.0 | Increase | Higher abundance indicates poor quality |
| 4 | hsa-miR-4649-5p | 9.0 | 9.9 | Increase | Higher abundance indicates poor quality |
| 5 | hsa-miR-6893-5p | 9.1 | 8.2 | Decrease | Lower abundance indicates poor quality |
| 6 | hsa-miR-187-5p | 8.4 | 9.0 | Increase | Higher abundance indicates poor quality |
| 7 | hsa-miR-6076 | 7.2 | 6.3 | Decrease | Lower abundance indicates poor quality |
| 8 | hsa-miR-4800-3p | 6.8 | 6.2 | Decrease | Lower abundance indicates poor quality |

<Example 2> Detection of Deterioration During Whole-Blood Coagulation Based on Plurality of miRNAs

It is also possible to judge deterioration of the quality of a body fluid sample using a combination of two arbitrary kinds of reference miRNAs instead of using a single miRNA.

The abundances of hsa-miR-204-3p (SEQ ID NO:1) and hsa-miR-4730 (SEQ ID NO:2) under the reference condition in Example 1 and under the condition where samples were left to stand in the whole-blood state at 30°C for 6 hours were used. The abundances of these miRNAs under each condition were as shown in Fig. 5. The difference between the abundances of these two miRNAs were calculated for each condition, and the result of the calculation is shown in Fig. 6. As shown in Table 3 and Fig. 5, hsa-miR-204-3p is a miRNA that exhibits a decreased abundance due to sample deterioration that has occurred in the whole-blood state, and hsa-miR-4730 is a miRNA that exhibits an increased abundance due to sample deterioration that has occurred in the whole-blood state. hsa-miR-204-3p is more abundant than hsa-miR-4730 in a non-deteriorated sample. In a body fluid sample in a state with a good quality (under the reference condition), the difference between the abundance of hsa-miR-204-3p and the abundance of hsa-miR-4730 is large, whereas, in a body fluid sample in a state where the quality has been deteriorated by leaving the sample to stand at 30°C, the difference between their abundances becomes small. In cases where deterioration of the quality of a body fluid sample caused by leaving the sample to stand in the whole-blood state at 30°C is to be judged, the threshold of the difference between the abundances of these two miRNAs may be, for example, set to 1, and, when the difference between the abundances of these miRNAs in a body fluid sample is smaller than this value, the sample may be judged to be deteriorated, that is, to have poor quality.

In cases where similar judgment is carried out using a combination other than the combination of hsa-miR-204-3p (SEQ ID NO:1) and hsa-miR-4730 (SEQ ID NO:2), two reference miRNAs may be selected from the reference miRNAs shown in Table 3 by selecting one reference miRNA from those that exhibit decreased abundances and one reference miRNA from those that exhibit increased abundances. In the case of a combination in which, under the reference condition, the abundance of the reference miRNA that exhibits a decrease is higher than the abundance of the reference miRNA that exhibits an increase, their abundances come close to each other due to deterioration. Thus, when using such a combination, the quality can be judged to be poor if the difference between their abundances is lower than an arbitrarily determined threshold, as in the case of Fig. 6. Conversely, in the case of a combination in which, under the reference condition, the abundance of the reference miRNA that exhibits a decrease is lower than the abundance of the reference miRNA that exhibits an increase, their abundances get away from each other due to deterioration. Thus, when using such a combination, the quality can be judged to be poor if the difference between their abundances is higher than an arbitrarily determined threshold.

In the case of a combination in which, under the reference condition, the abundance of the reference miRNA that exhibits a decrease is higher than the abundance of the reference miRNA that exhibits an increase, the abundance of the former miRNA may become lower than the abundance of the latter reference miRNA when the degree of deterioration is very high, so that the difference in the abundance may begin to increase again. Thus, in general, it is more preferred to select two reference miRNAs to provide a combination in which, under the reference condition, the abundance of the reference miRNA that exhibits a decrease is lower than the abundance of the reference miRNA that exhibits an increase, so that their abundances get away from each other due to deterioration. However, the combination of reference miRNAs is not limited to those mentioned in the present Example. For example, only a plurality of reference miRNAs that exhibit decreased abundances, or only a plurality of reference miRNAs that exhibit increased abundances, may be selected from Table 3 and combined, and the judgment results obtained by the individual reference miRNAs may be evaluated as a whole to judge whether the quality of the body fluid sample is good or poor.

<Example 3> Selection of Reference miRNAs Capable of Detecting Deterioration That Has Occurred in Serum State

(Preparation of Samples for Detecting Deterioration Due to Long Standing Time at 4°C in Serum State (Preparation 1))

From each of three healthy individuals, blood was collected into four blood collection tubes. All tubes were left to stand at room temperature (23°C) for 0.5 hour, and then centrifuged to obtain sera. The obtained serum in one tube was centrifuged, and aliquoted in 300-μL volumes within 10 minutes after the centrifugation, followed by storage in a freezer at -80°C (which condition is referred to as a reference condition). The obtained sera in the remaining three tubes were left to stand at 4°C for 12 hours, 21 hours, or 24 hours, respectively. After a lapse of each standing time, each serum was aliquoted in 300-μL volumes, and stored in a freezer at -80°C.

(Preparation of Samples for Detecting Deterioration Due to Standing in Serum State (Preparation 2))

From each of three healthy individuals, blood was collected into seven blood collection tubes. All tubes were left to stand at room temperature (23°C) for 0.5 hour, and then centrifuged to obtain sera. The obtained serum in one tube was centrifuged, and aliquoted in 300-μL volumes within 10 minutes after the centrifugation, followed by storage in a freezer at -80°C (which condition is referred to as a reference condition). The obtained sera in the remaining six tubes were left to stand for 0.5 hour, 1 hour, 2 hours, 3 hours, or 6 hours at room temperature (23°C), or at 4°C for 6 hours, respectively. After a lapse of each standing time, each serum was aliquoted in 300-μL volumes, and stored in a freezer at -80°C.

(Preparation of Samples for Detecting Deterioration Due to Influence of Temperature in Serum State (Preparation 3))

From each of three healthy individuals, blood was collected into four blood collection tubes. All tubes were left to stand at room temperature (23°C) for 0.5 hour, and then centrifuged to obtain sera. From one of the tubes, the obtained serum was aliquoted in 300-μL volumes within 10 minutes after the centrifugation, and then stored in a freezer at -80°C (which condition is referred to as a reference condition). The obtained sera in the remaining three tubes were left to stand at a temperature of 4°C, 10°C, or 14°C for 21 hours, respectively, and then aliquoted in 300-μL volumes, followed by storage in a freezer at -80°C.

(Preparation of Sample RNAs and Measurement of miRNA Abundances)

The sera prepared and left to stand in the freezer as described above were thawed at the same time, and RNAs contained in the serum samples (hereinafter referred to as sample RNAs) were extracted. For the extraction, a "3D-Gene" RNA extraction reagent from liquid sample kit (manufactured by Toray Industries, Inc.) was used. For purification, an RNeasy 96 QIAcube HT kit (QIAGEN) was used.

Each sample RNA obtained was labeled using a "3D-Gene" miRNA labeling kit (manufactured by Toray Industries, Inc.). In the labeling, an external standard nucleic acid was added for correcting the measured value of miRNA. The labeled sample RNA was subjected to hybridization using a "3D-Gene" miRNA chip (manufactured by Toray Industries, Inc.) according to the manufacturer's standard protocol. The DNA microarray after the hybridization was subjected to a microarray scanner (manufactured by Toray Industries, Inc.) to measure the fluorescence intensity. The following settings for the scanner were used: laser output, 100%; photomultiplier voltage, AUTO.

Each miRNA contained in the sample RNA prepared under each condition was measured with the DNA microarray. The measured value of each miRNA detected was converted to a base-2 logarithm, and an appropriate correction was carried out for standardization of data among the samples, to determine the miRNA abundance in each serum sample.

(Selection of Reference miRNAs)

The miRNA abundances in the serum samples obtained as described above were compared, and miRNAs showing high degrees of changes in the abundance depending on the standing time and/or temperature were extracted to select reference miRNAs.

Table 4 shows 15 reference miRNAs with which deterioration that has occurred during standing of the serum can be detected; their average changes, among the individuals, of the abundance under each condition from the abundance under the reference condition; and the overall change index value of miRNA in each sample calculated according to the above-described Equation 1 and Equation 2. These 15 miRNAs (SEQ ID NOs:1 to 5 and 7 to 16) exhibited 2-fold or greater changes in the abundance (the difference between the base-2 logarithmic values of the abundances was ≥1) under conditions where samples were left to stand for a long time at room temperature or left to stand for a long time at a temperature of 10°C or higher after the serum separation, that is, conditions where samples were stored in a state where miRNAs in the sera were relatively unstable. In general, in an assay using a DNA microarray, a 2-fold change in the abundance is thought to be a sufficient difference. Further, as the standing temperature of serum increased, or as the standing time at the refrigeration temperature (4°C) or at room temperature increased, the overall change index value increased to exhibit a value of as high as 1.5 or more, indicating that the degree of deterioration of the body fluid sample quality was high. Thus, it was confirmed that the miRNAs can be used as miRNA indices whose abundances are altered depending on the quality of a body fluid sample. It was thus found that the quality of a body fluid sample can be known by measuring the abundances of the 15 miRNAs shown in Table 4.

[Table 4]

Average changes, among individuals, of the expression levels of 15 reference miRNAs capable of detecting deterioration that has occurred in the serum state

| SEQ ID NO | Reference miRNA | Reference Condition | Prep. 2 Serum (room temp.) 0.5 hour | Prep. 2 Serum (room temp.) 1 hour | Prep. 2 Serum (room temp.) 2 hours | Prep. 2 Serum (room temp.) 3 hours | Prep. 2 Serum (room temp.) 6 hours | Prep. 2 Serum (4°C) 6 hours | Prep. 1 Serum (4°C) 12 hours | Prep. 1 Serum (4°C) 21 hours | Prep. 1 Serum (4°C) 24 hours | Prep. 3 Serum (4°C) 21 hours | Prep. 3 Serum (10°C) 21 hours | Prep. 3 Serum (14°C) 21 hours |
|---|---|---|---|---|---|---|---|---|---|---|---|---|---|---|
| 1 | hsa-miR-204-3p | 0 | 0.1 | 0.5 | 0.8 | **2.0** | **1.5** | 0.1 | 0.6 | **1.0** | **1.1** | **1.0** | **1.3** | **1.9** |
| 2 | hsa-miR-4730 | 0 | 0.1 | 0.6 | 0.9 | **1.9** | **1.8** | 0.2 | 0.7 | **1.1** | **1.2** | 0.7 | 0.9 | **1.3** |
| 3 | hsa-miR-128-2-5p | 0 | 0.2 | 0.5 | 0.9 | **1.4** | **1.4** | 0.5 | 0.4 | 0.7 | 0.8 | 0.5 | 0.6 | **1.2** |
| 4 | hsa-miR-4649-5p | 0 | 0.2 | 0.4 | 0.8 | **1.3** | **1.5** | 0.5 | 0.4 | 0.6 | 0.7 | 0.5 | 0.7 | **1.3** |
| 5 | hsa-miR-6893-5p | 0 | 0.1 | 0.2 | 0.4 | 0.7 | -0.1 | 0.0 | 0.2 | 0.4 | 0.6 | 0.7 | **1.0** | **1.2** |
| 7 | hsa-miR-6076 | 0 | 0.1 | 0.1 | 0.1 | **1.1** | 0.6 | 0.0 | 0.0 | 0.2 | 0.1 | 0.4 | 0.7 | **1.1** |
| 8 | hsa-miR-4800-3p | 0 | -0.5 | **-1.4** | **-2.0** | -0.9 | **-2.1** | **-1.3** | **-2.0** | **-2.3** | **-2.4** | **-2.0** | **-2.2** | **-2.3** |
| 9 | hsa-miR-744-5p | 0 | -0.6 | **-1.3** | **-1.5** | -0.4 | -0.9 | -0.4 | -0.7 | -0.9 | **-1.1** | -0.8 | **-1.2** | **-1.5** |
| 10 | hsa-miR-6511a-5p | 0 | -0.4 | **-1.2** | **-1.5** | -0.4 | **-1.0** | -0.2 | -0.7 | **-1.0** | **-1.2** | **-1.0** | **-1.4** | **-1.8** |
| 11 | hsa-miR-135a-3p | 0 | 0.1 | 0.4 | 0.6 | **2.2** | **2.2** | 0.0 | -0.4 | -0.2 | -0.4 | -0.2 | -0.1 | 0.7 |
| 12 | hsa-miR-940 | 0 | **-1.0** | **-1.6** | **-1.8** | **-1.0** | **-1.1** | **-1.3** | **-1.4** | **-1.3** | **-1.5** | **-1.3** | **-1.5** | **-1.4** |
| 13 | hsa-miR-4429 | 0 | -0.4 | **-1.2** | **-1.4** | -0.5 | **-1.1** | -0.2 | -0.7 | -0.9 | **-1.0** | -0.9 | **-1.3** | **-1.5** |
| 14 | hsa-miR-6068 | 0 | -0.4 | **-1.1** | **-1.3** | -0.9 | **-1.2** | -0.4 | -0.7 | -0.9 | **-1.1** | **-1.1** | **-1.4** | **-1.6** |
| 15 | hsa-miR-6511b-5p | 0 | 0.0 | -0.6 | -0.8 | -0.4 | -0.9 | 0.2 | -0.5 | -0.7 | -0.8 | -0.8 | **-1.1** | **-1.4** |
| 16 | hsa-miR-885-3p | 0 | -0.6 | **-1.0** | **-1.2** | -0.5 | -0.8 | -0.9 | -0.8 | -0.8 | -0.9 | -0.9 | **-1.0** | **-1.1** |
| Overall change index value | | - | 1.2 | 1.4 | **1.5** | **3.1** | **2.2** | 1.3 | 1.4 | **1.5** | **1.6** | **1.5** | **1.7** | **2.0** |

Fig. 7 shows the abundances of hsa-miR-4800-3p (SEQ ID NO:8) under the reference condition, and under the conditions where different standing times and temperatures were applied to samples in the serum state (eight conditions in total). The abundance of hsa-miR-4800-3p (SEQ ID NO:8) decreased as the degree of deterioration increased. For example, in cases where deterioration of the quality of a sample caused by leaving the sample to stand at 4°C for 6 hours or longer, or by leaving the sample to stand at a temperature of 10°C or higher for 21 hours is to be judged, the threshold of the abundance of hsa-miR-4800-3p may be set to 6.2, and, when the abundance of hsa-miR-4800-3p in a body fluid sample is lower than this value, the sample may be judged to be deteriorated, that is, to have poor quality.

Fig. 8 shows the abundances of hsa-miR-135a-3p (SEQ ID NO:11) under the reference condition, and under the conditions where different standing times were applied to samples at room temperature in the serum state (six conditions in total). As the standing time of hsa-miR-135a-3p (SEQ ID NO:11) at room temperature in the serum state increased, its abundance increased. For example, in cases where deterioration of the quality of a body fluid sample caused by leaving the sample to stand in the serum state at room temperature for 3 hours or longer is to be judged, the threshold of the abundance of hsa-miR-135a-3p may be set to 7.7, and, when the abundance of hsa-miR-135a-3p in a body fluid sample is higher than this value, the sample may be judged to be deteriorated, that is, to have poor quality.

Specific examples of the thresholds of the 15 reference miRNAs shown in Table 4, which can be set based on the results of the present Example 3, are shown in Table 5 below together with the average abundances in the reference samples. These thresholds can be used as thresholds for detection of deterioration that has occurred in the serum state, for example, during storage as a serum. For example, these thresholds may be preferably used in cases where a long time was required during the period between separation of serum from a clinical blood sample and cryopreservation, or during the period of keeping of the separated serum without freezing until expression analysis. After measuring a reference miRNA(s) in each body fluid sample whose quality is to be evaluated, each measured value may be converted to a base-2 logarithm, and an appropriate correction may be carried out for standardization of data among samples, followed by comparing the resulting value with its threshold. Depending on how severely the judgement is carried out, the thresholds shown in Table 5 $\pm$ $\alpha$ (wherein $\alpha$ is an arbitrary value which may be, for example, about 0.5 to 3) may be set as thresholds.

[Table 5]

| Examples of the thresholds of 15 reference miRNAs capable of detecting deterioration that has occurred in the serum state | | | | | |
|---|---|---|---|---|---|
| SEQ ID NO | Reference miRNA | Abundance under reference condition (average) | Threshold | Change upon deterioration | Judgment criterion |
| 1 | hsa-miR-204-3p | 12.7 | 13.7 | Increase | Higher abundance indicates poor quality |

(continued)

| Examples of the thresholds of 15 reference miRNAs capable of detecting deterioration that has occurred in the serum state | | | | | |
|---|---|---|---|---|---|
| SEQ ID NO | Reference miRNA | Abundance under reference condition (average) | Threshold | Change upon deterioration | Judgment criterion |
| 2 | hsa-miR-4730 | 10.0 | 11.1 | Increase | Higher abundance indicates poor quality |
| 3 | hsa-miR-128-2-5p | 9.3 | 10.5 | Increase | Higher abundance indicates poor quality |
| 4 | hsa-miR-4649-5p | 9.0 | 10.1 | Increase | Higher abundance indicates poor quality |
| 5 | hsa-miR-6893-5p | 9.1 | 9.3 | Increase | Higher abundance indicates poor quality |
| 7 | hsa-miR-6076 | 7.2 | 7.3 | Increase | Higher abundance indicates poor quality |
| 8 | hsa-miR-4800-3p | 6.8 | 6.2 | Decrease | Lower abundance indicates poor quality |
| 9 | hsa-miR-744-5p | 9.1 | 8.8 | Decrease | Lower abundance indicates poor quality |
| 10 | hsa-miR-6511 a-5p | 8.5 | 8.5 | Decrease | Lower abundance indicates poor quality |
| 11 | hsa-miR-135a-3p | 6.4 | 7.7 | Increase | Higher abundance indicates poor quality |
| 12 | hsa-miR-940 | 8.2 | 7.6 | Decrease | Lower abundance indicates poor quality |
| 13 | hsa-miR-4429 | 7.5 | 7.1 | Decrease | Lower abundance indicates poor quality |
| 14 | hsa-miR-6068 | 6.5 | 5.7 | Decrease | Lower abundance indicates poor quality |
| 15 | hsa-miR-6511b-5p | 6.3 | 5.9 | Decrease | Lower abundance indicates poor quality |
| 16 | hsa-miR-885-3p | 6.3 | 6.0 | Decrease | Lower abundance indicates poor quality |

<Example 4> Detection of Deterioration of Serum Based on Plurality of miRNAs

It is also possible to judge deterioration of the quality of a body fluid sample using, more preferably, a combination of two arbitrary kinds miRNAs instead of using a single miRNA.

The abundances of hsa-miR-204-3p (SEQ ID NO:1) and hsa-miR-4800-3p (SEQ ID NO:8) under the reference condition in Example 3 and under the condition where samples were left to stand in the serum state at 4°C for 24 hours were used. The abundances of these miRNAs under each condition were as shown in Fig. 9. The difference between the abundances of these two miRNAs were calculated for each condition, and the result of the calculation is shown in Fig. 10. As shown in Table 5 and Fig. 9, hsa-miR-204-3p is a miRNA that exhibits an increased abundance due to sample deterioration that has occurred in the serum state, and hsa-miR-4800-3p is a miRNA that exhibits a decreased abundance due to sample deterioration that has occurred in the serum state. hsa-miR-204-3p is more abundant than hsa-miR-4800-3p in a non-deteriorated sample. In a body fluid sample in a state with a good quality (under the reference condition), the difference between the abundance of hsa-miR-204-3p and the abundance of hsa-miR-4800-3p is small, whereas, in a body fluid sample in a state where the sample has been deteriorated by being left to stand at 4°C for 24 hours, the difference between their abundances becomes large. In cases where deterioration of the quality of a body fluid sample caused by leaving the sample to stand at 4°C for 24 hours is to be judged, the threshold of the difference between the abundances of these two miRNAs may be, for example, set to 8, and, when the difference between the abundances of these miRNAs in a body fluid sample is larger than this value, the sample may be judged to be deteriorated, that is, to have poor quality.

In cases where similar judgment is carried out using a combination other than the combination of hsa-miR-204-3p (SEQ ID NO:1) and hsa-miR-4800-3p (SEQ ID NO:8), two reference miRNAs may be selected from the reference miRNAs shown in Table 5 by selecting one reference miRNA from those that exhibit decreased abundances and one reference miRNA from those that exhibit increased abundances. In the case of a combination in which, under the reference condition, the abundance of the reference miRNA that exhibits a decrease is lower than the abundance of the reference miRNA that exhibits an increase, their abundances get away from each other due to deterioration. Thus, when using such a combination, the quality can be judged to be poor if the difference between their abundances is larger than an arbitrarily determined threshold, as in the case of Fig. 10. Conversely, in the case of a combination in which, under the reference condition, the abundance of the reference miRNA that exhibits a decrease is higher than the abundance of the reference miRNA that exhibits an increase, their abundances come close to each other due to deterioration. Thus, when using such a combination, the quality can be judged to be poor if the difference between their abundances is smaller than an arbitrarily determined threshold.

As explained in Example 2, in general, it is more preferred to select two reference miRNAs to provide a combination in which, under the reference condition, the abundance of the reference miRNA that exhibits a decrease is lower than the abundance of the reference miRNA that exhibits an increase, so that their abundances get away from each other due to deterioration. However, the combination of reference miRNAs is not limited to those mentioned in the present Example. For instance, only a plurality of reference miRNAs that exhibit decreased abundances, or only a plurality of reference miRNAs that exhibit increased abundances, may be selected from Table 5 and combined, and the judgment results obtained by the individual reference miRNAs may be evaluated as a whole to judge whether the quality of the body fluid sample is good or poor (whether or not deterioration occurred in the serum state).

## <Example 5> Selection of Reference miRNAs Capable of Detecting Deterioration That Has Occurred during Whole-Blood Coagulation

### (Sample Preparation)

From each of three healthy individuals, blood was collected into seven blood collection tubes. In the whole-blood state, one out of the seven tubes was left to stand at room temperature (24°C) for 0.5 hour (which condition is referred to as a reference condition), and the remaining six tubes were left to stand at a temperature of 20°C, 22°C, room temperature (24°C), 26°C, or 28°C for 1 hour, or at room temperature (24°C) for 3 hours, respectively. After a lapse of each standing time, centrifugation was performed to obtain serum, and the serum obtained was aliquoted in 300-$\mu$L volumes within 10 minutes after the centrifugation, followed by storing the aliquots in a freezer at -80°C.

### (Preparation of Sample RNAs and Measurement of miRNA Abundances, and Selection of Reference miRNAs)

The same procedure as in Example 1 was carried out, except that the purification was carried out using UNIFILTER 96 Well (GE Healthcare).

Table 6 shows twelve (SEQ ID NOs:1 to 4, 37 to 43, and 59) reference miRNAs; their average changes, among the individuals, of the abundance under each condition from the abundance under the reference condition; and the overall change index value of miRNA in each sample calculated according to the above-described Equation 1 and Equation 2. For comparison with the reference miRNAs, three miRNAs that do not exhibit changes in the abundance due to sample deterioration are shown in the same table. Among the reference miRNAs, miRNAs whose abundances increased exhibited 2-fold or greater changes in the abundance (the difference between the base-2 logarithmic values of the abundances was $\geq$1), and a miRNA whose abundance decreased exhibited a 1.5-fold change in the abundance (the difference between the base-2 logarithmic values of the abundances was $\geq$0.6), under the condition where samples were left to stand at room temperature for the longest period, 3 hours, that is, condition where samples were stored in a state where miRNAs in the sera were relatively unstable. In general, in an assay using a DNA microarray, a 2-fold change in the abundance is thought to be a sufficient difference. Further, as the standing temperature (coagulation temperature) of the whole blood increased, or as the standing time at room temperature increased, the overall change index value increased, indicating that the degree of deterioration of the sample quality was high. Thus, it was confirmed that these miRNAs can be used as miRNA indices whose abundances are altered depending on the quality of a body fluid sample. It was thus found that the quality of a body fluid sample can be known by measuring the abundances of the reference miRNAs shown in Table 6.

[Table 6]

| SEQ ID NO | Reference miRNA | Reference Condition | Whole blood 1 hour (20°C) | Whole blood 1 hour (22°C) | Whole blood 1 hour (24°C) | Whole blood 1 hour (26°C) | Whole blood 1 hour (28°C) | Whole blood 3 hours (24°C) |
|---|---|---|---|---|---|---|---|---|
| 1 | hsa-miR-204-3p | 0 | 0.0 | 0.2 | 0.5 | 0.5 | 0.6 | 1.2 |
| 2 | hsa-miR-4730 | 0 | 0.2 | 0.4 | 0.6 | 0.7 | 0.8 | 1.5 |
| 3 | hsa-miR-128-2-5p | 0 | 0.2 | 0.4 | 0.4 | 0.4 | 0.4 | 1.0 |
| 4 | hsa-miR-4649-5p | 0 | 0.3 | 0.4 | 0.4 | 0.4 | 0.4 | 1.0 |
| 37 | hsa-miR-3619-3p | 0 | 0.3 | 0.3 | 0.2 | 0.4 | 0.5 | 1.0 |
| 38 | hsa-miR-3648 | 0 | 0.0 | 0.1 | 0.3 | 0.6 | 0.8 | 1.3 |
| 39 | hsa-miR-4485-5p | 0 | 0.0 | 0.1 | 0.8 | 1.0 | 1.1 | 1.1 |
| 40 | hsa-miR-4497 | 0 | 0.0 | 0.2 | 0.5 | 0.6 | 0.6 | 1.2 |
| 41 | hsa-miR-4745-5p | 0 | 0.0 | 0.2 | 0.4 | 0.6 | 0.6 | 1.1 |
| 42 | hsa-miR-663b | 0 | 0.3 | 0.4 | 0.5 | 0.4 | 0.3 | 1.1 |
| 43 | hsa-miR-92a-2-5p | 0 | 0.0 | 0.2 | 0.4 | 0.8 | 1.0 | 1.1 |
| 59 | hsa-miR-6780b-5p | 0 | -0.2 | -0.1 | -0.1 | -0.2 | -0.2 | -0.6 |
| Comp. 1 | hsa-miR-3180-3p | 0 | -0.1 | 0.0 | -0.1 | 0.0 | 0.0 | 0.0 |
| Comp. 2 | hsa-miR-4726-5p | 0 | 0.1 | 0.1 | 0.1 | 0.1 | 0.3 | 0.2 |
| Comp. 3 | hsa-miR-4632-5p | 0 | 0.0 | -0.1 | -0.1 | -0.1 | -0.1 | -0.3 |
| Overall change index value | | - | 1.3 | 1.4 | 1.4 | 1.4 | 1.4 | 1.6 |

Fig. 11 shows the abundances of hsa-miR-3648 (SEQ ID NO:38) under the reference condition, and under the conditions where different coagulation temperatures and times were applied to samples in the whole-blood state (seven conditions in total). The abundance of hsa-miR-3648 increased as the coagulation temperature increased, and as the coagulation time increased. For example, in cases where deterioration of the quality of a body fluid sample caused by leaving the sample to stand in the whole-blood state for 3 hours or longer is to be judged, the threshold of the abundance of hsa-miR-3648 may be set to 11.6, and, when the abundance of hsa-miR-3648 in a body fluid sample is higher than this value, the sample can be judged to be deteriorated, that is, to have poor quality.

Fig. 12 shows the abundances of hsa-miR-4632-5p (comparison 3) under the reference condition, and under the conditions where different coagulation temperatures and times were applied to samples in the whole-blood state (seven conditions in total). Since the changes in the abundance due to sample deterioration are very small, setting of a threshold is difficult. Thus, such a miRNA is inappropriate for detection of sample deterioration.

Specific examples of the thresholds of the twelve reference miRNAs shown in Table 6, which can be set based on the results of the present Example 5, are shown in Table 7 below together with the average abundances under the reference condition. These thresholds can be used as thresholds for detection of deterioration that has occurred in the whole-blood state, for example, during storage as a whole blood. For example, these thresholds may be preferably used in cases where a long time was required before separation of serum from a clinical blood sample. After measuring a reference miRNA(s) in each body fluid sample whose quality is to be evaluated, each measured value may be converted to a base-2 logarithm, and an appropriate correction may be carried out for standardization of data among samples, followed by comparing the resulting value with its threshold. Depending on how severely the judgement is carried out, the thresholds shown in Table 7 ± α (wherein α is an arbitrary value which may be, for example, about 0.5 to 3) may be set as thresholds.

[Table 7]

| Examples of the thresholds of 12 reference miRNAs capable of detecting quality change that has occurred in a short time in the whole-blood state | | | | | |
|---|---|---|---|---|---|
| SEQ ID NO | Reference miRNA | Abundance under reference condition (average) | Threshold | Change upon deterioration | Judgment criterion |
| 1 | hsa-miR-204-3p | 13.4 | 14.5 | Increase | Higher abundance indicates poor quality |
| 2 | hsa-miR-4730 | 9.1 | 10,4 | | |
| 3 | hsa-miR-128-2-5p | 8.7 | 9.6 | | |
| 4 | hsa-miR-4649-5p | 8.5 | 9.4 | | |
| 37 | hsa-miR-3619-3p | 6.4 | 6.9 | | |
| 38 | hsa-miR-3648 | 10.4 | 11.6 | | |
| 39 | hsa-miR-4485-5p | 6.6 | 7.2 | | |
| 40 | hsa-miR-4497 | 12.0 | 13.0 | | |
| 41 | hsa-miR-4745-5p | 10.7 | 11.6 | | |
| 42 | hsa-miR-663b | 6:1 | 6.9 | | |
| 43 | hsa-miR-92a-2-5p | 7.5 | 8.5 | | |
| 59 | hsa-miR-6780b-5p | 10.9 | 10.2 | Decrease | Lower abundance indicates poor quality |

<Example 6> Detection of Deterioration during Whole-Blood Coagulation Based on Plurality of miRNAs

It is also possible to judge deterioration of the quality of a body fluid sample using a combination of two arbitrary kinds of reference miRNAs instead of using a single miRNA.

The abundances of hsa-miR-3648 (SEQ ID NO:38) and hsa-miR-6780b-5p (SEQ ID NO:59) under the reference condition in Example 5 and under the condition where samples were left to stand in the whole-blood state at room temperature (24°C) for 3 hours were used. The abundances of these miRNAs under each condition were as shown in Fig. 13. The difference between the abundances of these two miRNAs were calculated for each condition, and the result of the calculation is shown in Fig. 14. As shown in Table 7 and Fig. 13, hsa-miR-3648 is a miRNA that exhibits an increased abundance due to sample deterioration that has occurred in the whole-blood state, and hsa-miR-6780b-5p is a miRNA that exhibits a decreased abundance due to sample deterioration that has occurred in the whole-blood state. The abundance of hsa-miR-6780b-5p is higher than the abundance of hsa-miR-3648 in a non-deteriorated sample, but, as the deterioration proceeds, reversal of the abundance occurs and hsa-miR-3648 becomes more abundant. In a body fluid sample in a state with a good quality (under the reference condition), a negative value is obtained when the abundance of hsa-miR-6780b-5p is subtracted from the abundance of hsa-miR-3648, whereas, in a body fluid sample whose quality has been deteriorated due to standing at room temperature, the difference between the abundances increases to a positive value. In cases where deterioration of the quality of a body fluid sample caused by leaving the sample to stand in the whole-blood state at room temperature for 3 hours or longer is to be judged, the threshold of the difference between the abundances of these two miRNAs may be, for example, set to 1, and, when the difference between the abundances of these miRNAs in a body fluid sample is larger than this value, the sample may be judged to be deteriorated, that is, to have poor quality.

In cases where similar judgment is carried out using a combination other than the combination of hsa-miR-3648 (SEQ ID NO:38) and hsa-miR-6780b-5p (SEQ ID NO:59), two reference miRNAs may be selected from the reference miRNAs shown in Table 7 by selecting one reference miRNA from those that exhibit decreased abundances and one reference miRNA from those that exhibit increased abundances. However, the combination of reference miRNAs is not limited to those mentioned in the present Example. For instance, only a plurality of reference miRNAs that exhibit increased abundances may be selected from Table 7 and combined, and the judgment results obtained by the individual reference miRNAs may be evaluated as a whole to judge whether the quality of the body fluid sample is good or poor (whether or not deterioration occurred in a short time in the whole-blood state).

<Example 7> Selection of Reference miRNAs Capable of Detecting Deterioration That Has Occurred in Serum State

(Sample Preparation)

From each of three healthy individuals, blood was collected into eight blood collection tubes. All tubes were left to stand at room temperature (23°C) for 0.5 hour, and then centrifuged to obtain sera. The obtained serum in one tube was centrifuged, and aliquoted in 300-μL volumes within 10 minutes after the centrifugation, followed by storage in a freezer at -80°C (reference condition). The obtained sera in the remaining seven tubes were left to stand at room temperature (24°C) for 0.5 hour; at 20°C, 22°C, room temperature (24°C), 26°C, or 28°C for 1 hour; or at room temperature (24°C) for 2 hours, respectively. After a lapse of each standing time, each serum was aliquoted in 300-μL volumes, and stored in a freezer at -80°C.

(Preparation of Sample RNAs and Measurement of miRNA Abundances, and Selection of Reference miRNAs)

The same procedure as in Example 3 was carried out, except that the purification was carried out using UNIFILTER 96 Well (GE Healthcare).

Table 8 shows thirty-four (SEQ ID NOs:1 to 5, 8 to 10, 12, 13, 16, 37, 38, 40 to 58, 60, and 61) reference miRNAs; their average changes, among the individuals, of the abundance under each condition from the abundance under the reference condition; and the overall change index value of miRNA in each sample calculated according to the above-described Equation 1 and Equation 2. These miRNAs exhibited 2-fold or greater changes in the abundance (the difference between the base-2 logarithmic values of the abundances was ≥1) under conditions where samples were left to stand for a long time at room temperature, or left to stand at a temperature of 28°C or higher, that is, conditions where samples were stored in a state where miRNAs in the sera were relatively unstable. In general, in an assay using a DNA microarray, a 2-fold change in the abundance is thought to be a sufficient difference. Further, as the standing temperature (coagulation temperature) of the whole blood increased, or as the standing time at room temperature increased, the overall change index value increased, indicating that the degree of deterioration of the sample quality was high. Thus, it was confirmed that these miRNAs can be used as miRNA indices whose abundances are altered depending on the quality of a body fluid sample. It was thus found that the quality of a body fluid sample can be known by measuring the abundances of the reference miRNAs shown in Table 8.

[Table 8]

| SEQ ID NO | Reference miRNA | Reference Condition | Serum 0.5 hour (24°C) | Serum 1 hour (20°C) | Serum 1 hour (22°C) | Serum 1 hour (24°C) | Serum 1 hour (26°C) | Serum 1 hour (28°C) | Serum 2 hours (24°C) |
|---|---|---|---|---|---|---|---|---|---|
| 1 | hsa-miR-204-3p | 0 | 0.8 | 1.0 | 1.0 | 1.3 | 1.6 | 1.9 | 2.1 |
| 2 | hsa-miR-4730 | 0 | 0.7 | 0.8 | 0.9 | 1.3 | 1.4 | 1.8 | 1.8 |
| 3 | hsa-miR-128-2-5p | 0 | 0.3 | 0.5 | 0.5 | 0.7 | 0.7 | 1.1 | 1.1 |
| 4 | hsa-miR-4649-5p | 0 | 0.2 | 0.4 | 0.5 | 0.6 | 0.7 | 1.0 | 1.0 |
| 5 | hsa-miR-6893-5p | 0 | 0.4 | 0.2 | 0.4 | 0.6 | 0.7 | 0.9 | 1.0 |
| 8 | hsa-miR-4800-3p | 0 | -0.3 | -1.0 | -0.8 | -0.9 | -1.3 | -1.5 | -1.7 |
| 9 | hsa-miR-744-5p | 0 | -0.7 | -0.8 | -1.0 | -1.4 | -1.6 | -1.9 | -1.9 |
| 10 | hsa-miR-6511a-5p | 0 | -0.4 | -0.7 | -0.7 | -1.0 | -1.3 | -1.4 | -1.3 |
| 12 | hsa-miR-940 | 0 | -1.2 | -1.6 | -1.6 | -1.7 | -1.9 | -1.7 | -1.7 |
| 13 | hsa-miR-4429 | 0 | -0.4 | -0.6 | -0.8 | -1.0 | -1.2 | -1.0 | -1.2 |
| 16 | hsa-miR-885-3p | 0 | -0.8 | -0.9 | -0.9 | -0.9 | -1.1 | -1.2 | -0.8 |
| 37 | hsa-miR-3619-3p | 0 | 0.5 | 0.8 | 0.9 | 1.0 | 1.2 | 1.2 | 1.5 |
| 38 | hsa-miR-3648 | 0 | 0.3 | 0.3 | 0.5 | 0.7 | 1.0 | 1.7 | 1.5 |
| 40 | hsa-miR-4497 | 0 | 0.7 | 0.9 | 0.9 | 1.2 | 1.5 | 2.0 | 2.0 |
| 41 | hsa-miR-4745-5p | 0 | 0.4 | 0.6 | 0.6 | 0.9 | 1.2 | 1.8 | 1.6 |
| 42 | hsa-miR-663b | 0 | 0.3 | 0.8 | 0.8 | 0.9 | 1.0 | 1.2 | 1.4 |
| 43 | hsa-miR-92a-2-5p | 0 | 0.7 | 0.6 | 0.7 | 1.1 | 1.5 | 1.8 | 1.7 |
| 44 | hsa-miR-1260b | 0 | -1.1 | -1.1 | -1.0 | -1.0 | -1.1 | -1.0 | -0.7 |
| 45 | hsa-miR-3197 | 0 | 0.4 | 0.5 | 0.6 | 0.8 | 0.8 | 1.1 | 1.0 |
| 46 | hsa-miR-3663-3p | 0 | 0.5 | 0.6 | 0.7 | 1.0 | 1.1 | 1.5 | 1.4 |
| 47 | hsa-miR-4257 | 0 | -0.6 | -0.6 | -0.6 | -0.8 | -1.0 | -1.2 | -1.0 |
| 48 | hsa-miR-4327 | 0 | -0.4 | -0.6 | -0.7 | -0.9 | -1.0 | -1.2 | -1.1 |
| 49 | hsa-miR-4476 | 0 | 0.4 | 0.5 | 0.5 | 0.8 | 0.9 | 1.1 | 1.2 |
| 50 | hsa-miR-4505 | 0 | -0.5 | -0.6 | -0.7 | -0.9 | -1.1 | -1.4 | -1.2 |
| 51 | hsa-miR-4532 | 0 | 0.2 | 0.5 | 0.5 | 0.7 | 0.8 | 1.0 | 1.1 |
| 52 | hsa-miR-4674 | 0 | 0.2 | 0.4 | 0.4 | 0.6 | 0.7 | 0.9 | 1.0 |
| 53 | hsa-miR-4690-5p | 0 | -0.5 | -0.6 | -0.6 | -0.7 | -0.9 | -1.0 | -0.7 |
| 54 | hsa-miR-4792 | 0 | 0.2 | 0.3 | 0.4 | 0.6 | 0.6 | 0.9 | 1.0 |
| 55 | hsa-miR-5001-5p | 0 | -0.5 | -0.6 | -0.6 | -0.7 | -0.9 | -1.0 | -0.8 |
| 56 | hsa-miR-6075 | 0 | -0.5 | -0.8 | -0.7 | -0.8 | -1.0 | -1.0 | -0.9 |
| 57 | hsa-miR-6132 | 0 | -0.6 | -0.8 | -0.9 | -1.3 | -1.6 | -1.7 | -1.7 |
| 58 | hsa-miR-6885-5p | 0 | 0.3 | 0.5 | 0.5 | 0.7 | 0.7 | 1.1 | 1.0 |
| 60 | hsa-miR-4723-5p | 0 | -0.5 | -0.9 | -0.8 | -0.9 | -1.1 | -1.2 | -1.1 |
| 61 | hsa-miR-5100 | 0 | -0.5 | -0.6 | -0.6 | -0.7 | -0.9 | -0.9 | -1.0 |
| Overall change index value | | - | 1.5 | 1.6 | 1.6 | 1.7 | 1.8 | 2.2 | 2.0 |

Fig. 15 shows the abundances of hsa-miR-4497 (SEQ ID NO:40) under the reference condition, and under the conditions where different standing times and temperatures were applied to samples in the serum state (eight conditions in total). The abundance of hsa-miR-4497 (SEQ ID NO:40) increased as the degree of deterioration increased. For example, in cases where deterioration of the quality of a sample caused by leaving the sample to stand at 28°C for 1 hour or longer, or by leaving the sample to stand at 24°C for 2 hours is to be judged, the threshold of the abundance of hsa-miR-4497 may be set to 13.6, and, when the abundance of hsa-miR-4497 in a body fluid sample is higher than this value, the sample may be judged to be deteriorated, that is, to have poor quality.

Fig. 16 shows the abundances of hsa-miR-744-5p (SEQ ID NO:9) under the reference condition, and under the conditions where different standing times and temperatures were applied to samples in the serum state (eight conditions in total). The abundance of hsa-miR-744-5p (SEQ ID NO:9) increased as the degree of deterioration increased. For example, in cases where deterioration of the quality of a body fluid sample caused by leaving the sample to stand in the serum state at room temperature for 2 hours or longer is to be judged, the threshold of the abundance of hsa-miR-744-5p may be set to 8.1, and, when the abundance of hsa-miR-744-5p in a body fluid sample is lower than this value, the sample may be judged to be deteriorated, that is, to have poor quality.

Specific examples of the thresholds of the 34 reference miRNAs shown in Table 8, which can be set based on the results of the present Example 7, are shown in Table 9 below together with the average abundances in the reference samples. These thresholds can be used as thresholds for detection of deterioration that has occurred in the serum state, for example, during storage as a serum. For example, these thresholds may be preferably used in cases where a long time was required during the period between separation of serum from a clinical blood sample and cryopreservation, or during the period of keeping of the separated serum without freezing until expression analysis. After measuring a reference miRNA(s) in each body fluid sample whose quality is to be evaluated, each measured value may be converted to a base-2 logarithm, and an appropriate correction may be carried out for standardization of data among samples, followed by comparing the resulting value with its threshold. Depending on how severely the judgement is carried out, the thresholds shown in Table 9 $\pm \alpha$ (wherein $\alpha$ is an arbitrary value which may be, for example, about 0.5 to 3) may be set as thresholds.

[Table 9]

| Examples of the thresholds of 34 reference miRNAs capable of detecting deterioration that has occurred in a short time in the serum state | | | | | |
|---|---|---|---|---|---|
| SEQ ID NO | Reference miRNA | Abundance under reference condition (average) | Threshold | Change upon deterioration | Judgment criterion |
| 1 | hsa-miR-204-3p | 13.3 | 15.2 | Increase | Higher abundance indicates poor quality |
| 2 | hsa-miR-4730 | 9.3 | 11.0 | Increase | Higher abundance indicates poor quality |
| 3 | hsa-miR-128-2-5p | 8.8 | 9.6 | Increase | Higher abundance indicates poor quality |
| 4 | hsa-miR-4649-5p | 8.6 | 9.5 | Increase | Higher abundance indicates poor quatity |
| 5 | hsa-miR-6893-5p | 9.9 | 10.8 | Increase | Higher abundance indicates poor quality |
| 8 | hsa-miR-4800-3p | 6.6 | 5.3 | Decrease | Lower abundance indicates poor quality |
| 9 | hsa-miR-744-5p | 9.8 | 8.1 | Decrease | Lower abundance indicates poor quality |
| 10 | hsa-miR-6511a-5p | 7.9 | 6.9 | Decrease | Lower abundance indicates poor quality |
| 12 | hsa-miR-940 | 7.6 | 6.2 | Decrease | Lower abundance indicates poor quality |
| 13 | hsa-miR-4429 | 6.9 | 5.9 | Decrease | Lower abundance indicates poor quality |
| 16 | hsa-miR-885-3p | 6.4 | 5.8 | Decrease | Lower abundance indicates poor quality |

(continued)

| Examples of the thresholds of 34 reference miRNAs capable of detecting deterioration that has occurred in a short time in the serum state | | | | | |
|---|---|---|---|---|---|
| SEQ ID NO | Reference miRNA | Abundance under reference condition (average) | Threshold | Change upon deterioration | Judgment criterion |
| 37 | hsa-miR-3619-3p | 6.6 | 7.8 | Increase | Higher abundance indicates poor quality |
| 38 | hsa-miR-3648 | 10.6 | 11.8 | Increase | Higher abundance indicates poor quality |
| 40 | hsa-miR-4497 | 11.9 | 13.7 | Increase | Higher abundance indicates poor quality |
| 41 | hsa-miR-4745-5p | 10.6 | 12.0 | Increase | Higher abundance indicates poor quality |
| 42 | hsa-miR-663b | 6.2 | 7.2 | Increase | Higher abundance indicates poor quality |
| 43 | nsa-miR-92a-2-5p | 7.4 | 8.9 | Increase | Higher abundance indicates poor quality |
| 44 | hsa-miR-1260b | 11.4 | 11.4 | Decrease | Lower abundance indicates poor quality |
| 45 | hsa-miR-3197 | 10.7 | 11.6 | Increase | Higher abundance indicates poor quality |
| 46 | hsa-miR-3663-3p | 10.0 | 11.3 | Increase | Higher abundence indicates poor quality |
| 47 | hsa-miR-4257 | 8.1 | 7.4 | Decrease | Lower abundance indicates poor quality |
| 48 | hsa-miR-4327 | 9.8 | 8.7 | Decrease | Lower abundance indicates poor quality |
| 49 | hsa-miR-4476 | 7.1 | 8.1 | Increase | Higher abundance indicates poor quality |
| 50 | hsa-miR-4505 | 10.8 | 9.7 | Decrease | Lower abundance indicates poor quality |
| 51 | hsa-miR-4532 | 10.8 | 11.7 | Increase | Higher abundance indicates poor quality |

(continued)

| Examples of the thresholds of 34 reference miRNAs capable of detecting deterioration that has occurred in a short time in the serum state | | | | | |
|---|---|---|---|---|---|
| SEQ ID NO | Reference miRNA | Abundance under reference condition (average) | Threshold | Change upon deterioration | Judgment criterion |
| 52 | hsa-miR-4674 | 9.5 | 10.2 | Increase | Higher abundance indicates poor quality |
| 53 | hsa-miR-4690-5p | 7.4 | 6.9 | Decrease | Lower abundance indicates poor quality |
| 54 | hsa-miR-4792 | 6.7 | 7.5 | Increase | Higher abundance indicates poor quality |
| 55 | hsa-miR-5001-5p | 9.5 | 8.8 | Decrease | Lower abundance indicates poor quality |
| 56 | hsa-miR-6075 | 9.9 | 9.3 | Decrease | Lower abundance indicates poor quality |
| 57 | hsa-miR-6132 | 10.3 | 8.7 | Decrease | Lower abundance indicates poor quality |
| 58 | hsa-miR-6885-5p | 9.4 | 10.2 | Increase | Higher abundance indicates poor quality |
| 60 | hsa-miR-4723-5p | 9.2 | 8.2 | Decrease | Lower abundance indicates poor quality |
| 61 | hsa-miR-5100 | 12.1 | 11.6 | Decrease | Lower abundance indicates poor quality |

<Example 8> Detection of Deterioration of Serum That Has Occurred in Short Time Based on Plurality of miRNAs

It is also possible to judge deterioration of the quality of a body fluid sample using a combination of two arbitrary kinds of reference miRNAs instead of using a single miRNA.

The abundances of hsa-miR-4497 (SEQ ID NO:40) and hsa-miR-744-5p (SEQ ID NO:9) under the reference condition in Example 7 and under the condition where samples were left to stand in the serum state at room temperature (24°C) for 2 hours were used. The abundances of these miRNAs under each condition were as shown in Fig. 17. The difference between the abundances of these two miRNAs were calculated for each condition, and the result of calculation is shown in Fig. 18. As shown in Table 9 and Fig. 17, hsa-miR-4497 is a miRNA that exhibits an increased abundance due to sample deterioration that has occurred in the serum state, and hsa-miR-744-5p is a miRNA that exhibits a decreased abundance due to sample deterioration that has occurred in the serum state. hsa-miR-4497 is more abundant than hsa-miR-744-5p in a non-deteriorated sample. In a body fluid sample in a state with a good quality (under the reference condition), the difference between the abundance of hsa-miR-4497 and the abundance of hsa-miR-744-5p is small, whereas, in a body fluid sample in a state where the quality has been deteriorated by being left to stand at 24°C for 2 hours, the difference between their abundances becomes large. In cases where deterioration of the quality of a body fluid sample caused by leaving the sample to stand in the serum state at 24°C is to be judged, the threshold of the difference between the abundances of these two miRNAs may be, for example, set to 4, and, when the difference between the abundances of these miRNAs in a body fluid sample is larger than this value, the sample may be judged to be deteriorated, that is, to have poor quality.

In cases where similar judgment is carried out using a combination other than the combination of hsa-miR-4497 (SEQ ID NO:40) and hsa-miR-744-5p (SEQ ID NO:9), two reference miRNAs may be selected from the reference miRNAs shown in Table 9 by selecting one reference miRNA from those that exhibit decreased abundances and one reference miRNA from those that exhibit increased abundances. In the case of a combination in which, under the reference condition, the abundance of the reference miRNA that exhibits a decrease is higher than the abundance of the reference miRNA that exhibits an increase, their abundances come close to each other due to deterioration. Thus, when using such a combination, the quality can be judged to be poor if the difference between their abundances is smaller than an arbitrarily determined threshold, as in the case of Fig. 6. Conversely, in the case of a combination in which, under the reference condition, the abundance of the reference miRNA that exhibits a decrease is lower than the abundance of the reference miRNA that exhibits an increase, their abundances get away from each other due to deterioration. Thus, when using such a combination, the quality can be judged to be poor if the difference between their abundances is larger than an arbitrarily determined threshold. However, the combination of reference miRNAs is not limited to those mentioned in the present Example. For instance, only a plurality of reference miRNAs that exhibit decreased abundances, or only a plurality of reference miRNAs that exhibit increased abundances, may be selected from Table 9 and combined, and the judgment results obtained by the individual reference miRNAs may be evaluated as a whole to judge whether the quality of the body fluid sample is good or poor (whether or not deterioration occurred in a short time in the serum state).

<Example 9> Detection of Deterioration of Serum Samples, by Quantitative RT-PCR

(Preparation of Samples for Detecting Deterioration Due to Long Standing Time at 4°C in Serum State)

From each of two healthy individuals, blood was collected into two blood collection tubes. All tubes were left to stand at room temperature (24°C) for 0.5 hour, and then centrifuged to obtain sera. The obtained serum in one tube was aliquoted in 300-μL volumes within 10 minutes after the centrifugation, followed by storage in a freezer at -80°C (which condition is referred to as a reference condition). The obtained serum in the remaining one tube was left to stand at 4°C for 24 hours. After a lapse of the standing time, the serum was aliquoted in 300-μL volumes, and stored in a freezer at -80°C.

(Preparation of Sample RNAs and Measurement of miRNA Abundances)

The sera prepared and stored in the freezer as described above were thawed at the same time, and RNA contained in each serum sample (hereinafter referred to as sample RNA) was extracted. For the extraction, a "3D-Gene" RNA extraction reagent from liquid sample kit (manufactured by Toray Industries, Inc.) was used. For purification, UNIFILTER 96 Well (GE Healthcare) was used.

The RNAs from the two individuals, each of which was placed under the two conditions, were subjected to measurement of the abundance of hsa-miR-204-3p (SEQ ID NO:1) using TaqMan (registered trademark) Small RNA Assays (Life Technologies) according to the manufacturer's protocol. In addition, a dilution series was prepared using a standard substance of hsa-miR-204-3p, and a calibration curve was prepared therewith. Based on the resulting Ct value and the calibration curve, the concentration of hsa-miR-204-3p under each condition was calculated.

Fig. 19 shows the abundances of hsa-miR-204-3p (SEQ ID NO:1) under the reference condition, and under the condition where the sample was left to stand in the serum state at 4°C for 24 hours. The abundance of hsa-miR-204-3p increased as the degree of deterioration increased. For example, in cases where deterioration of the quality of a sample caused by leaving the sample to stand at 4°C for 24 hours or longer is to be judged, the threshold of the abundance of hsa-miR-204-3p may be set to 0.002 atto mole/μL, and, when the abundance of hsa-miR-204-3p in a body fluid sample is higher than this value, the sample may be judged to be deteriorated, that is, to have poor quality.

## Claims

1. A method of evaluating the quality of a body fluid sample, the method comprising:

   a measuring step of measuring the abundance(s) of one or more reference miRNAs selected from miRNAs consisting of the base sequences shown in SEQ ID NOs: 1 to 16 and 37 to 61 in the body fluid sample; and
   a judging step of judging the quality of the body fluid sample by comparing the abundance(s) of the one or more reference miRNAs obtained in the measuring step, or by comparing an index value(s) calculated from the abundances of the plurality of reference miRNAs, with an arbitrarily predetermined threshold(s).

2. The method according to claim 1, wherein the index value is a difference or ratio between the abundances of two arbitrarily selected reference miRNAs.

3. The method according to claim 1 or 2, wherein:

each of the miRNAs consisting of the base sequences shown in SEQ ID NOs: 1, 5, and 7 is a miRNA which indicates poor quality of the body fluid sample in a case where the abundance in the body fluid sample is higher than a first threshold or lower than a second threshold;
each of the miRNAs consisting of the base sequences shown in SEQ ID NOs: 2, 3, 4, 6, 11, 37 to 43, 45, 46, 49, 51, 52, 54, and 58 is a miRNA which indicates poor quality of the body fluid sample in a case where the abundance in the body fluid sample is higher than a threshold; and
each of the miRNAs consisting of the base sequences shown in SEQ ID NOs: 8, 9, 10, 12 to 16, 44, 47, 48, 50, 53, 55 to 57, and 59 to 61 is a miRNA which indicates poor quality of the body fluid sample in a case where the abundance in the body fluid sample is lower than a threshold.

4. The method according to any one of claims 1 to 3, wherein the measuring step is a step of carrying out hybridization by bringing a probe(s) for capturing one or more reference miRNAs selected from miRNAs consisting of the base sequences shown in SEQ ID NOs:1 to 16 and 37 to 61, the probe(s) being immobilized on a support, into contact with a nucleic acid sample which is derived from the body fluid sample and labeled with a labeling substance, to measure the abundance(s) of the one or more reference miRNAs in the body fluid sample.

5. The method according to any one of claims 1 to 4, further comprising a correction step of correcting the measured value(s) of the abundance(s) of the one or more reference miRNAs obtained in the measuring step, wherein the judging step is carried out using the corrected value(s) of the abundance(s).

6. The method according to any one of claims 1 to 5, wherein the measuring step comprises measuring the abundance(s) of a target miRNA(s) in the body fluid sample at the same time as the measurement of the abundance(s) of the one or more reference miRNAs in the body fluid sample.

7. The method according to claim 6, wherein the measuring step is a step of carrying out hybridization by bringing a probe(s) for capturing a target miRNA(s) and a probe(s) for capturing one or more reference miRNAs selected from miRNAs consisting of the base sequences shown in SEQ ID NOs:1 to 16 and 37 to 61, the probes being immobilized on a support, into contact with a nucleic acid sample which is derived from the body fluid sample and labeled with a labeling substance, to measure the abundance of each of the target miRNA(s) and the one or more reference miRNAs in the body fluid sample.

8. The method according to claim 6 or 7, further comprising a correction step of correcting the measured value(s) of the abundance(s) of the target miRNA(s) and the measured value(s) of the abundance(s) of the one or more reference miRNAs in the body fluid sample, obtained in the measuring step.

9. The method according to any one of claims 1 to 8, wherein the body fluid sample is whole blood, serum, or plasma.

10. A program(s) for evaluating the quality of a body fluid sample, said program(s) causing one or more computers to execute:

a measured value-obtaining step of obtaining a measured value(s) of the abundance(s) of one or more reference miRNAs selected from miRNAs consisting of the base sequences shown in SEQ ID NOs:1 to 16 and 37 to 61 in the body fluid sample, the measured value(s) being a value(s) measured using an RNA sample prepared from the body fluid sample; and
a judging step of judging the quality of the body fluid sample by comparing the abundance(s) of the one or more reference miRNAs, or by comparing an index value(s) calculated from the abundances of the plurality of reference miRNAs, with an arbitrarily predetermined threshold(s).

11. A computer-readable recording medium in which the program(s) according to claim 10 is recorded.

12. A chip for miRNA quality evaluation, comprising a support on which a probe(s) for capturing one or more reference miRNAs selected from miRNAs consisting of the base sequences shown in SEQ ID NOs:1 to 16 and 37 to 61 is/are immobilized.

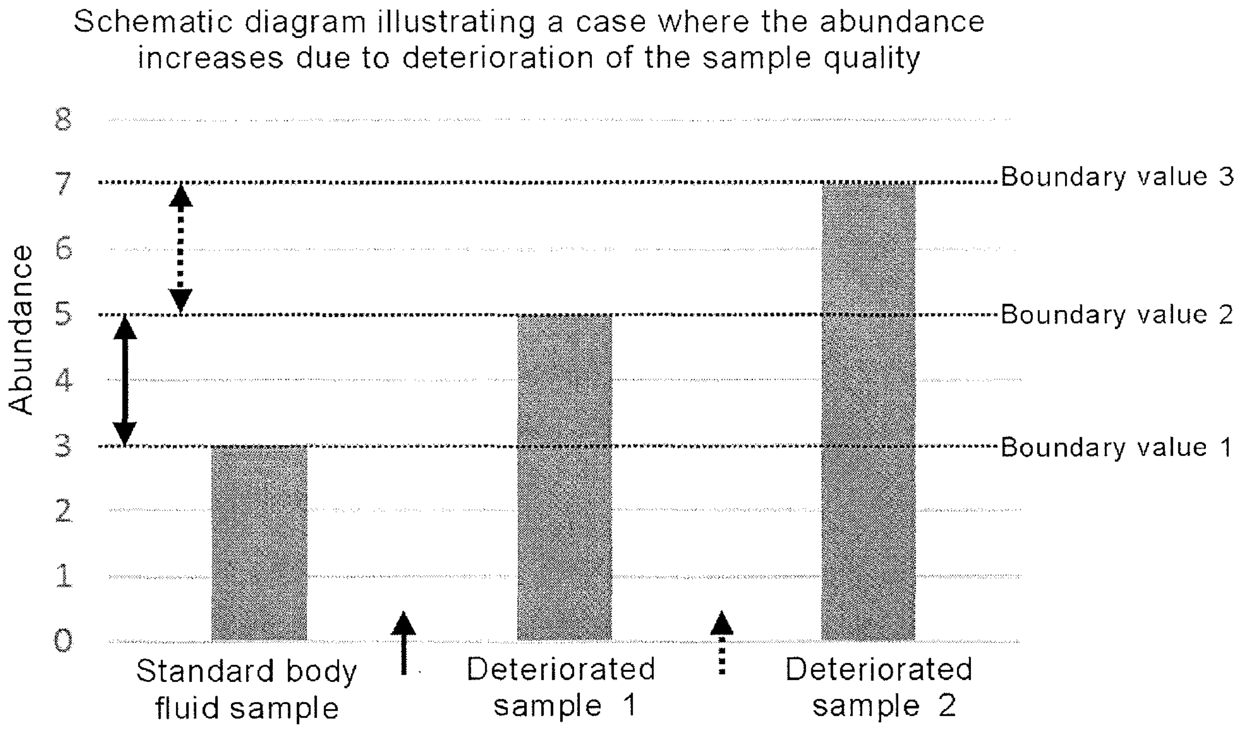
Schematic diagram illustrating a case where the abundance increases due to deterioration of the sample quality
Abundance
0
1
2
3
4
5
6
7
8
Boundary value 3
Boundary value 2
Boundary value 1
Standard body fluid sample
Deteriorated sample 1
Deteriorated sample 2

Fig.1

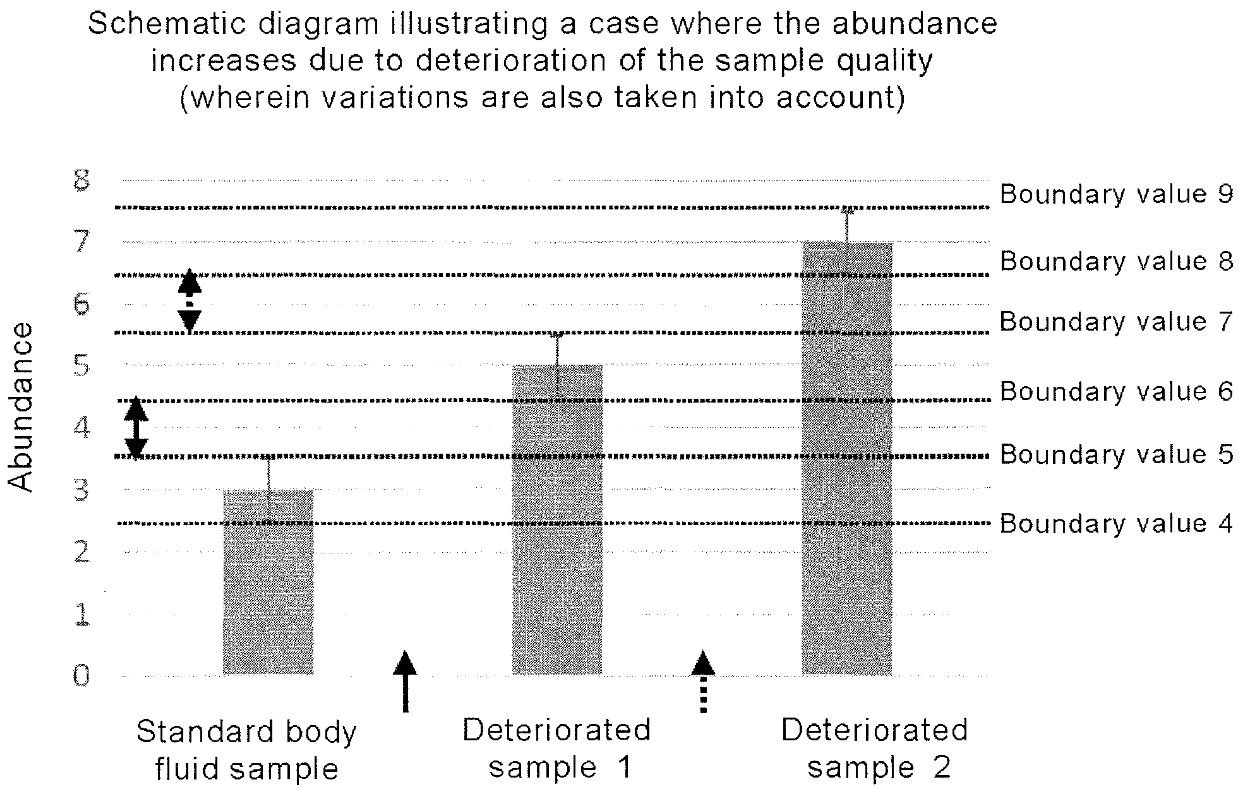
Schematic diagram illustrating a case where the abundance increases due to deterioration of the sample quality (wherein variations are also taken into account)
Abundance
0
1
2
3
4
5
6
7
8
Boundary value 9
Boundary value 8
Boundary value 7
Boundary value 6
Boundary value 5
Boundary value 4
Standard body fluid sample
Deteriorated sample 1
Deteriorated sample 2

Fig.2

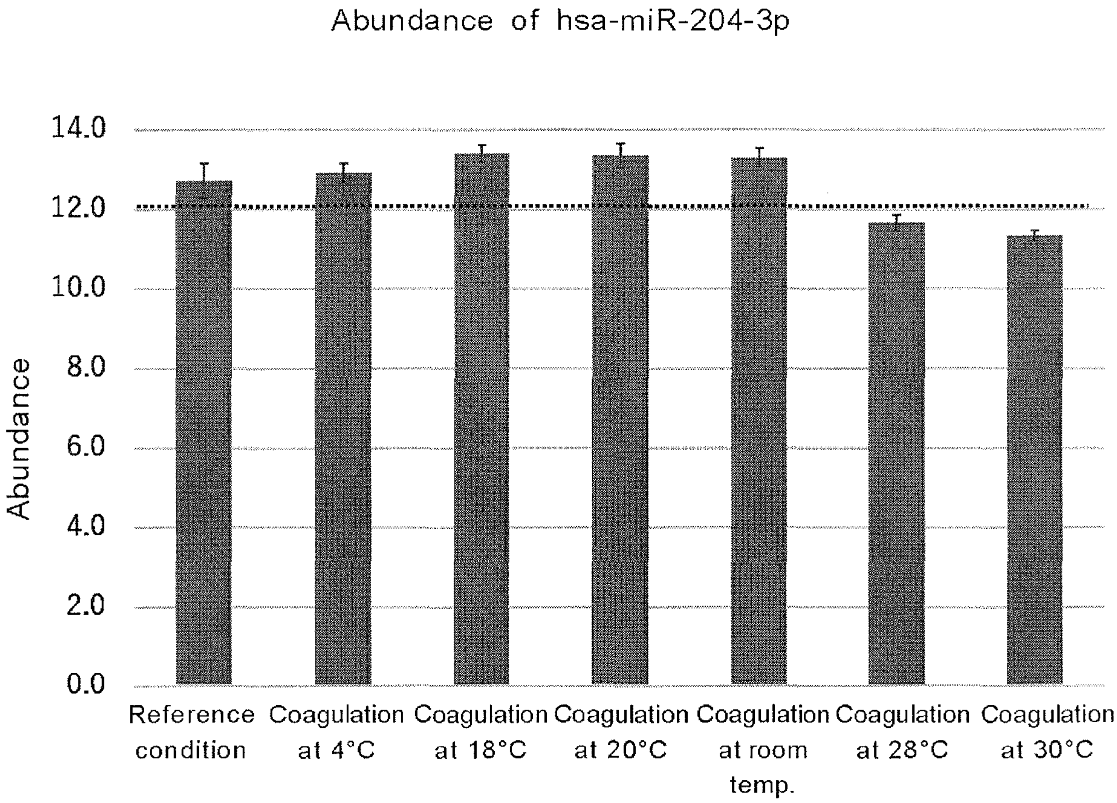
Abundance of hsa-miR-204-3p
Abundance
14.0
12.0
10.0
8.0
6.0
4.0
2.0
0.0
Reference condition
Coagulation at 4°C
Coagulation at 18°C
Coagulation at 20°C
Coagulation at room temp.
Coagulation at 28°C
Coagulation at 30°C

**Fig.3**

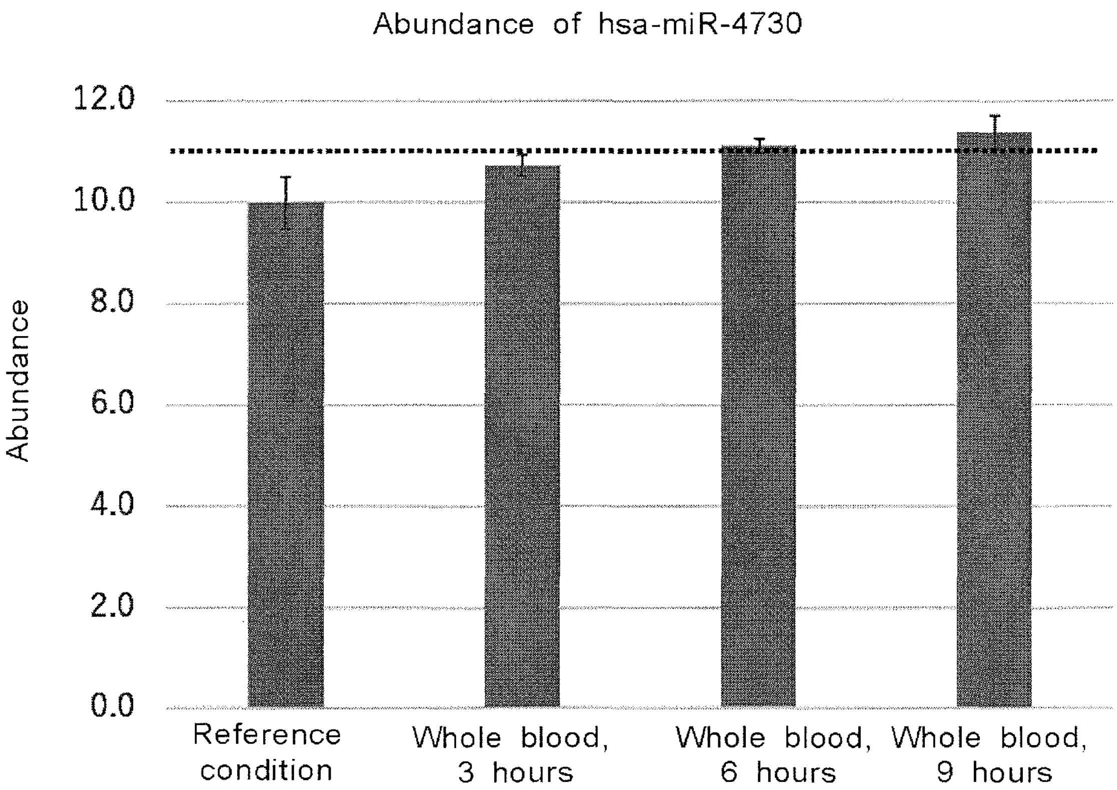
Abundance of hsa-miR-4730
Abundance
12.0
10.0
8.0
6.0
4.0
2.0
0.0
Reference condition
Whole blood, 3 hours
Whole blood, 6 hours
Whole blood, 9 hours

**Fig.4**

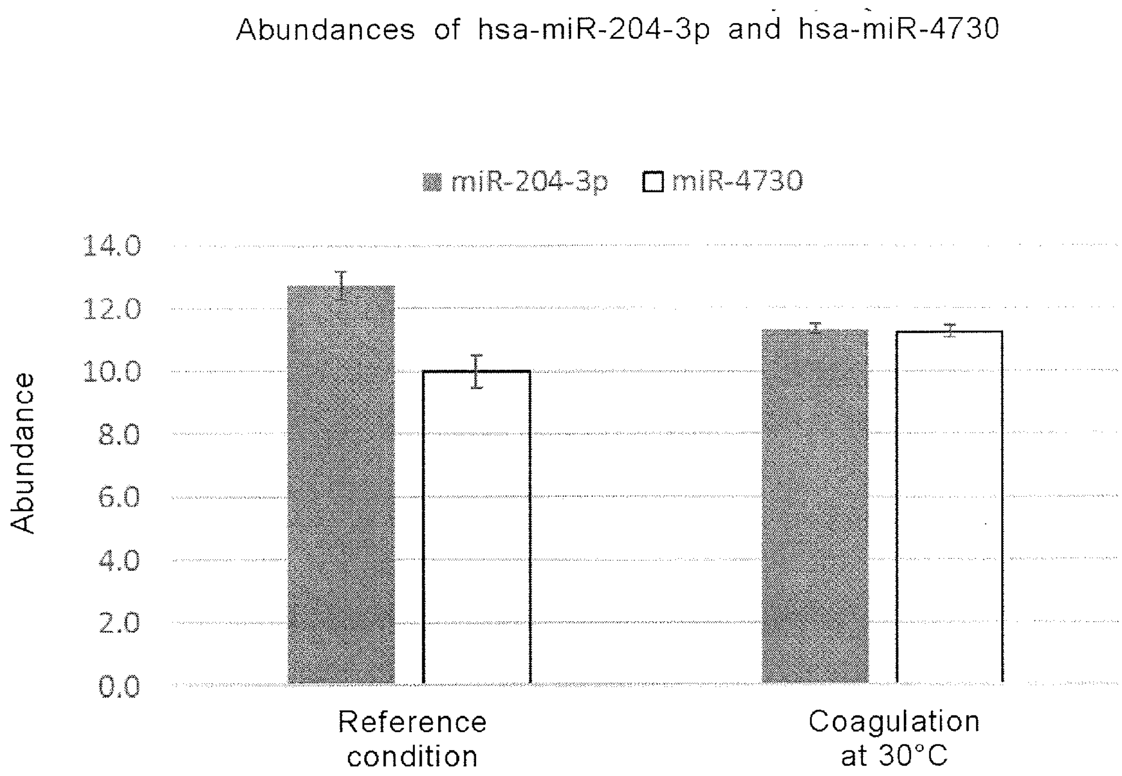
Abundances of hsa-miR-204-3p and hsa-miR-4730
miR-204-3p
miR-4730
14.0
12.0
10.0
8.0
6.0
4.0
2.0
0.0
Abundance
Reference condition
Coagulation at 30°C

Fig.5

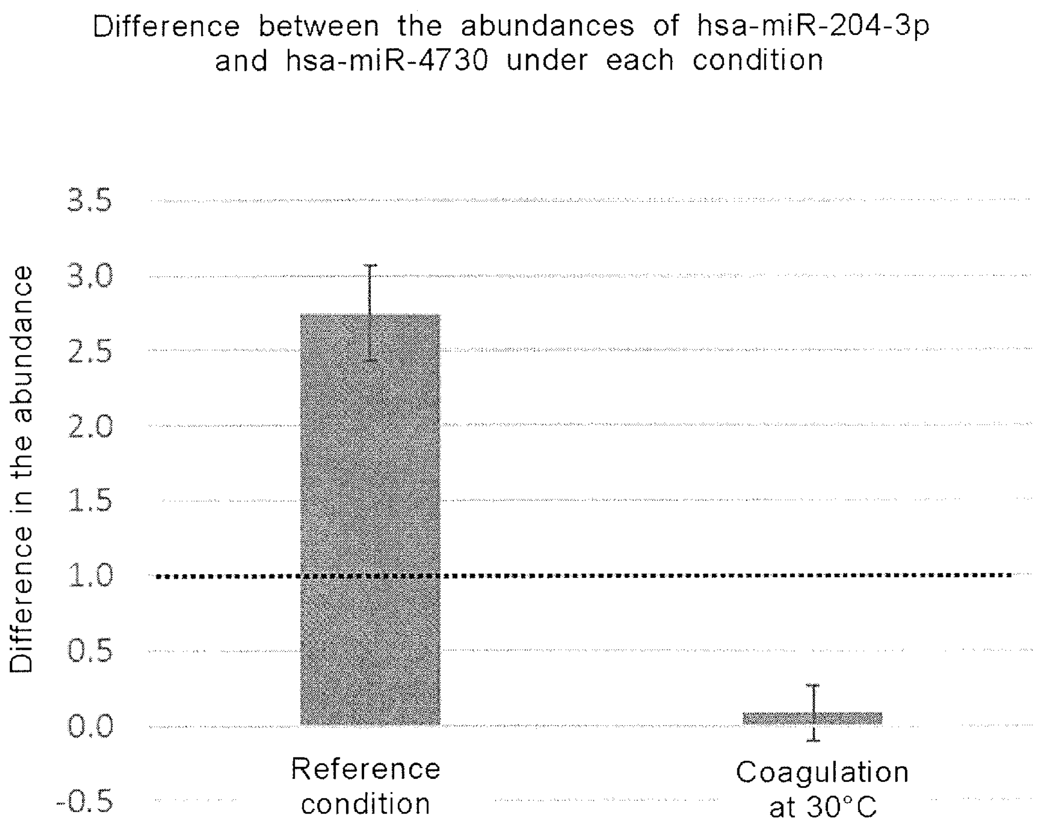
Difference between the abundances of hsa-miR-204-3p and hsa-miR-4730 under each condition
3.5
3.0
2.5
2.0
1.5
1.0
0.5
0.0
-0.5
Difference in the abundance
Reference condition
Coagulation at 30°C

Fig.6

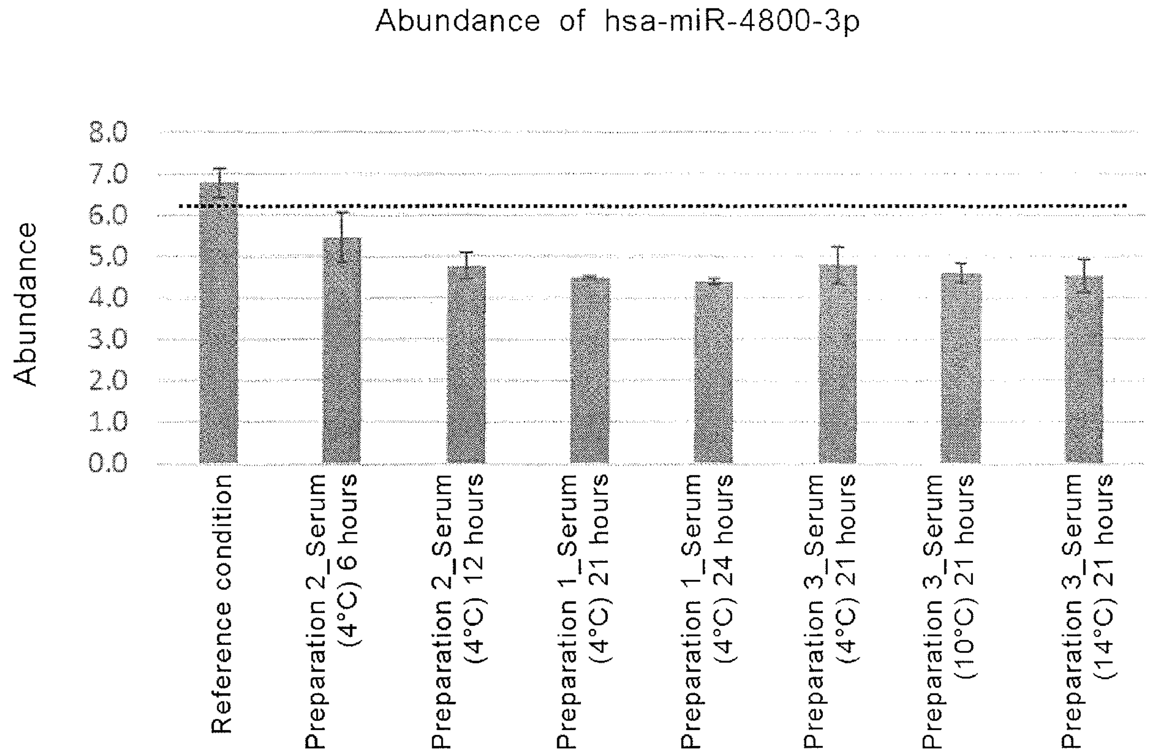

Abundance of hsa-miR-4800-3p
Abundance
8.0
7.0
6.0
5.0
4.0
3.0
2.0
1.0
0.0
Reference condition
Preparation 2_Serum (4°C) 6 hours
Preparation 2_Serum (4°C) 12 hours
Preparation 1_Serum (4°C) 21 hours
Preparation 1_Serum (4°C) 24 hours
Preparation 3_Serum (4°C) 21 hours
Preparation 3_Serum (10°C) 21 hours
Preparation 3_Serum (14°C) 21 hours


**Fig.7**

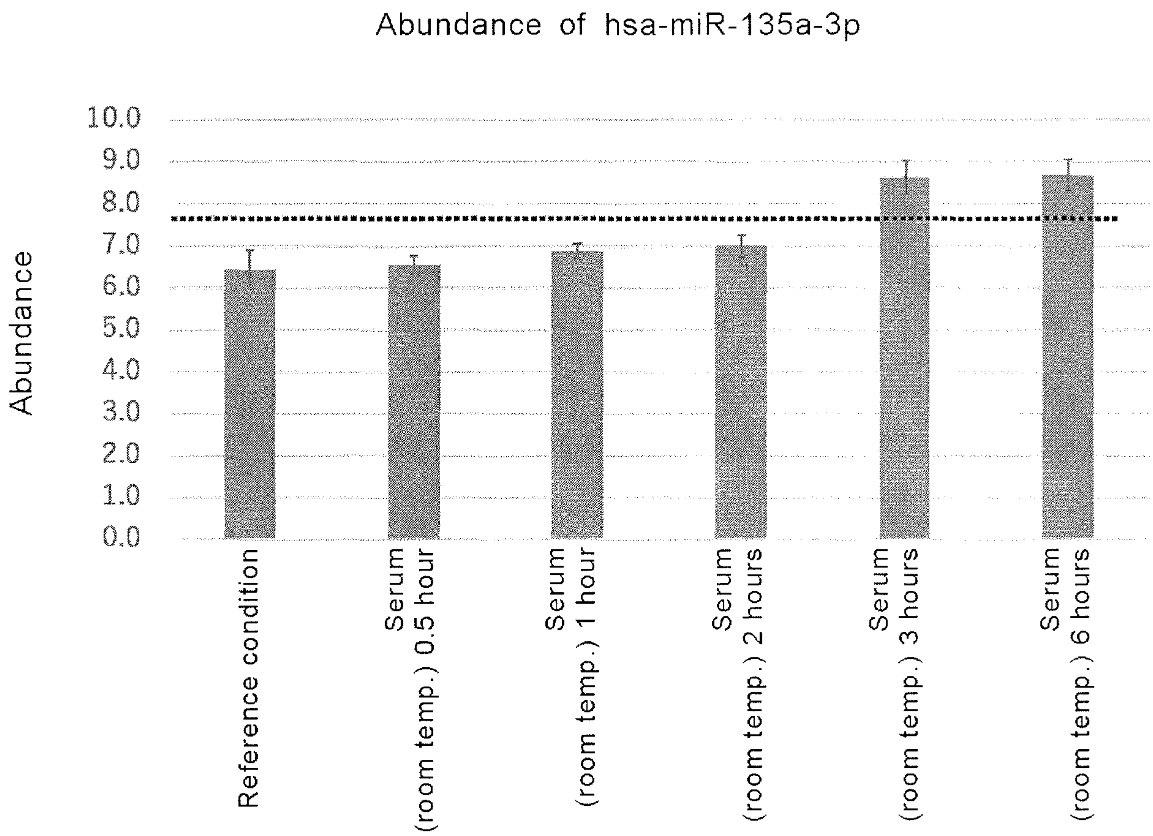

Abundance of hsa-miR-135a-3p
Abundance
10.0
9.0
8.0
7.0
6.0
5.0
4.0
3.0
2.0
1.0
0.0
Reference condition
Serum (room temp.) 0.5 hour
Serum (room temp.) 1 hour
Serum (room temp.) 2 hours
Serum (room temp.) 3 hours
Serum (room temp.) 6 hours


**Fig.8**

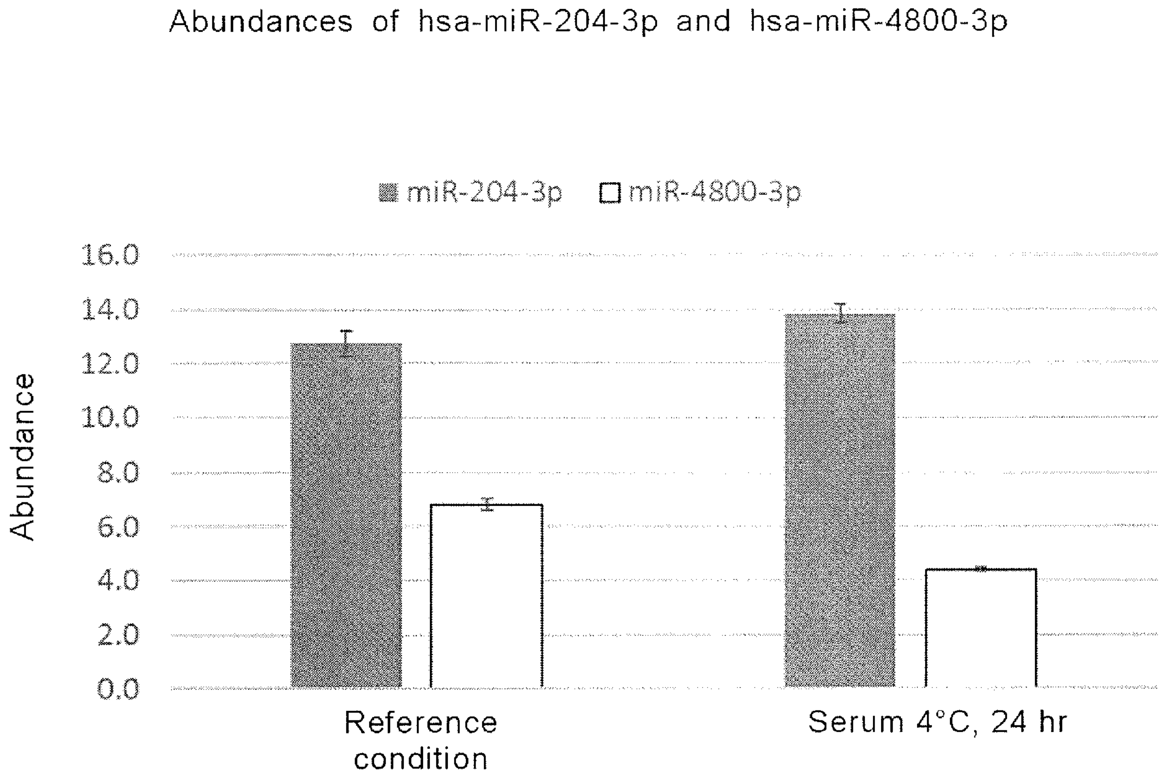
Abundances of hsa-miR-204-3p and hsa-miR-4800-3p
miR-204-3p
miR-4800-3p
Abundance
16.0
14.0
12.0
10.0
8.0
6.0
4.0
2.0
0.0
Reference condition
Serum 4°C, 24 hr

**Fig.9**

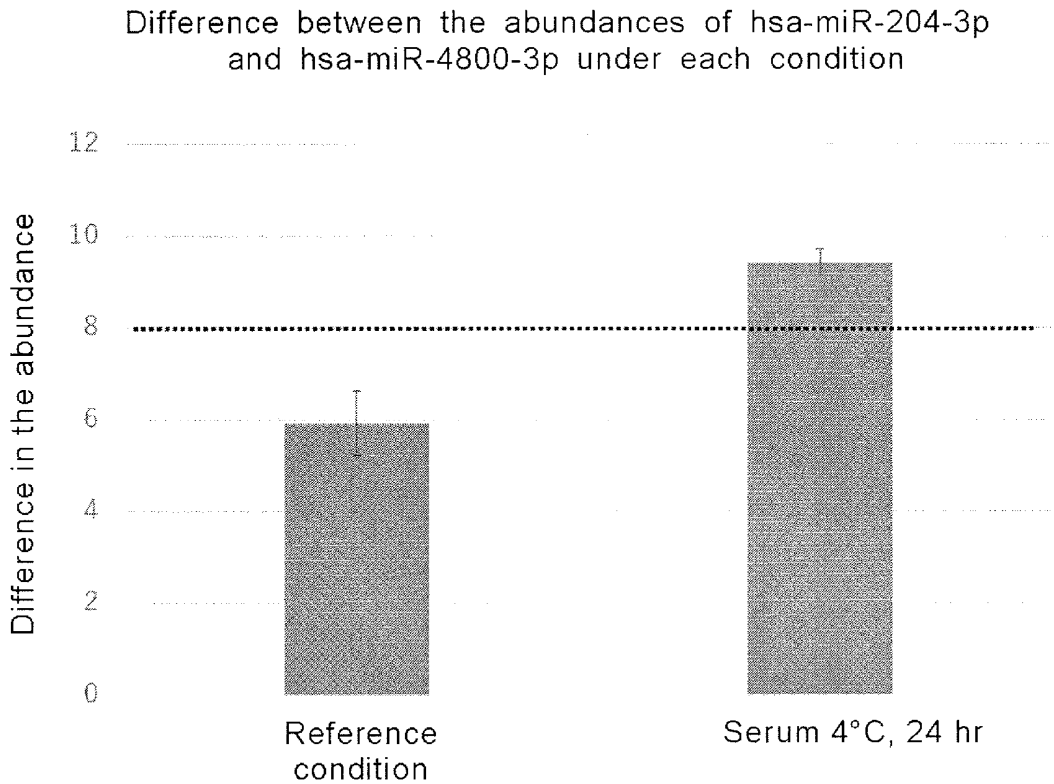
Difference between the abundances of hsa-miR-204-3p and hsa-miR-4800-3p under each condition
Difference in the abundance
12
10
8
6
4
2
0
Reference condition
Serum 4°C, 24 hr

**Fig.10**

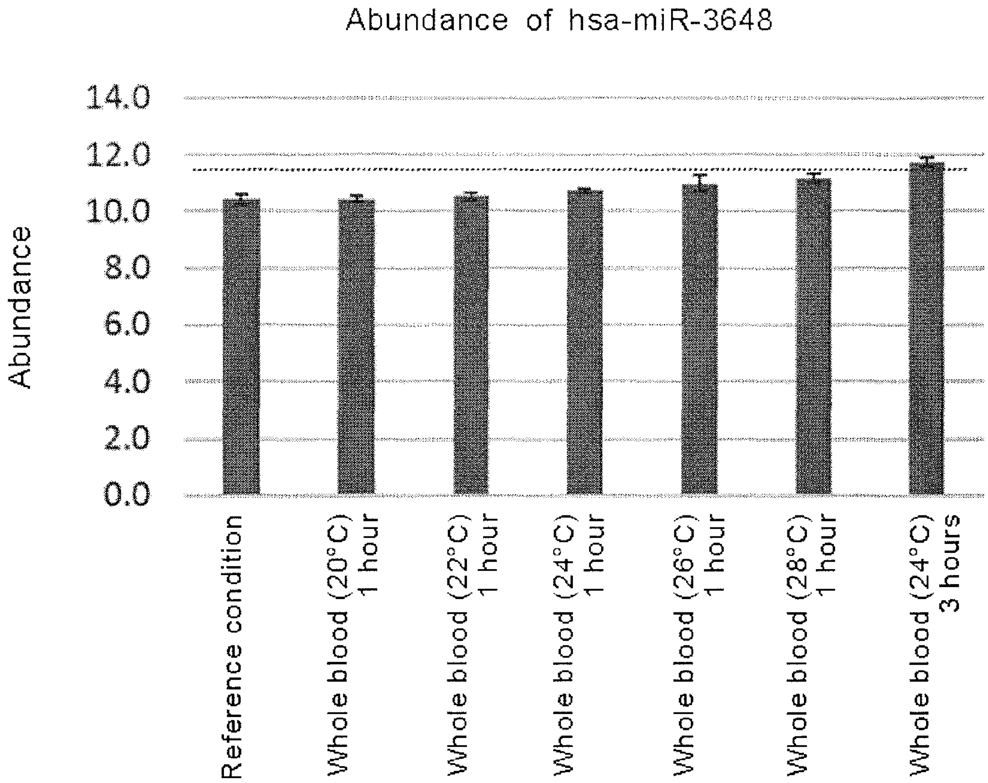
Abundance of hsa-miR-3648
Abundance
14.0
12.0
10.0
8.0
6.0
4.0
2.0
0.0
Reference condition
Whole blood (20°C) 1 hour
Whole blood (22°C) 1 hour
Whole blood (24°C) 1 hour
Whole blood (26°C) 1 hour
Whole blood (28°C) 1 hour
Whole blood (24°C) 3 hours

**Fig.11**

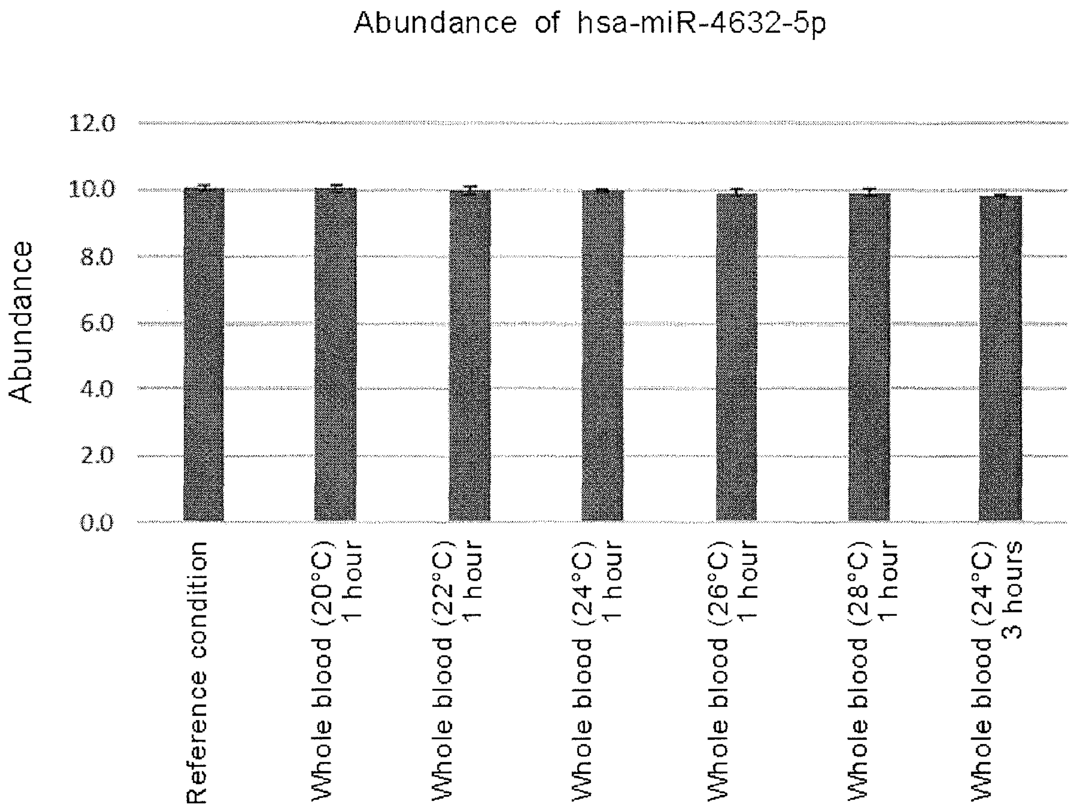
Abundance of hsa-miR-4632-5p
Abundance
12.0
10.0
8.0
6.0
4.0
2.0
0.0
Reference condition
Whole blood (20°C) 1 hour
Whole blood (22°C) 1 hour
Whole blood (24°C) 1 hour
Whole blood (26°C) 1 hour
Whole blood (28°C) 1 hour
Whole blood (24°C) 3 hours

**Fig.12**

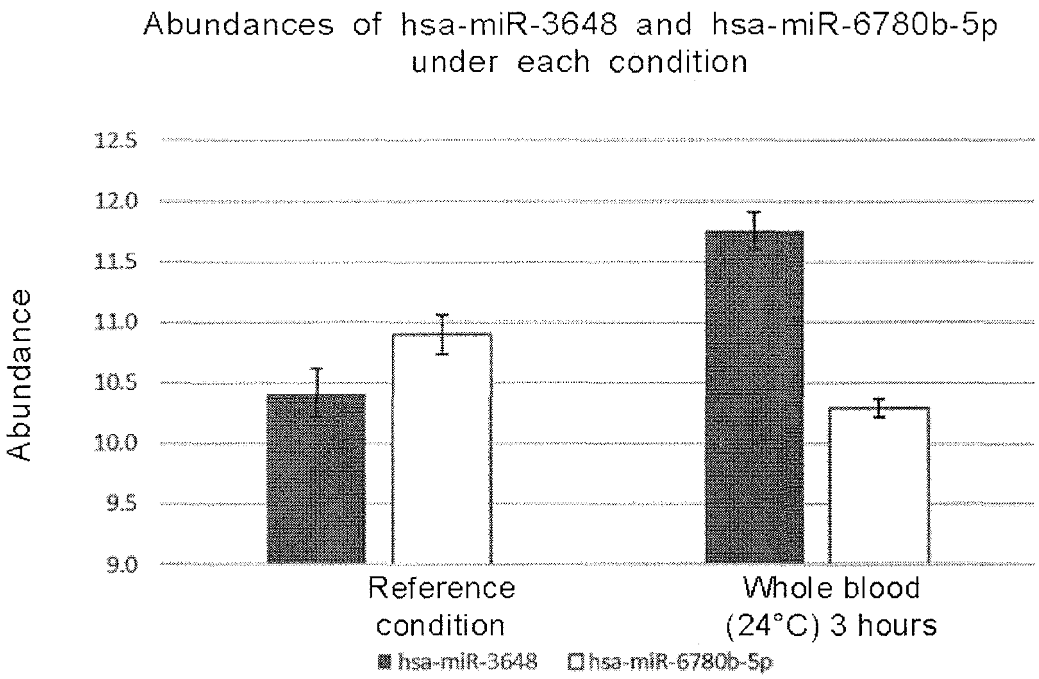
Abundances of hsa-miR-3648 and hsa-miR-6780b-5p under each condition
Abundance
12.5
12.0
11.5
11.0
10.5
10.0
9.5
9.0
Reference condition
Whole blood (24°C) 3 hours
hsa-miR-3648
hsa-miR-6780b-5p

**Fig.13**

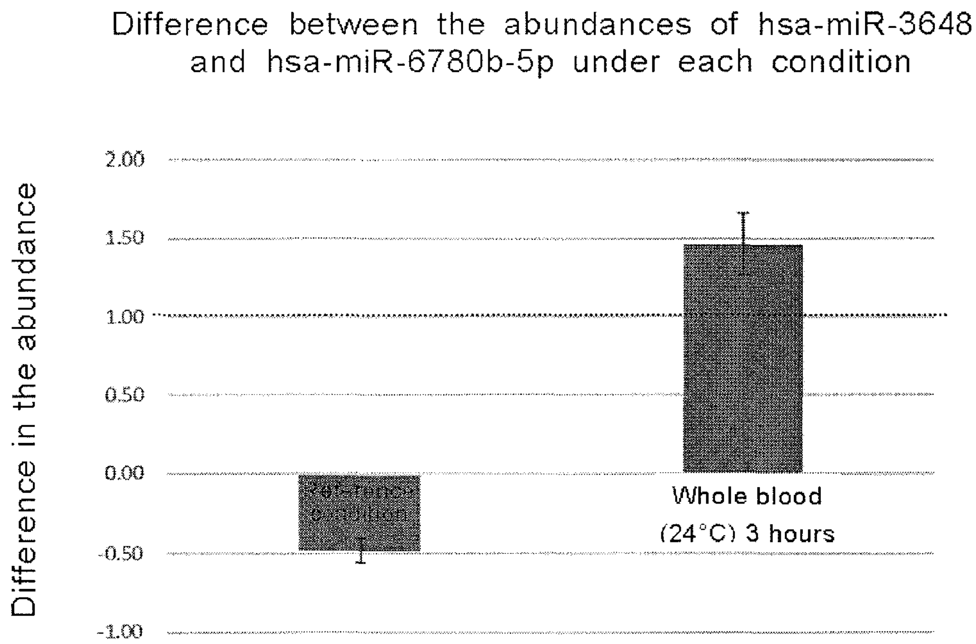
Difference between the abundances of hsa-miR-3648 and hsa-miR-6780b-5p under each condition
Difference in the abundance
2.00
1.50
1.00
0.50
0.00
-0.50
-1.00
Whole blood (24°C) 3 hours

**Fig.14**

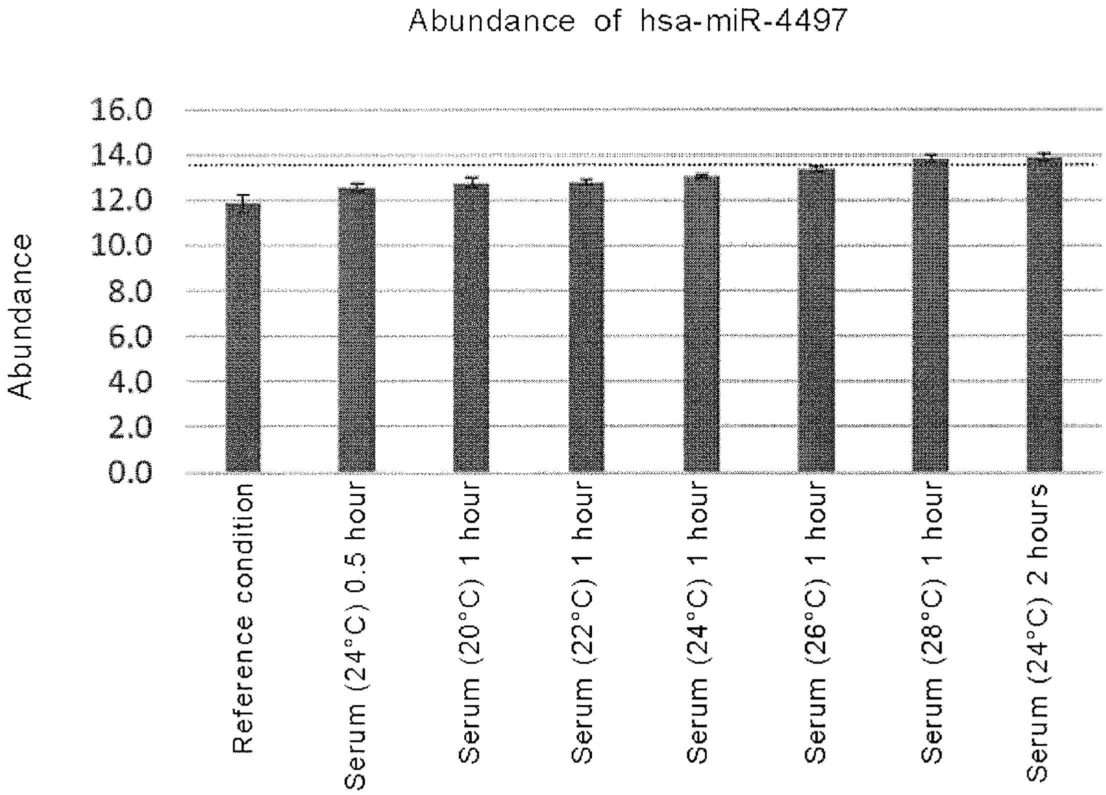
Abundance of hsa-miR-4497
16.0
14.0
12.0
10.0
8.0
6.0
4.0
2.0
0.0
Abundance
Reference condition
Serum (24°C) 0.5 hour
Serum (20°C) 1 hour
Serum (22°C) 1 hour
Serum (24°C) 1 hour
Serum (26°C) 1 hour
Serum (28°C) 1 hour
Serum (24°C) 2 hours

**Fig.15**

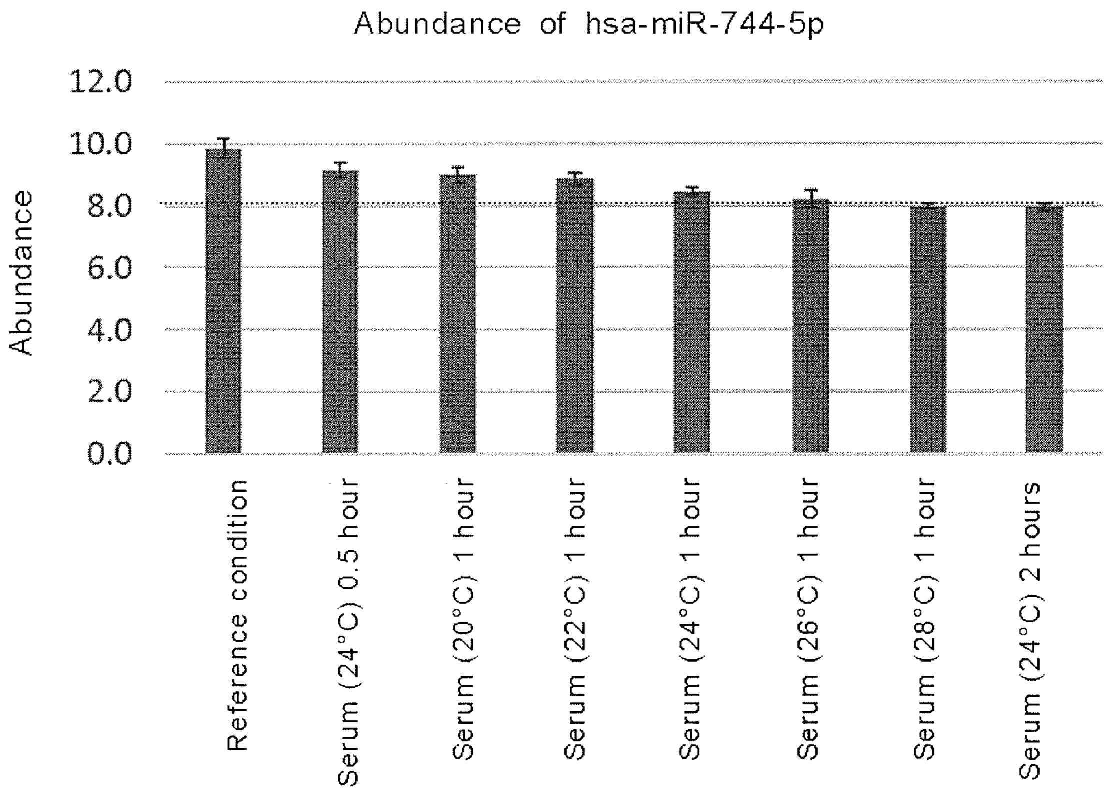
Abundance of hsa-miR-744-5p
12.0
10.0
8.0
6.0
4.0
2.0
0.0
Abundance
Reference condition
Serum (24°C) 0.5 hour
Serum (20°C) 1 hour
Serum (22°C) 1 hour
Serum (24°C) 1 hour
Serum (26°C) 1 hour
Serum (28°C) 1 hour
Serum (24°C) 2 hours

**Fig.16**

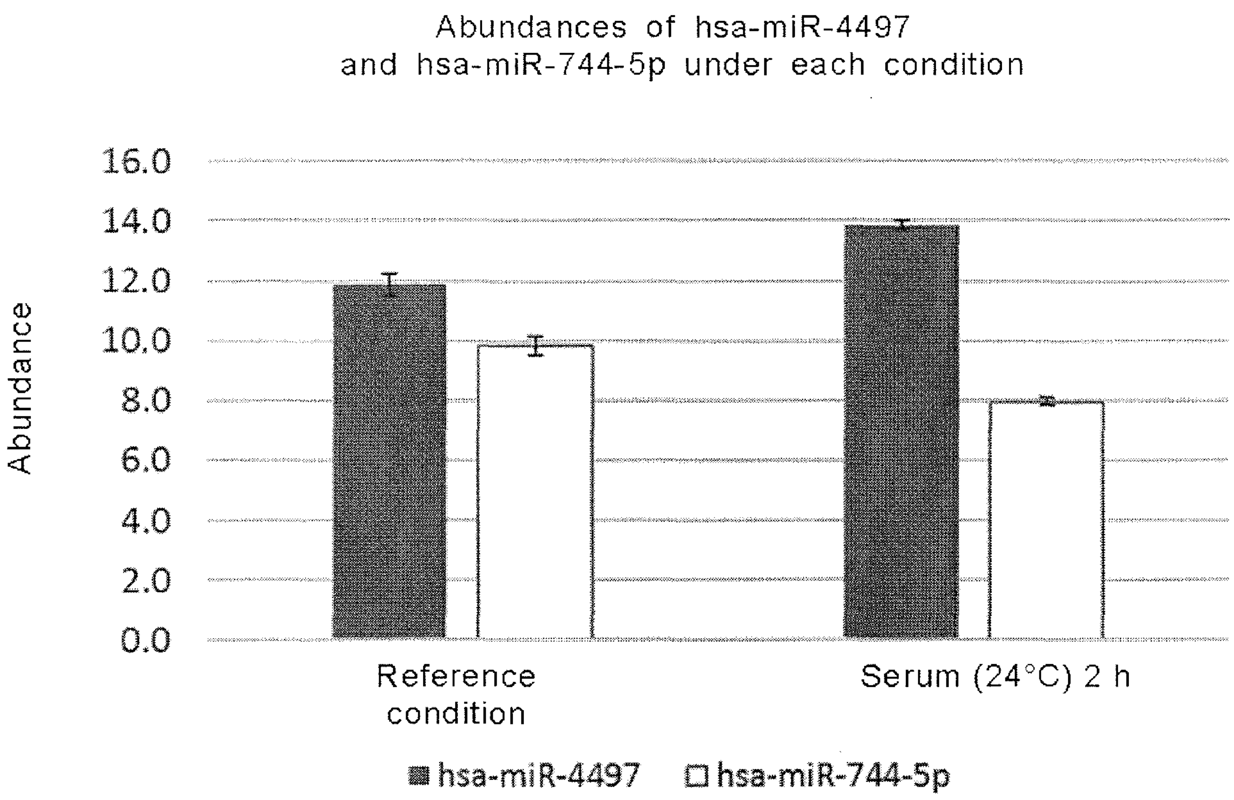
Abundances of hsa-miR-4497
and hsa-miR-744-5p under each condition
Abundance
16.0
14.0
12.0
10.0
8.0
6.0
4.0
2.0
0.0
Reference condition
Serum (24°C) 2 h
hsa-miR-4497
hsa-miR-744-5p

**Fig.17**

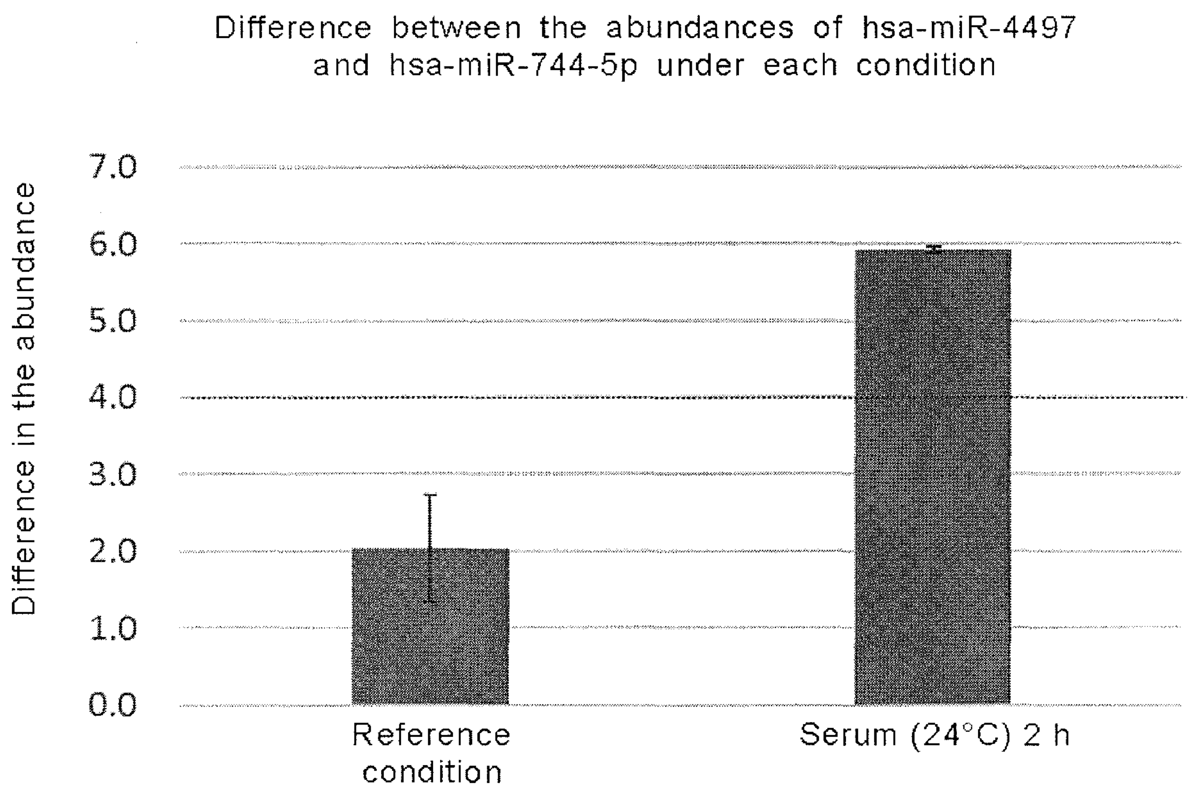
Difference between the abundances of hsa-miR-4497
and hsa-miR-744-5p under each condition
Difference in the abundance
7.0
6.0
5.0
4.0
3.0
2.0
1.0
0.0
Reference condition
Serum (24°C) 2 h

**Fig.18**

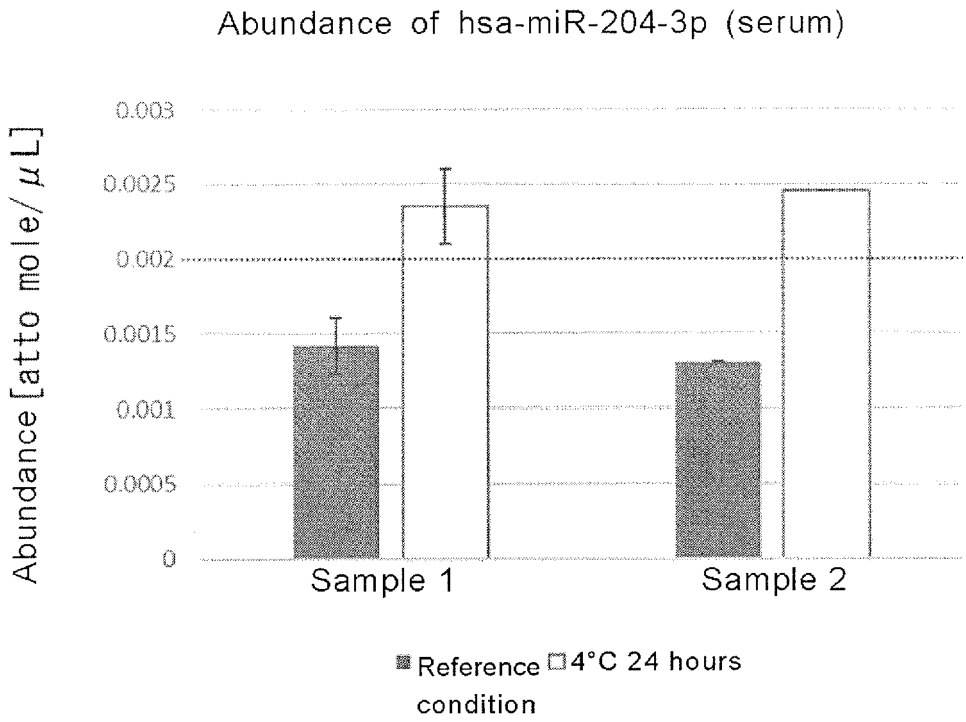
Abundance of hsa-miR-204-3p (serum)
Abundance [atto mole/μL]
0.003
0.0025
0.002
0.0015
0.001
0.0005
0
Sample 1
Sample 2
Reference condition
4°C 24 hours

Fig.19

| INTERNATIONAL SEARCH REPORT | International application No. PCT/JP2019/029761 |
|---|---|

**A. CLASSIFICATION OF SUBJECT MATTER**
Int.Cl. C12N15/09(2006.01)i, C12M1/00(2006.01)i, C12Q1/6837(2018.01)i, G01N33/53(2006.01)i, C12N15/113(2010.01)n

According to International Patent Classification (IPC) or to both national classification and IPC

**B. FIELDS SEARCHED**

Minimum documentation searched (classification system followed by classification symbols)
Int.Cl. C12N15/09, C12M1/00, C12Q1/6837, G01N33/53, C12N15/113

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS(STN), JSTPlus/JMEDPlus/JST7580(JDreamIII), GenBank/EMBL/DDBJ/GeneSeq, PubMed

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2018/056432 A1 (TORAY INDUSTRIES, INC.) 29 March 2018, claims 1, 4-6, 8, example 1 (Family: none) | 1-11<br>12 |
| Y | WO 2017/171048 A1 (TORAY INDUSTRIES, INC. et al.) 05 October 2017, claims 8, 11, 15-18 & EP 3438284 A1, claims 8, 11, 15-18 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26.08.2019 | 03.09.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/029761

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2015/194627 A1 (TORAY INDUSTRIES, INC. et al.) 23 December 2015, claims 12, 18, 23-25 & EP 3159406 A1, claims 12, 18, 23-25 & US 2017/0130273 A1 | 1-12 |
| Y | WO 2015/194615 A1 (TORAY INDUSTRIES, INC. et al.) 23 December 2015, claims 12, 23-25 & EP 3159398 A1, claims 12, 23-25 & US 2017/0166975 A1 | 1-12 |
| Y<br>A | WO 2017/146033 A1 (TORAY INDUSTRIES, INC.) 31 August 2017, claims 1, 15 & EP 3421610 A1, claims 1, 15 & US 2019/0048408 A1 | 12<br>1-11 |
| A | WO 2016/071729 A1 (BIOMIRNA HOLDINGS, LTD.) 12 May 2016, entire text, all drawings (Family: none) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015519045 A
- JP 2008035779 A
- WO 2017146033 A
- JP 4244788 B
- US 5705610 B, Ronald
- US 6142266 B, Michel
- US 7037659 B, Francesco
- JP 3922454 B, Hirota

### Non-patent literature cited in the description


- **FAVALORO et al.** *Methods Enzymol.,* 1980, vol. 65, 718
- **SAMBROOK, J. et al.** Molecular Cloning: A Laboratory Manual. Cold Spring Harbor Laboratory Press, 1998
- **KOBERLE V. et al.** *Translational Res.,* 2016, vol. 169, 40-46
- miRNA Experimental Protocols. Yodosha Co., Ltd, 2008
- Microarray data statistical analysis protocols. Yodosha Co., Ltd, 2008
- **LIM LP et al.** *Science,* 2003, vol. 299, 1540
- **PERSSON H et al.** *Cancer Res,* 2011, vol. 71, 78-86
- **LAGOS-QUINTANA M et al.** *Curr Biol,* 2002, vol. 12, 735-739
- **LADEWIG E et al.** *Genome Research,* 2012, vol. 22, 1634-1645
- **VOELLENKLE C et al.** *RNA,* 2012, vol. 18, 472-484
- **BEREZIKOV E et al.** *Genome Res,* 2006, vol. 16, 1289-1298
- **JOYCE CE et al.** *Hum Mol Genet,* 2011, vol. 20, 4025-4040
- **LUI WO et al.** *Cancer Res.,* 2007, vol. 67, 6031-6043
- **JIMA DD et al.** *Blood,* 2010, vol. 116, e118-e127
- **LI Y et al.** *Gene,* 2012, vol. 497, 330-335
- **WITTEN D et al.** *BMC Biol,* 2010, vol. 8, 58
- **MEIRI E et al.** *Nucleic Acids Res,* 2010, vol. 38, 6234-6246
- **TAKADA S et al.** *Leukemia,* 2008, vol. 22, 1274-1278
- **MOURELATOS Z et al.** *Genes Dev,* 2002, vol. 16, 720-728
- **STARK MS et al.** *PLoS One,* 2010, vol. 5, e9685
- **LIAO JY et al.** *PLoS One,* 2010, vol. 5, e10563
- **GOFF LA et al.** *PLoS One,* 2009, vol. 4, e7192
- **HANSEN TB et al.** *RNA Biol,* 2011, vol. 8, 378-383
- **DANNEMANN M et al.** *Genome Biol Evol,* 2012, vol. 4, 552-564
- **TANDON M et al.** *Oral Dis,* 2012, vol. 18, 127-131